# NEW EUROPEAN PATENT SPECIFICATION

(11) **EP 1 991 678 B2**
(45) Date of publication and mention of the opposition decision: **15.07.2020**
(45) Mention of the grant of the patent: 09.09.2015
(21) Application number: 07750780.4
(22) Date of filing: 15.02.2007
(51) Int. Cl.: C12N 15/117, A61K 31/70, A61K 48/00

(54) **COMPOSITIONS AND METHODS FOR OLIGONUCLEOTIDE FORMULATIONS**
ZUSAMMENSETZUNGEN UND VERFAHREN FÜR OLIGONUKLEOTIDFORMULIERUNGEN
COMPOSITIONS ET PROCÉDÉS POUR FORMULATIONS À BASE D'OLIGONUCLÉOTIDES

(30) Priority: 15.02.2006 US 773505 P; 09.11.2006 US 858240 P
(43) Date of publication of application: 19.11.2008
(60) Divisional application: 11183857.9
(73) Proprietor: Rechtsanwalt Thomas Beck, 08523 Plauen (DE)
(72) Inventor: UHLMANN, Eugen, 61479 Glashuetten (DE); VOLLMER, Joerg, 40591 Duesseldorf (DE); KRIEG, Arthur, M., Wellesley, MA 02482 (US); SAMULOWITZ, Ulrike, 40764 Langenfeld (DE); NOLL, Bernhard, O., 41468 Neuss (DE)
(74) Representative: Kalhammer, Georg
(86) International application number: PCT/US2007/003964
(87) International publication number: WO 2007/095316

(56) References cited:
- WO-A2-2004/016805
- WO-A2-2005/042018
- WO-A2-2005/042018
- WO-A2-2006/110607
- WO-A2-2006/134423
- WO-A2-2006/135434
- WO-A2-2007/026190
- US-A1- 2005 215 501
- US-A1- 2006 019 923
- TOKUNAGA T. ET AL.: "Synthetic oligonucleotides with particulate base sequences from the cDNA encoding proteins of Mycobacterium bovis BCG induce Interferons and activate Natural Killer cells" MICROBIOLOGY AND IMMUNOLOGY, TOKYO, JP, vol. 36, no. 1, 1992, pages 55-66, XP008024955 ISSN: 0385-5600
- KURAMOTO E. ET AL.: "Oligonucleotide sequences required for Natural Killer cell activation" JAPANESE JOURNAL OF CANCER RESEARCH, JAPANESE CANCER ASSOCIATION, TOKYO, JP, vol. 83, no. 11, November 1992 (1992-11), pages 1128-1131, XP008024190 ISSN: 0910-5050
- VOLLMER J. ET AL.: "Characterization of three CpG oligodeoxynucleotide classes with distinct immunostimulatory activities" EUROPEAN JOURNAL OF IMMUNOLOGY, WEINHEIM, DE, vol. 34, no. 1, January 2004 (2004-01), pages 251-262, XP002398079 ISSN: 0014-2980
- KANDIMALLA E.R. ET AL.: "Towards optical design of second-generation immunomodulatory oligonucleotides" CURRENT OPINION IN MOLECULAR THERAPEUTICS, CURRENT DRUGS, LONDON,, GB, vol. 4, no. 2, 2002, pages 122-129, XP008050943 ISSN: 1464-8431
- IHO S. ET AL.: "Oligodeoxynucleotides containing palindrome sequences with internal 5'-CpG-3' act directly on human NK and activated T cells to induce IFN-gamma production in vitro" THE JOURNAL OF IMMUNOLOGY, vol. 163, 1999, pages 3642-3652, XP002457881
- YAMAMOTO S. ET AL.: "Unique palindromic sequences in synthetic oligonucleotides are required to induce INF and augment INF-mediated Natural Killer activity" THE JOURNAL OF IMMUNOLOGY, vol. 148, no. 12, 15 June 1992 (1992-06-15), pages 4072-4076, XP002457882
- YAMAMOTO S. ET AL.: "Oligodeoxyribonucleotides with 5-ACGT-3' or 5'TCGA-3' sequence induce production of interferons" CURRENT TOPICS IN MICROBIOLOGY AND IMMUNOLOGY, SPRINGER, BERLIN,, DE, vol. 247, 2000, pages 23-39, XP002245207 ISSN: 0070-217X

## Description

### FIELD OF THE INVENTION

The present invention relates generally to immunostimulatory nucleic acids, compositions thereof and methods of using the immunostimulatory nucleic acids.

### BACKGROUND OF THE INVENTION

Cancer is the second leading cause of death in the United States, resulting in one out of every four deaths. In 1997, the estimated total number of new diagnoses for lung, breast, prostate, colorectal and ovarian cancer was approximately two million. Due to the ever increasing aging population in the United States, it is reasonable to expect that rates of cancer incidence will continue to grow.

Asthma is a chronic inflammatory disease effecting 14-15 million persons in the United States alone.

Infectious disease is one of the leading causes of death throughout the world. In the United States alone the death rate due to infectious disease rose 58% between 1980 and 1992. During this time, the use of anti-infective therapies to combat infectious disease has grown significantly and is now a multi-billion dollar a year industry. Even with these increases in anti-infective agent use, the treatment and prevention of infectious disease remains a challenge to the medical community throughout the world.

Bacterial DNA has immune stimulatory effects to activate B cells and natural killer cells, but vertebrate DNA does not (Tokunaga, T., et al., 1988. Jpn. J. Cancer Res. 79:682-686; Tokunaga, T., et al., 1984, JNCI 72:955-962; Messina, J.P., et al., 1991, J. Immunol. 147:1759-1764; and reviewed in Krieg, 1998, In: Applied Oligonucleotide Technology, C.A. Stein and A.M. Krieg, (Eds.), John Wiley and Sons, Inc., New York, NY, pp. 431-448). It is now understood that these immune stimulatory effects of bacterial DNA are a result of the presence of unmethylated CpG dinucleotides in particular base contexts (CpG motifs), which are common in bacterial DNA, but methylated and underrepresented in vertebrate DNA (Krieg et al, 1995 Nature 374:546-549; Krieg, 1999 Biochim. Biophys. Acta 93321:1-10). The immune stimulatory effects of bacterial DNA can be mimicked with synthetic oligodeoxynucleotides (ODN) containing these CpG motifs. Such CpG ODN have highly stimulatory effects on human and murine leukocytes, inducing B cell proliferation; cytokine and immunoglobulin secretion; natural killer (NK) cell lytic activity and IFN-γ secretion; and activation of dendritic cells (DCs) and other antigen presenting cells to express costimulatory molecules and secrete cytokines, especially the Th1-like cytokines that are important in promoting the development of Th1-like T cell responses. Yamamoto et al. (1992) J Immunol 148: 4072-4076 showed that phosphodiester palindromic sequences containing CpG dinucleotides are directly responsible for secretion of interferons. Iho et al. (1999) J Immunol 163: 3642-3652 described that phosphodiester palindromic sequences containing CpG dinucleotides activate NK cells and induce the production of IFNγ. The immune stimulatory effects of native phosphodiester backbone CpG ODN are highly CpG specific in that the effects are dramatically reduced if the CpG motif is methylated, changed to a GpC, or otherwise eliminated or altered (Krieg et al, 1995 Nature 374:546-549; Hartmann et al, 1999 Proc. Natl. Acad. Sci USA 96:9305-10).

In early studies, it was thought that the immune stimulatory CpG motif followed the formula purine-purine-CpG-pyrimidine-pyrimidine (Krieg et al, 1995 Nature 374:546-549; Pisetsky, 1996 J. Immunol. 156:421-423; Hacker et al., 1998 EMBO J. 17:6230-6240; Lipford et al, 1998 Trends in Microbiol. 6:496-500). However, it is now clear that mouse lymphocytes respond quite well to phosphodiester CpG motifs that do not follow this "formula" (Yi et al., 1998 J. Immunol. 160:5898-5906) and the same is true of human B cells and dendritic cells (Hartmann et al, 1999 Proc. Natl. Acad. Sci USA 96:9305-10; Liang, 1996 J. Clin. Invest. 98:1119-1129).

Several different classes of CpG oligonucleotides have recently been described. One class is potent for activating B cells but is relatively weak in inducing IFN-α and NK cell activation; this class has been termed the B class. The B class CpG oligonucleotides are typically fully stabilized and include an unmethylated CpG dinucleotide within certain preferred base contexts. See, e.g., U.S. Patent Nos. 6,194,388; 6,207,646; 6,214,806; 6,218,371; 6,239,116; and 6,339,068. Another class of CpG oligonucleotides activates B cells and NK cells and induces IFN-α; this class has been termed the C-class. The C-class CpG oligonucleotides, as first characterized, are typically fully stabilized; include a B class-type sequence and a GC-rich palindrome or near-palindrome. This class has been described in co-pending U.S. provisional patent application 60/313,273, filed August 17, 2001 and US10/224,523 filed on August 19, 2002 and related PCT Patent Application PCT/US02/26468 published under International Publication Number WO 03/015711. Various C class CpG oligonucleotides are disclosed in WO 2004/016805 A2. Kandimalla et al. (2002) Current Opinion in Molecular Therapeutics Vol 4 No. 2 summarizes structural and chemical characteristics of CpG oligonucleotides that are important for molecular recognition.

The distinct immunomodulatory activities of A class, B class, and C class CpG oligonucleotides have been characterized in Vollmer et al. (2004) Eur. J. Immunol. 34: 251-262.

### SUMMARY OF THE INVENTION

The present invention is defined in the appended claims.

The present invention relates in part to immunostimulatory CpG containing oligonucleotides, in particular a new class of immunostimulatory oligonucleotides termed P-Class. Originally the structure of CpG oligonucleotides was not considered to be particularly important to immune activation, but subsequently it was realized that oligonucleotides containing poly G motifs at one or both ends (A-Class), or oligonucleotides with a 3' palindrome (C-Class), induced higher levels of NK cell activation and pDC IFN-α secretion than oligonucleotides with no potential to form secondary structures. The A-Class oligonucleotides can form very complex higher ordered structures such as nanoparticles (Kerkmann et al., J Biol Chem. (2005) 280(9):8086-93.), and the C-Class may form intermolecular duplexes or hairpins. The present invention concerns the identification of a new sub-class of CpG oligonucleotides, that contain duplex forming regions such as, for example, perfect or imperfect palindromes at or near both the 5' and 3' ends, giving them the potential to form concatamers. These oligonucleotides referred to as P-Class oligonucleotides have the ability in some instances to induce much high levels of IFN-α secretion than the C-Class. The P-Class oligonucleotides have the ability to spontaneously self-assemble into concatamers either *in vitro* and/or *in vivo.* Without being bound by any particular theory for the method of action of these molecules, one potential hypothesis is that this property endows the P-Class oligonucleotides with the ability to more highly crosslink TLR9 inside certain immune cells, inducing a distinct pattern of immune activation compared to the previously described classes of CpG oligonucleotides.

In one aspect of the invention the immunostimulatory oligonucleotide contains a 5' TLR activation domain and at least two palindromic regions, one palindromic region being a 5' palindromic region of at least 6 nucleotides in length and connected to a 3' palindromic region of at least 8 nucleotides in length either directly or through a spacer, wherein the oligonucleotide includes at least one unmethylated CpG, and wherein the oligonucleotide has the formula 5' XP₁SP₂T 3', wherein X is the TLR activation domain, P₁ is a palindrome, S is a spacer, P₂ is a palindrome, and T is a 3' tail of 0-100 nucleotides in length, wherein a "palindrome" or "palindromic region" is a nucleic acid sequence which is its own perfect reverse complement. In another embodiment the TLR activation domain is TCG, TTCG, TTTCG, TYpR, TTYpR, TTTYpR, UCG, UUCG, UUUCG, TTT, or TTTT. In still another embodiment the 5' palindromic region is at least 8 nucleotides in length. In another embodiment the 3' palindromic region is at least 10 nucleotides in length. In another embodiment the 5' palindromic region is at least 10 nucleotides in length. In yet another embodiment the 3' palindromic region includes an unmethylated CpG dinucleotide. In another embodiment the 3' palindromic region includes two unmethylated CpG dinucleotides. In another embodiment the 5' palindromic region includes an unmethylated CpG dinucleotide. In yet another embodiment the 5' palindromic region includes two unmethylated CpG dinucleotides. In another embodiment the 5' and 3' palindromic regions have a duplex stability value of at least 25. In another embodiment the 5' and 3' palindromic regions have a duplex stability value of at least 30. In another embodiment the 5' and 3' palindromic regions have a duplex stability value of at least 35. In another embodiment the 5' and 3' palindromic regions have a duplex stability value of at least 40. In another embodiment the 5' and 3' palindromic regions have a duplex stability value of at least 45. In another embodiment the 5' and 3' palindromic regions have a duplex stability value of at least 50. In another embodiment the 5' and 3' palindromic regions have a duplex stability value of at least 55. In another embodiment the 5' and 3' palindromic regions have a duplex stability value of at least 60. In another embodiment the 5' and 3' palindromic regions have a duplex stability value of at least 65.

In one embodiment the spacer is a nucleic acid having a length of 1-50 nucleotides. In another embodiment the spacer is a nucleic acid having a length of 1 nucleotide. In another embodiment the spacer is a non-nucleotide spacer. In one embodiment the non-nucleotide spacer is a D-spacer. In another embodiment the non-nucleotide spacer is a linker. In one embodiment the oligonucleotide has the formula 5' XP₁SP₂T 3', wherein X is the TLR activation domain, P₁ is a palindrome, S is a spacer, P₂ is a palindrome, and T is a 3' tail of 0-100 nucleotides in length. In one embodiment X is TCG, TTCG, or TTTCG. In another embodiment T is 5-50 nucleotides in length. In yet another embodiment T is 5-10 nucleotides in length. In one embodiment S is a nucleic acid having a length of 1-50 nucleotides. In another embodiment S is a nucleic acid having a length of 1 nucleotide. In another embodiment S is a non-nucleotide spacer. In one embodiment the non-nucleotide spacer is a D-spacer. In another embodiment the non-nucleotide spacer is a linker. In another embodiment the oligonucleotide is not an antisense oligonucleotide or a ribozyme. In one embodiment P₁ is A and T rich. In another embodiment P₁ includes at least 4 Ts. In another embodiment P₂ is a perfect palindrome. In another embodiment P₂ is G-C rich. In still another embodiment P₂ is CGGCGCX₁GCGCCG (SEQ ID NO:334), where X₁ is T or nothing.

In one embodiment the oligonucleotide includes at least one phosphorothioate linkage. In another embodiment all internucleotide linkages of the oligonucleotide are phosphorothioate linkages. In another embodiment the oligonucleotide includes at least one phosphodiester-like linkage. In another embodiment the phosphodiester-like linkage is a phosphodiester linkage. In another embodiment a lipophilic group is conjugated to the oligonucleotide. In one embodiment the lipophilic group is cholesterol.

Another aspect of the invention is an immunostimulatory oligonucleotide with a 5' TLR activation domain and at least two complementarity-containing regions, a 5' and a 3' complementarity-containing region, each complementarity-containing region being at least 8 nucleotides in length and connected to one another through a spacer, wherein the oligonucleotide includes at least one unmethylated CpG dinucleotide, and wherein at least one of the complementarity-containing regions is not a perfect palindrome, and wherein the TLR activation domain is immediately 5' to the 5' complementarity-containing region, wherein the oligonucleotide has the formula 5' XNSPT 3' or the formula 5' XPSNT 3', wherein X is the TLR activation domain, N is a non-perfect palindrome, P is a palindrome, S is a spacer, and T is a 3' tail of 0-100 nucleotides in length. In another embodiment the TLR activation domain is TCG, TTCG, TTTCG, TYpR, TTYpR, TTTYpR, UCG, UUCG, UUUCG, TTT, or TTTT. In another embodiment the 3' complementarity-containing region is at least 10 nucleotides in length. In yet another embodiment the 5' complementarity-containing region is at least 10 nucleotides in length. In one embodiment the 3' complementarity-containing region includes an unmethylated CpG dinucleotide. In another embodiment the 3' complementarity-containing region includes two unmethylated CpG dinucleotides. In yet another embodiment the 5' complementarity-containing region includes an unmethylated CpG dinucleotide. In another embodiment the 5' complementarity-containing region includes two unmethylated CpG dinucleotides. In another embodiment the complementarity-containing regions include at least one nucleotide analog. In another embodiment the complementarity-containing regions form an intramolecular duplex.

In one embodiment the intramolecular duplex includes at least one non-Watson Crick base pair. In another embodiment the non-Watson Crick base pair is G-T, G-A, G-G, or C-A. In one embodiment the complementarity-containing regions form intermolecular duplexes. In another embodiment at least one of the intermolecular duplexes includes at least one non-Watson Crick base pair. In another embodiment the non-Watson Crick base pair is G-T, G-A, G-G, or C-A. In yet another embodiment the complementarity-containing regions contain a mismatch. In still another embodiment the complementarity-containing regions contain two mismatches. In another embodiment the complementarity-containing regions contain an intervening nucleotide. In another embodiment the complementarity-containing regions contain two intervening nucleotides.

In one embodiment the 5' and 3' complementarity-containing regions have a duplex stability value of at least 25. In another embodiment the 5' and 3' complementarity-containing regions have a duplex stability value of at least 30. In another embodiment the 5' and 3' complementarity-containing regions have a duplex stability value of at least 35. In another embodiment the complementarity-containing regions have a duplex stability value of at least 40. In another embodiment the complementarity-containing regions have a duplex stability value of at least 45. In another embodiment the complementarity-containing regions have a duplex stability value of at least 50. In another embodiment the complementarity-containing regions have a duplex stability value of at least 55. In another embodiment the complementarity-containing regions have a duplex stability value of at least 60. In another embodiment the complementarity-containing regions have a duplex stability value of at least 65.

In another embodiment X is TCG, TTCG, or TTTCG. In another embodiment T is 5-50 nucleotides in length. In another embodiment T is 5-10 nucleotides in length. In another embodiment S is a nucleic acid having a length of 1-50 nucleotides. In another embodiment S is a nucleic acid having a length of 1 nucleotide. In another embodiment S is a non-nucleotide spacer. In another embodiment the non-nucleotide spacer is a D-spacer. In another embodiment the non-nucleotide spacer is a linker. In another embodiment the oligonucleotide is not an antisense oligonucleotide or a ribozyme. In another embodiment N is A and T rich. In another embodiment N is includes at least 4 Ts. In another embodiment P is a perfect palindrome. In another embodiment P is G-C rich. In another embodiment P is CGGCGCX₁GCGCCG (SEQ ID NO:334), wherein X₁ is T or nothing. In another embodiment the oligonucleotide includes at least one phosphorothioate linkage. In another embodiment all internucleotide linkages of the oligonucleotide are phosphorothioate linkages. In another embodiment the oligonucleotide includes at least one phosphodiester-like linkage. In another embodiment the phosphodiester-like linkage is a phosphodiester linkage. In another embodiment a lipophilic group is conjugated to the oligonucleotide. In one embodiment the lipophilic group is cholesterol.

Another aspect of the invention provides for therapeutic compositions of the aforementioned oligonucleotides. In one embodiment the composition includes a mixture of duplex forming immunostimulatory oligonucleotides formulated in a low salt buffer and including a solute. In one embodiment the solute is an amino acid. In one embodiment the amino acid has a hydrophobic side chain. In another embodiment the amino acid is isoleucine. In still another embodiment the amino acid is glycine. In another embodiment the amino acid has a charged side chain. In another embodiment the solute is an alcohol. In one embodiment the alcohol is a saccharide. In some embodiments the saccharide is dextrose, fructose, lactose, sucrose, ribose, arabinose or a disaccharide. In one embodiment the duplex forming immunostimulatory oligonucleotides are any of the aforementioned oligonucleotides. In another embodiment the composition includes at least two different duplex forming immunostimulatory oligonucleotides having different nucleotide sequences. In another embodiment the composition includes at least two duplex forming immunostimulatory oligonucleotides having the same nucleotide sequences as one another. In another embodiment each duplex forming immunostimulatory oligonucleotide includes at least one duplex forming sequence. In another embodiment each duplex forming immunostimulatory oligonucleotide includes at least two duplex forming sequences. In another embodiment each duplex forming sequence has a duplex stability value of at least 25. In another embodiment each duplex forming sequence has a duplex stability value of at least 30. In another embodiment each duplex forming sequence has a duplex stability value of at least 35. In another embodiment the duplex forming immunostimulatory oligonucleotides include at least one poly G sequence having 4 consecutive G nucleotides.

Another aspect of the disclosure is a method for preparing a substantially homogenous mixture of oligonucleotides, by identifying duplex forming immunostimulatory oligonucleotides and formulating the duplex forming immunostimulatory oligonucleotides in a low salt buffer and a solute to produce a substantially homogenous mixture of oligonucleotides.

Another aspect of the disclosure is a composition of a mixture of at least two different duplex forming immunostimulatory oligonucleotides, wherein the at least two different duplex forming immunostimulatory oligonucleotides each have a 5' TLR activation domain including an unmethylated CpG dinucleotide and a 3' duplex forming sequence of at least 8 nucleotides in length, wherein the 3' duplex forming sequence of each of the at least two different duplex forming immunostimulatory oligonucleotides are complementary to one another, and wherein the at least two different duplex forming immunostimulatory oligonucleotides are 11-100 nucleotides in length. In one embodiment the composition includes a low salt buffer and a solute. In another embodiment the TLR activation domain is TCG, TTCG, or TTTCG. In one embodiment the TLR activation domain is connected directly to the 3' duplex forming sequence. In another embodiment the two palindromic regions are connected via a 3'-3' linkage. In another embodiment the TLR activation domain and the 3' duplex forming sequence are connected through a spacer. In one embodiment the spacer is a nucleic acid having a length of 1-50 nucleotides. In another embodiment the spacer is a nucleic acid having a length of 1 nucleotide. In yet another embodiment the spacer is a non-nucleotide spacer. In yet another embodiment the non-nucleotide spacer is a D-spacer. In another embodiment the non-nucleotide spacer is a linker. In one embodiment the oligonucleotide includes at least one phosphorothioate linkage. In another embodiment all internucleotide linkages of the oligonucleotide are phosphorothioate linkages. In another embodiment the oligonucleotide includes at least one phosphodiester-like linkage. In another embodiment the phosphodiester-like linkage is a phosphodiester linkage. In another embodiment a lipophilic group is conjugated to the oligonucleotide. In another embodiment the lipophilic group is cholesterol.

Another aspect of the disclosure is a method for treating cancer by administering to a subject in need thereof any one of the aforementioned oligonucleotides or any of the aforementioned compositions in an effective amount to treat the cancer. In one embodiment the anti-cancer treatment is administered to the subject. In another embodiment the anti-cancer treatment is chemotherapy. In another embodiment the anti-cancer treatment is radiation. In another embodiment the anti-cancer treatment includes an antibody.

Another aspect of the disclosure is a method for treating asthma by administering to a subject in need thereof any one of the aforementioned oligonucleotides or any of the aforementioned compositions in an effective amount to treat asthma. In one embodiment an additional asthma treatment is co-administered to the subject.

Another aspect of the disclosure is a method for treating allergy by administering to a subject in need any one of the aforementioned oligonucleotides or any of the aforementioned compositions in an effective amount to treat allergy. In one embodiment an additional allergy treatment is administered to the subject. In one embodiment the subject has allergic rhinitis. In another embodiment the subject has ocular allergy.

Another aspect of the disclosure is a method for modulating an immune response in a subject by administering to a subject in need thereof any one of the aforementioned oligonucleotides or any of the aforementioned compositions in an effective amount to modulate an immune response. In one embodiment an additional immune modulator is administered to the subject. In another embodiment the oligonucleotide or composition is delivered to the subject to treat autoimmune disease in the subject. In another embodiment, the oligonucleotide or composition is delivered to the subject to treat an inflammatory disease in the subject. In another embodiment the oligonucleotide or composition is delivered to the subject to treat airway remodeling in the subject. In another embodiment the oligonucleotide or composition is administered without an antigen to the subject. In yet another embodiment the oligonucleotide or composition is delivered by a route selected from the group consisting of oral, nasal, sublingual, intravenous, subcutaneous, mucosal, ocular, respiratory, direct injection, and dermally. In another embodiment the oligonucleotide or composition is delivered to the subject in an effective amount to induce cytokine and/or chemokine expression. In another embodiment the cytokine and/or chemokine is selected from the group consisting of IFN-α, IFN-β, IL-28, IL-29, IFN-ω, TNF-α, IL-10, IL-6, IFN-γ, IP-10, MCP-1, and IL-12.

Another aspect of the disclosure is a method for treating asthma exacerbated by viral infection by administering to a subject in need thereof any one of the aforementioned oligonucleotides or any of the aforementioned compositions in an effective amount to treat the asthma exacerbated by viral infection.

Another aspect of the disclosure is a method for treating infectious disease by administering to a subject in need thereof any one of the aforementioned oligonucleotides or any of the aforementioned compositions in an effective amount to treat the infectious disease. In one embodiment the subject has a viral infection. In one embodiment the viral infection is caused by hepatitis B virus (HBV), hepatitis C virus (HCV), human immunodeficiency virus (HIV), influenza virus, respiratory syncytial virus (RSV) or human papilloma virus (HPV). In another embodiment an anti-viral agent is co-administered to the subject. In one embodiment the anti-viral agent is linked to the oligonucleotide. In another embodiment the oligonucleotide or composition is delivered by a route selected from the group consisting of oral, nasal, sublingual, intravenous, subcutaneous, mucosal, ocular, respiratory, direct injection, and dermally.

The present disclosure also contemplates a use of any of the immunostimulatory oligonucleotides of the invention or compositions of the invention for the treatment of diseases described herein, for instance, cancer, infectious disease, asthma, allergy, inflammatory disorders, and autoimmune diseases.

The invention also contemplates a use of any of the immunostimulatory oligonucleotides of the invention or compositions of the invention in the manufacture of a medicament for the treatment of diseases described herein, for instance, cancer, infectious disease, asthma, allergy, inflammatory disorders, and autoimmune diseases.

### BRIEF DESCRIPTION OF THE DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:
Figure 1 is two graphs showing the induction of IFN-α in relation to amount of oligonucleotide in a comparison of a P-Class oligonucleotide containing two palindromes to a C-class oligonucleotide containing one 3' palindrome. The y-axes are the amount of IFN-α in pg/ml and the x-axes are concentration of oligonucleotide in µM.
Figure 2 is two graphs showing the induction of IFN-α in relation to amount of oligonucleotide in an analysis of the effectiveness of palindrome length. The y-axes are the amount of IFN-α in pg/ml and the x-axes are concentration of oligonucleotide in µM.
Figure 3 is two graphs showing the induction of IFN-α in relation to amount of oligonucleotide in an analysis of duplex forming regions such as imperfect palindromes. The y-axes are the amount of IFN-α in pg/ml and the x-axes are concentration of oligonucleotide in µM.
Figure 4 is two graphs showing stimulation of Th1-like cytokine and chemokine responses *in vivo* in mice after treatment with conventional C-Class ODN. Figure 4A shows IL-12 induction and Figure 4B shows IP-10 induction. The y-axes represent induction in pg/ml and the x axis represents the ODN.
Figure 5 is two graphs showing IFN-α induction *in vivo* in mice in response to C-class and P-Class ODN. Figure 5A shows IFN-α induction after subcutaneous (SC) administration of ODN and Figure 5B shows IFN-α induction after intravenous (IV) administration of ODN. The y-axes represent IFN-α induction and the x-axes represent the ODN used.
Figure 6 is a graph showing a comparison of anti-HBs response following A, B, C, and P class ODN stimulation *in vivo*. The y-axis represents anti-HBs and the x-axis represents the ODN used.
Figure 7 is a diagram depicting a concatamer formed by hybridization of a P-Class oligonucleotide containing two palindromic regions.
Figure 8 is a graph showing dimer formation of SEQ ID NO:234 in phosphate solution with a variety of additives. The y-axis is % dimer formation and the x-axis indicates the different additives.
Figure 9 is two graphs showing IFN-α induction *in vivo* in mice in response to A-, B-, C- and P-Class ODN. Figure 9A shows IFN-α induction after SC administration of ODN and Figure 9B shows IFN-α induction after IV administration. The y-axes represent IFN-α in pg/ml and the x-axes represent the ODN used.
Figure 10 is three graphs showing the induction of IFN-α in relation to amount of oligonucleotide in analysis of the effect of the addition of linkers. Figure 10A shows IFN-α induction, figure 10b shows IL-10 induction, and figure 10c shows IL-6 induction. The y axes are cytokine concentration in pg/ml and the x-axes are concentration of oligonucleotide in µM.
Figure 11 is a graph showing the induction of IFN-α in relation to amount of oligonucleotide in analysis of the effect of sugar modification of the P-class ODN. The y axis is IFN-α concentration in pg/ml and the x-axis is concentration of oligonucleotide in µM.

### DETAILED DESCRIPTION OF THE INVENTION

A new class of immunostimulatory oligonucleotides, referred to herein as P-class oligonucleotides, capable of inducing high levels of IFN-α has been discovered. C-Class CpG oligonucleotides, which contain a single palindrome in or near the 3' half of the oligonucleotide, are known to induce both strong B-cell proliferation and IFN-α production. The P-Class oligonucleotides of the invention, like C-class oligonucleotides, induce B cell activation and IFN-α production, but are capable of producing, in some instances, much higher levels of IFN-α than C-class oligonucleotides. The 3'-palindromic sequence of C-class oligonucleotides is thought to be required for the specific immunostimulatory profile observed for the C-class oligonucleotides, most likely because of the formation of dimers with 2 free 5' ends. The 5' end of a CpG ODN is thought to be the region that is most important for activation of the TLR9 receptor, and two free 5' ends in a single ODN may induce cross-linking of two TLR9 receptors. Cross-linking of TLR9 receptors may induce activation of stronger IFN-α secretion through the type I IFNR feedback loop in plasmacytoid dendritic cells.

Surprisingly it was discovered that a new class of oligonucleotides, which are not optimized to maintain free 5' ends, are capable of inducing high IFN-α. The P-Class oligonucleotides of the invention have two duplex forming regions; one near the 5' end, and the other closer to or in the 3' half of the ODN. It is believed that the oligonucleotide design leads to the formation of complex higher-ordered structures called concatamers. Concatamer formation can be observed by size exclusion chromatography (SEC) of the oligonucleotide in solution under physiologic or high salt conditions. Although the invention is not limited by a particular mechanism, it is thought that these structures may function by causing a high degree of TLR9 crosslinking, resulting in even stronger activation of plasmacytoid dendritic cell IFN-α secretion through the type I IFNR feedback loop. It is also possible that these higher-ordered structures result in the recruitment of additional cofactors or adapter molecules to the TLR9 signaling complex. Another possibility is a different intracellular distribution of the concatamer ODN due to the higher-ordered structures.

The immunostimulatory oligonucleotides of this invention can be used to treat diseases in which Th1-like immune stimulation or immune modulation would be of advantage. Applications include but are not limited to autoimmune diseases, inflammatory disorders, infectious diseases, cancer, asthma and allergies. Because of the ability to induce high levels of IFN-α and Th1 and Th1-like cytokines the treatment of viral diseases, such as Hepatitis B and C, Cytomegalovirus (CMV), Papilloma Virus, HIV and Herpes simplex viruses (HSV) are of particular interest. The oligonucleotides of the invention are also useful as vaccine adjuvants. The compounds of the present invention can be used for prophylaxis and therapy, either as a stand alone therapy or in combination with other therapeutics or medical devices.

In general, the immunostimulatory oligonucleotides of the invention have several domains, including a 5'TLR activation domain, 2 duplex forming regions and a spacer and a 3' tail.

The term "duplex forming region" as used herein is defined as a region capable of forming a duplex with another duplex forming region. Such regions can comprise palindromes, complementarity-containing regions, imperfect palindromes or non-palindromic regions that are able to form intermolecular Watson-Crick or non-Watson-Crick base pairs with a complementary region of a second oligonucleotide.

In some instances the immunostimulatory oligonucleotides can form secondary structures arising from the formation of intramolecular duplexes. As used herein an "intramolecular duplex" is formed when multiple duplex forming regions on a single molecule form a duplex with each other. Often in such cases the regions will be connected through a spacer. In some instances the immunostimulatory oligonucleotides can form intermolecular duplexes. As used herein an "intermolecular duplex" is formed when the duplex forming regions are on different molecules and form base pair interactions with each other that connect the molecules. In some instances an intermolecular duplex forms between the two oligonucleotides having the same sequence. In other instances the intermolecular duplex forms between different oligonucleotides having different nucleotide sequences. In some instances the immunostimulatory oligonucleotides can form both intermolecular and intramolecular duplexes.

The duplex forming regions may be palindromes. A "palindrome" and, equivalently, "palindromic region" as used herein refers to a nucleic acid sequence which is its own perfect reverse complement (i.e., a sequence such as ABCDEE'D'C'B'A' in which A and A', B and B', C and C', D and D', and E and E' are bases capable of forming the usual Watson-Crick base pairs, i.e., G-C, A-T, and A-U). As used herein, a "palindrome" in a strict sense excludes intervening sequence or intervening non-nucleotide structure that does not participate in forming the usual Watson-Crick base pairs.

The palindrome may be a 3' or 5' palindrome. The two palindromes may be the same or they may be distinct. Thus, the 5' palindromic region and the 3' palindromic region need not be complementary to one another. In fact they may be completely distinct and only pair with palindromic regions in other oligonucleotides rather than in the same oligonucleotide. Alternatively the palindromes may be a match such that they can form an intramolecular base pair interaction. Both palindromes can have various base compositions (A, T, G or C), although in some embodiments a higher GC content is preferred for one palindrome (either 3' or 5').

The two palindromic regions may have different duplex stability values. The duplex stability value is indicative of the strength of the duplex formed by the palindrome with its pair in a second oligonucleotide in an intermolecular pairing or with itself or the second palindrome in an intramolecular pairing. As used herein, "duplex stability" is a measure of the strength of a palindromic, complementarity-containing or duplex forming region when forming a duplex with its own complementary sequence. The measure of duplex stability of a double stranded molecule is dependent on total strand concentration, base composition, temperature, pH and buffer salts. A duplex stability value can be calculated by the thermodynamic model developed by John SantaLucia, Jr. (1998) Proc. Natl. Acad. Sci. 95 1460 - 1465. This folding program is e.g. available at http://lna-tm.com/or at http://www.bioinfo.rpi.edu/applications/hybrid/twostate.php.

An example of a calculation of duplex stability is based on a 0.1 µM oligonucleotide total strand concentration and 140 mM salt concentration (approximate physiologic salt). The melting temperature (Tm) prediction is DNA oligonucleotide hybridized against perfect match DNA nucleotides in-solution. An example of the calculation using SEQ ID No. 234 is shown below.

| | | |
|---|---|---|
| SEQ ID NO:234 | TCGTCGACGATCGGCGCGCGCCG | |
| 5' palindrome | TCGTCGACGA | Tm: 41°C |
| 3' palindrome | CGGCGCGCGCCG | Tm: 68°C |

In the case of phosphorothioate modification, the predicted Tm is depressed by approximately 1°C per modification. Thus, in a fully phosphorothioate molecule the duplex stability corrected for this modification would be

| | | |
|---|---|---|
| 5' palindrome | TCGTCGACGA | Tm: 32°C |
| 3' palindrome | CGGCGCGCGCCG | Tm: 57°C |

The actual measured Tm will vary within a 5-10 °C range. For instance the actual measured Tm for SEQ ID NO:234 was (0.04mg/ml ODN in PBS):

| | | |
|---|---|---|
| 5' palindrome | TCGTCGACGA | Tm: 33.9°C |
| 3' palindrome | CGGCGCGCGCCG | Tm: 65.7°C |

Although the SantaLucia formula is useful for calculating duplex stability of oligonucleotides it is artificially low for some oligonucleotides that form hairpin structures. For prediction of the stability of hairpins, the Mfold algorithm is used for nucleic acid folding and hybridization prediction as described by M. Zuker Nucleic Acids Res. 31(13), 3406-15, (2003) which is available at http://www.bioinfo.rpi.edu/applications/mfold/old/dna/form1.cgi.

| | |
|---|---|
| SEQ ID NO:237 | TCGTCGACGTTCGGCGCCGTGCCG |

3' palindrome CGGCGCCGTGCCG Tm: 73°C for hairpin with 4 Watson-Crick base pairs

3' palindrome CGGCGCCGTGCCG Tm: 73°C for hairpin with 4 Watson-Crick base pairs bp and G-T base pairs

The corresponding dimer has a calculated Tm of 42 °C and is thus less favored than the intramolecular structures.

A "weak duplex" is considered to have a duplex stability value of at least 25 to 40. A "strong duplex" is considered to have a duplex stability value of at least 40 to 60. Intramolecular duplexes, such as hairpins, usually require fewer base pairs to obtain the same duplex stability value as compared to intermolecular duplexes. In addition, intramolecular duplex stability (e.g. stability of a hairpin) is independent of ODN strand concentration.

In some embodiments the 5' palindromic region is weaker than the 3' palindromic region on the same molecule. Thus, the 5' palindromic region may have a lower duplex stability when complexed with itself than the 3' palindromic region, possibly due to a lower GC content. Alternatively, in some cases the 5' palindrome can have a higher duplex stability than the 3' palindrome.

A "complementarity-containing region", as used herein, refers to a duplex forming region that comprises a perfect palindrome or an imperfect palindrome. An imperfect palindrome is a nucleic acid sequence which includes both nucleotides capable of forming the usual Watson-Crick base pairs and nucleotides, nucleotide analogs, or other structures that do not participate in forming the usual Watson-Crick base pairs (e.g., a sequence such as ABCDE-S-E'D'C'B'A' in which A and A', B and B', C and C', D and D', and E and E' are bases capable of forming the usual Watson-Crick base pairs, and S is a non-palindromic sequence or a non-nucleotidic linker or an abasic linker (dSpacer)). Examples of non-nucleotidic linkers include but are not limited to diols such as 1,3-propane diol or dodecane-1,12-diol, cyclohexanediol, or linkers such as cholesterol, nitroindol, triethylene glycol and hexaethylene glycol. In certain embodiments the nucleotides, nucleotide analogs, or other structures that do not participate in forming the usual Watson-Crick base pairs interrupt an otherwise perfect palindrome. In certain embodiments the nucleotides that do not participate in forming the usual Watson-Crick base pairs can form non-Watson-Crick base pairs with another nucleotide, e.g., G-T. A non-Watson-Crick base pair as used herein is any base pair other than a Watson-Crick base pair, including but not limited to a Hoogsteen base pair and a so-called wobble base pair. In certain embodiments the nucleotides that do not participate in forming the usual Watson-Crick base pairs are unmatched and have no nucleotide base or nucleotide base analog with which to form a Watson-Crick or non-Watson-Crick base pair, e.g., G opposite to dSpacer. In some embodiments the non-Watson Crick base pair is G-T, G-A, G-G, or C-A. G-T is a preferred non-Watson-Crick base pair because it has less destabilizing effect on duplex formation. In certain embodiments the nucleotides that do not participate in forming base pairs can form non-standard base pairs with another nucleotide, e.g., diaminopyridine can form a base pair with xanthosine. In some instances the doublestranded part of the molecule may also contain unnatural (non-standard) base pairs (e.g., diaminopyridine paired with xanthosine). Lutz MJ et al. (1998) Recognition of a non-standard base pair by thermostable DNA polymerases. Bioorg Med Chem Lett 8:1149-52.

In certain embodiments the complementarity-containing region can contain a mismatch. A "mismatch" as used herein refers to a portion of the complementarity-containing region in which one or more bases in the sequence does not form a usual Watson-Crick base pair with its opposite base in a duplex. A mismatch can result in a "bulge" in which a portion of the complementarity-containing region does not participate in the duplex formation. In some embodiments the complementarity-containing region can contain two mismatches.

In one embodiment an imperfect palindrome is an "inverted repeat capable of forming a hairpin or stem-loop structure". This type of structure may include a sequence of nucleotides that forms a GC-rich stem or hairpin that is 3 to 10 consecutive base pairs long, and includes at least one unmatched or mismatched base. In individual embodiments the GC-rich stem is 2, 3, 4, 5, 6, 7, 8, 9, or 10 consecutive base pairs long. In some embodiments the GC-rich stem includes at least 2, 3, or 4 G-C base pairs. In another embodiment an inverted repeat capable of forming a hairpin or stem-loop structure refers to a sequence of nucleotides that forms an AT-rich stem or hairpin that is 2 to 10 consecutive base pairs long, and includes at least one unmatched or mismatched base. In individual embodiments the AT-rich stem is 3, 4, 5, 6, 7, 8, 9, or 10 consecutive base pairs long. In some embodiments the AT-rich stem includes at least 3, 4, 5, or 6 A-T base pairs.

In some instances the at least one unmatched or mismatched base bridges the ends of the stem or hairpin. This may allow the formation of the secondary structure by providing a flexible point in the molecule for the stems to base pair and form a hairpin. Alternatively the unmatched or mismatched base(s) may be within the stem. Preferably if the mismatched base is within the stem, then the stem is at least 3 base pairs long. The unmatched or mismatched bases(s) may be any nucleotide. In some embodiments the unmatched or mismatched base is a T. Unmatched nucleotides at the end of double-strands are also known as overhanging nucleotides or dangling ends which can significantly stabilize duplex formation or hairpin formation. Freier SM et al. (1983) Effects of 3' dangling end stacking on the stability of GGCC and CCGG double helixes. Biochemistry 22:6198-206.

The complementarity-containing region typically has either a duplex stability value of at least 20, or it may include fewer than 5 mismatches/10 base pair region and/or 1-5 extra-palindromic (bulge forming/intervening) nucleotides/10 base pair region.

The duplex forming regions may include one or more immune stimulatory domains such as Toll-like receptor 9 (TLR9) activation domains. The oligonucleotide includes at least one TLR9 activation domain, positioned at the 5'end of the molecule. In some embodiments the 5' TLR9 activation domain is encompassed partially or completely within the 5' duplex forming region, such that it forms part or all of the duplex forming region. Alternatively the 5' TLR9 activation domain may be distinct from the 5' duplex forming region. When these two domains are distinct, they may be connected directly to one another with an internucleotide bond or they may be separated by a spacer, such as a nucleotidic linker or non-nucleotidic linker.

A TLR9 activation domain includes any sequence motif that is immune stimulatory, producing a pattern of immune stimulation consistent with the immune activation patterns observed with TLR9 receptor activation. These motifs include but are not limited to YpR, CpG, TCG, TTCG, TTTCG, TYpR, UCG, TCG, TTYpR, TTTYpR, UUCG, UUUCG, TTT, TTTT, methylated CpG, and CpI. The nucleotides of the motif may include a semi-soft or stereo-specific backbone.

Embodiments of the CpG oligonucleotides of the invention may be depicted by the following formulas:
5' XP₁SP₂T 3'
and
5' XNSPT 3'
and
5' XPSNT 3'
And
5' XN₁SN₂T 3'

X is a TLR activation domain. P, P₁ and P₂ are palindromes. S is a spacer. T is a 3' tail. N, N₁, and N₂ are complementarity containing regions that comprise imperfect palindromes. In the formulas 5' refers to the free 5' end of the oligonucleotide and 3' refers to the free 3' end of the oligonucleotide.

The immunostimulatory oligonucleotides of the invention can include A and T rich regions or G and C rich regions. An "A and T rich region" as used herein is one in which the A and T nucleotides outnumber the G and C nucleotides in the sequence. Alternatively, a "G and C rich region" as used herein is one in which the G and C nucleotides outnumber the A and T nucleotides in the sequence. In some cases the oligonucleotides can have four or more G nucleotides near the 3' end of the molecule.

The molecule includes a 3' tail. The 3' tail has a lengthof 0 to 100 nucleotides. This 3' tail can be of any base content. In some embodiments the 3' tail contains one or more immune stimulatory domains such as poly T or CpG motifs.

A spacer is located between the two duplex forming regions. The spacer may be a flexible linker that is either a non-nucleotidic linker or "intervening nucleotides", i.e., nucleotides that do not form duplexes. The "intervening nucleotides" can include from 0-100 nucleotides. When the spacer is a nucleic acid spacer it may be any nucleotide or nucleotides, or nucleoside(s). In some embodiments it is a T or T rich spacer. A "non-nucleotidic linker" or equivalently "non-nucleotidic spacer" as used herein refers to any linker element that is not a nucleotide or polymer thereof (i.e., a polynucleotide), wherein a nucleotide includes a purine or pyrimidine nucleobase and a sugar phosphate. A non-nucleotidic linker thus is any linker known in the art, including but not limited to a simple carbon chain, an abasic nucleotide (dSpacer), i.e., a nucleotide-like sugar phosphate unit in which the nucleobase is replaced by a hydrogen atom, a polyethyleneglycol, including but not limited to a triethyleneglcol and a hexaethylene glycol. The spacer can include one or more immune stimulatory domains such as poly T or CpG motifs. In some embodiments the linker is a 3'-3' linkage between the duplex forming regions.

In general, the duplex forming regions can be connected directly or indirectly. The term "connected directly", as used herein, refers to an oligonucleotide in which the nucleosides of the palindrome are attached by a phosphodiester, phosphodiester-like, or phosphorothioate chemical bond. It is possible for two duplex forming regions to overlap. The duplex forming regions may overlap by one or two nucleotides. When the duplex forming regions overlap they are considered to be connected directly. In some embodiments the duplex forming regions do not overlap. The term "connected indirectly", as used herein, refers to an oligonucleotide in which the nucleosides of the duplex forming region are connected through a spacer, as described above.

The oligonucleotides of the invention include at least one YpR dinucleotide. As used herein a "YpR dinucleotide" is one in which a pyrimidine is followed by a purine. In certain embodiments the letter Y is used to refer to a nucleotide containing a cytosine or a modified cytosine. A modified cytosine as used herein is a naturally occurring or non-naturally occurring pyrimidine base analog of cytosine which can replace this base without impairing the immunostimulatory activity of the oligonucleotide. Modified cytosines include but are not limited to 5-substituted cytosines (e.g. 5-methyl-cytosine, 5-fluoro-cytosine, 5-chloro-cytosine, 5-bromo-cytosine, 5-iodo-cytosine, 5-hydroxy-cytosine, 5-hydroxymethyl-cytosine, 5-difluoromethyl-cytosine, and unsubstituted or substituted 5-alkynyl-cytosine), 6-substituted cytosines, N4-substituted cytosines (e.g. N4-ethyl-cytosine), 5-aza-cytosine, 2-mercapto-cytosine, isocytosine, pseudo-isocytosine, cytosine analogs with condensed ring systems (e.g. N,N'-propylene cytosine or phenoxazine), and uracil and its derivatives (e.g. 5-fluoro-uracil, 5-bromo-uracil, 5-bromovinyl-uracil, 4-thio-uracil, 5-hydroxy-uracil, 5-propynyl-uracil). Some of the preferred cytosines include 5-methyl-cytosine, 5-fluoro-cytosine, 5-hydroxy-cytosine, 5-hydroxymethyl-cytosine, and N4-ethyl-cytosine. In another embodiment of the invention, the cytosine base is substituted by a universal base (e.g. 3-nitropyrrole, P-base), an aromatic ring system (e.g. fluorobenzene or difluorobenzene) or a hydrogen atom (dSpacer).

The letter R is used to refer to a purine, including for instance G and A. In some embodiments R is Z, wherein Z is used to refer to guanine or a modified guanine base. A modified guanine as used herein is a naturally occurring or non-naturally occurring purine base analog of guanine which can replace this base without impairing the immunostimulatory activity of the oligonucleotide. Modified guanines include but are not limited to 7-deazaguanine, 7-deaza-7-substituted guanine (such as 7-deaza-7-(C2-C6)alkynylguanine), 7-deaza-8-substituted guanine, hypoxanthine, N2-substituted guanines (e.g. N2-methyl-guanine), 5-amino-3-methyl-3H,6H-thiazolo[4,5-d]pyrimidine-2,7-dione, 2,6-diaminopurine, 2-aminopurine, purine, indole, adenine, substituted adenines (e.g. N6-methyl-adenine, 8-oxo-adenine) 8-substituted guanine (e.g. 8-hydroxyguanine and 8-bromoguanine), and 6-thioguanine. In another embodiment of the invention, the guanine base is substituted by a universal base (e.g. 4-methyl-indole, 5-nitro-indole, and K-base), an aromatic ring system (e.g. benzimidazole or dichloro- benzimidazole, 1-methyl-1H-[1,2,4]triazole-3-carboxylic acid amide) or a hydrogen atom (dSpacer).

In certain embodiments of the invention the immunostimulatory oligonucleotides include a YpR motif that is a CpG dinucleotide. A CpG dinucleotide can be methylated or unmethylated. An immunostimulatory oligonucleotide containing at least one unmethylated CpG dinucleotide is a nucleic acid molecule which contains an unmethylated cytosine-guanine dinucleotide sequence (i.e., an unmethylated 5' cytidine followed by 3' guanosine and linked by a phosphate bond) and which activates the immune system; such an immunostimulatory oligonucleotide is a CpG oligonucleotide. CpG oligonucleotides have been described in a number of issued patents, published patent applications, and other publications, including U.S. Patent Nos. 6,194,388; 6,207,646; 6,214,806; 6,218,371; 6,239,116; and 6,339,068. An immunostimulatory oligonucleotide containing at least one methylated CpG dinucleotide is an oligonucleotide which contains a methylated cytosine-guanine dinucleotide sequence (i.e., a methylated 5' cytidine followed by a 3' guanosine and linked by a phosphate bond) and which activates the immune system.

In some embodiments the oligonucleotide has one of the structures shown in Table 1:

**Table 1. Oligonucleotide Sequences**

| | |
|---|---|
| T-C-G-T-C-G-A-C-G-A-T*T*T*T-A-C-G-A-C-G-T-C-G-T-T*T*T | SEQ ID NO:1 |
| T-C-G-T-C-G-A-C-G-A-T-T-T-T-A-C-G-A-C-G-T-C-G-T-T-T-T | SEQ ID NO:2 |
| T-C-G-T-C-G-A-C-G-A-A-C-G-A-C-G-T-C-G-T | SEQ ID NO:3 |
| T-C-G-T-C-G-A-C-G-A-T*T*T*T-T-C-G-T-C-G-A-C-G-A-T*T*T | SEQ ID NO:4 |
| T-C-G-T-C-G-A-C-G-A-T-T-T-T-T-C-G-T-C-G-A-C-G-A-T-T-T | SEQ ID NO:5 |
| T-C-G-T-C-G-A-C-G-A-T-C-G-T-C-G-A-C-G-A | SEQ ID NO:6 |
| C*G*C*G*C*G*C*G*C*G*C*G*C*G*C*G*C*G*C*G | SEQ ID NO:7 |
| G*A*G*A*A*C*G*C*T*C*G*A*C*C*T*T*C*G*A*T*biot | SEQ ID NO:8 |
| A*G*C*T*C*C*A*T*G*G*T*G*C*T*C*A*C*T*G | SEQ ID NO:9 |
| T*C*T*C*C*C*A*G*C*G*T*G*C*G*C*C*A*T | SEQ ID NO:10 |
| T*C*C*A*T*G*A*C*G*T*T*C*C*T*G*A*C*G*T*T | SEQ ID NO:11 |
| T*C*C*A*G*G*A*C*T*T*C*T*C*T*C*A*G*G*T*T | SEQ ID NO:12 |
| T*C*C*A*C*G*A*C*G*T*T*T*T*C*G*A*C*G*T*T | SEQ ID NO:13 |
| T*C*G*T*C*G*T*T*T*T*G*A*C*G*T*T*T*T*G*A*C*G*T*T | SEQ ID NO:14 |
| T*C*C*T*G*A*C*G*T*T*C*G*G*C*G*C*G*C*G*C*C*C | SEQ ID NO:15 |
| T*C*G*C*G*T*G*C*G*T*T*T*T*G*T*C*G*T*T*T*T*G*A*C*G*T*T | SEQ ID NO:16 |
| T*C*G*C*G*A*C*G*T*T*C*G*G*C*G*C*G*C*G*C*C*G | SEQ ID NO:17 |
| dig-C*C*G*G*C*C*G*G*C*C*G*G*C*C*G*G*C*C*G*G | SEQ ID NO:18 |
| dig-C*G*C*G*C*G*C*G*C*G*C*G*C*G*C*G*C*G*C*G | SEQ ID NO:19 |
| T*C*C*A*G*G*A*C*T*T*C*T*C*T*C*A*G*G*T*T*T*T*T*T | SEQ ID NO:20 |
| G*T*G*C*T*C*G*A*G*G*A*T*G*C*G*C*T*T*C*G*C | SEQ ID NO:21 |
| G*C*C*G*A*G*G*T*C*C*A*T*G*T*C*G*T*A*C*G*C | SEQ ID NO:22 |
| T-C-G-C-G-T-G-C-G-T-T-T-T-G-T-C-G-T-T-T-T-G-A-C-G-T-T | SEQ ID NO:23 |
| A*C*C*G*A*T*A*C*C*G*G*T*G*C*C*G*G*T*G*A*C*G*G*C*A*C*C*A*C*G | SEQ ID NO:24 |
| A*C*C*G*A*T*A*A*C*G*T*T*G*C*C*G*G*T*G*A*C*G*G*C*A*C*C*A*C*G | SEQ ID NO:25 |
| A*C*C*G*A*T*G*A*C*G*T*C*G*C*C*G*G*T*G*A*C*G*G*C*A*C*C*A*C*G | SEQ ID NO:26 |
| C*G*G*C*G*C*G*C*G*C*C*G*C*G*G*C*G*C*G*C*G*C*C*G | SEQ ID NO:27 |
| T*C*G*A*T*C*G*T*T*T*T*T*C*G*T*G*C*G*T*T*T*T*T | SEQ ID NO:28 |
| T*C*G*T*C*C*A*G*G*A*C*T*T*C*T*C*T*C*A*G*G*T*T | SEQ ID NO:29 |
| T*C*G*T*C*G*T*C*C*A*G*G*A*C*T*T*C*T*C*T*C*A*G*G*T*T | SEQ ID NO:30 |
| T*C*G*T*G*A*C*G*G*G*C*G*G*C*G*C*G*C*G*C*C*C | SEQ ID NO:31 |
| A*C*G*A*C*G*T*C*G*T*tC*G*G*C*G*G*C*C*G*C*C*G | SEQ ID NO:32 |
| G*G*G-G-A-C-G-A-C-G-T-C-G-T-G-C*G*G*C*G*G*C*C*G*C*C*G | SEQ ID NO:33 |
| G*G*G*G*A*C*G*A*C*G*T*C*G*T*G*C*G*G*C*G*G*C*C*G*C*C*G | SEQ ID NO:34 |
| C*C-A*C-G*A*C-G*T*C-G*T*C-G-A-A-G*A*C-G*A*C-G*T*C-G*T-G*G | SEQ ID NO:35 |
| C*T-G*C*A*G-C*T-G-C*A*G-C*T-G-C*A*G-C*T-G*C*A*G | SEQ ID NO:36 |
| C*G*G-C*C-G*C*T-G*C*A-G-C*G-G*C*C-G*C*T-G*C*A*G | SEQ ID NO:37 |
| C*A*T*G*A*C*G*T*T*T*T*T*G*A*T*G*T*T | SEQ ID NO:38 |
| A*T*G*A*C*G*T*T*T*T*T*G*A*T*G*T*T | SEQ ID NO:39 |
| T*G*A*C*G*T*T*T*T*T*G*A*T*G*T*T | SEQ ID NO:40 |
| A*T*G*A*C*G*T*T*T*T*T*G*A*T*G*T*T*G*T | SEQ ID NO:41 |
| T*C*C*A*T*G*A*C-G-T*T*T*T*T*G*A*T*G*T*T | SEQ ID NO:42 |
| T*C*C*A*T*G*A-C-G-T*T*T*T*T*G*A*T*G*T*T | SEQ ID NO:43 |
| T*C*C*A*T*G*A*C*G*T*T*T*T*T*G*A*T-G-T*T | SEQ ID NO:44 |
| T*C*C*A*T*G*A*C-G-T*T*T*T*T*G*A*T-G*T*T | SEQ ID NO:45 |
| T*C*C*A*T*G*A-C-G-T*T*T*T*T*G*A*T-G*T*T | SEQ ID NO:46 |
| A*T*G*A*C-G*T*T*T*T*T*G*A*T*G*T*T*G*T | SEQ ID NO:47 |
| A*T*G*A*C*G*T*T*T*T*T*G*A*T-G*T*T*G*T | SEQ ID NO:48 |
| A*T*G*A*C-G*T*T*T*T*T*G*A*T-G*T*T*G*T | SEQ ID NO:49 |
| A*T*G*A-C-G-T*T*T*T*T*G*A-T-G-T*T*G*T | SEQ ID NO:50 |
| T*C*C*A*T*G*C*G*T*T*T*T*T*G*A*A*T*G*T*T | SEQ ID NO:51 |
| T*C*C*A*T*G*A*C*G*T*C*T*T*T*G*A*T*G*T*C | SEQ ID NO:52 |
| A-C-G-A-C-G-T-C-G-T-T-C-A-C-G-A-C-G-T-C-G-T-chol | SEQ ID NO:53 |
| A-C-G-A-C-G-T-C-G-T-G-G-C-C-A-C-G-A-C-G-T-C-G-T-D-D-D | SEQ ID NO:54 |
| A-C-G-A-C-G-T-C-G-T-D-D-D-D-A-C-G-A-C-G-T-C-G-T-D-D-D | SEQ ID NO:55 |
| D-D-D-A-C-G-A-C-G-T-C-G-T-D-D-D-D-A-C-G-A-C-G-T-C-G-T-D-D-D | SEQ ID NO:56 |
| D-D-D-A-C-G-A-C-G-T-C-G-T-D-D-D-D-A-C-G-A-C-G-T-C-G-T-chol | SEQ ID NO:57 |
| G*G*G-A-C-G-A-C-G-T-C-G-T-G*G*C*C-A-C-G-A-C-G-T-C-G-T-C*C*C | SEQ ID NO:58 |
| C*C*C-A-C-G-A-C-G-T-C-G-T-G*G*G | SEQ ID NO:59 |
| C*C*C*C-A-C-G-A-C-G-T-C-G-T-G*G*G*G | SEQ ID NO:60 |
| T*C*G*A*T*C*G*T*T*T*T-T-C-G*T*G*C*G*T*T*T*T*T | SEQ ID NO:61 |
| T*C*G*A*T*C*G*T*T*T-T-T-C-G-T*G*C*G*T*T*T*T*T | SEQ ID NO:62 |
| T*C*G*A*T*C*G*T*T-T-T-T-C-G-T-G*C*G*T*T*T*T*T | SEQ ID NO:63 |
| T*C*G*A*T*C*G-T-T-T-T-T-C*G*T*G*C*G*T*T*T*T*T | SEQ ID NO:64 |
| A*T-G*A*C-G*T*T*T*T*T-G*A*C-G*T*T | SEQ ID NO:65 |
| A*C-G*A*C-G*T*T*T*T*T-G*A*T-G*T*T | SEQ ID NO:66 |
| A*C-G*A*C-G*T*T*T*T*C-G*A*C-G*T*T | SEQ ID NO:67 |
| A*T-G*A*T-G*T*T*T*T*T-G*A*T-G*T*T | SEQ ID NO:68 |
| A*T-G*A*C-G*T*T*T*T*G-A*T*G-T*T | SEQ ID NO:69 |
| A*T-G*A*C-G*T*T*T*G*T-G*A*T-G*T*T | SEQ ID NO:70 |
| T*T-G*A*C-G*T*T*T*T*T-G*A*T-G*T*T | SEQ ID NO:71 |
| A*T-G*A*T-G*T*T*T*T*T-G*A*T-G*T*T | SEQ ID NO:72 |
| A*T-G*A*G-C*T*T*T*T*G-T*A*T-G*T*T | SEQ ID NO:73 |
| T*C*G*A*C*G*T*T*T*T*C*G*G*C*G*G*C*C*G*C*C*G | SEQ ID NO:74 |
| T*C*C*T*G*A*C*G*T*T*T*T*C*G*G*C*G*G*C*C*G*C*C*G | SEQ ID NO:75 |
| T*C*C*T*G*A*C*G*T*T*C*G*G*C*G*G*C*C*G*C*C*G | SEQ ID NO:76 |
| T*C*C*A*T*G*A*C*G*T*T*C*G*G*C*G*C*G*C*G*C*C*C | SEQ ID NO:77 |
| T*C*C*T*G*A*C*G*T*T*C*G*G*C*G*C*G*C*G*C*C | SEQ ID NO:78 |
| T*C*G*A*C*G*T*T*T-T-C-G-G-C*G*C*G*C*G*C*C*G | SEQ ID NO:79 |
| T*C*G*A*C*G*T*T*T-T-C-G-G-C*G*G*C*C*G*C*C*G | SEQ ID NO:80 |
| T*C*G*A*C*G*T*C*G-A-C-G-T-T-A-G-G-G-T-T-A*G*G*G | SEQ ID NO:81 |
| A*C*G*A*C*G*T*C*G-T-T-A-G-G-G-T-T-A*G*G*G | SEQ ID NO:82 |
| G*T*C-G*G*C-G*T*T-G*A*C | SEQ ID NO:83 |
| A-C-G-A-C-G-T-C-G-T-C-G-D-D-D-D-C-G-G-C-C-G-C-C-G | SEQ ID NO:84 |
| A-C-G-A-C-G-T-C-G-T-C-G-D-D-D-D*C*G*G*C*C*G*C*C*G | SEQ ID NO:85 |
| T-C-G-T-C-G-A*C*G*A*C*G*T*C*G*T*C*G | SEQ ID NO:86 |
| T-C-G-T-C-G-A-C-G-A-C-G-T-C-G-T-C-G-D-D-D-D | SEQ ID NO:87 |
| A-C-G-A-C-G-T-C-G-T-T*T*T*T-A-C-G-A-C-G-T-C-G-T-teg | SEQ ID NO:88 |
| A*C*G*A*C*G*T*C*G*T*D*D*D*D*A*C*G*A*C*G*T*C*G*T*D*D*D | SEQ ID NO:89 |
| D*D*D*A*C*G*A*C*G*T*C*G*T*D*D*D*D*A*C*G*A*C*G*T*C*G*T*D*D*D | SEQ ID NO:90 |
| A-C-G-A-C-G-T-C-G-T-T*T*T*T-A-C-G-A-C-G-T-C-G-T-D-D-D | SEQ ID NO:91 |
| A-C-G-A-C-G-T-C-G-T-T*T*T*T-A-C-G-A-C-G-T-C-G-T-T*T*T | SEQ ID NO:92 |
| A*C-G-A-C-G-T-C-G-T-T*T*T*T-A-C-G-A-C-G-T-C-G-T-T*T*T | SEQ ID NO:93 |
| A*C-G-A-C-G-T-C-G-T-T*T*T*T-A-C-G-A-C-G-T-C-G*T | SEQ ID NO:94 |
| A-C-G-A-C-G-T-C-G-T-D-D-D-D-A-C-G-A-C-G-T-C-G-T-L | SEQ ID NO:95 |
| A-C-G-A-C-G-T-C-G-T-L-A-C-G-A-C-G-T-C-G-T-L | SEQ ID NO:96 |
| A-C-G-A-C-G-T-C-G-T-teg-teg-A-C-G-A-C-G-T-C-G-T-teg | SEQ ID NO:97 |
| C-G-A-C-G-T-C-G-T-D-D-D-D-A-C-G-A-C-G-T-C-G-D-D-D | SEQ ID NO:98 |
| A-C-G-A-C-G-T-C-G-D-D-D-D-C-G-A-C-G-T-C-G-T-D-D-D | SEQ ID NO:99 |
| C-G-A-C-G-T-C-G-D-D-D-D-C-G-A-C-G-T-C-G-D-D-D | SEQ ID NO:100 |
| T-C-G-A-C-G-T-C-G-T-D-D-D-D-A-C-G-A-C-G-T-C-G-A-D-D-D | SEQ ID NO:101 |
| A-C-G-T-C-G-T-C-G-T-D-D-D-D-A-C-G-A-C-G-A-C-G-T-D-D-D | SEQ ID NO:102 |
| T-C-G-T-C-G-A-C-G-T-D-D-D-D-A-C-G-T-C-G-A-C-G-A-D-D-D | SEQ ID NO:103 |
| T-C-G-A-C-G-T-C-G-T-D-D-D-D-A-C-G-A-C-G-T-C-G-T-D-D-D | SEQ ID NO:104 |
| A-C-G-A-C-G-T-C-G-T-D-D-D-D-A-C-G-T-C-G-T-C-G-T-D-D-D | SEQ ID NO:105 |
| A-C-G-A-C-G-T-T-D-D-D-D-A-A-C-G-T-C-G-T-D-D-D | SEQ ID NO:106 |
| A-C-G-T-C-G-T-D-D-D-D-A-C-G-A-C-G-T-D-D-D | SEQ ID NO:107 |
| G-G-C-G-G-C-C-G-D-D-D-D-C-G-G-C-C-G-C-C-D-D-D | SEQ ID NO:108 |
| G-C-G-G-C-C-G-G-D-D-D-D-C-C-G-G-C-C-G-C-D-D-D | SEQ ID NO:109 |
| A-C-G-T-C-G-T-D-D-D-D-A-C-G-A-C-G-T-C-G-T-D-D-D | SEQ ID NO:110 |
| D-A-C-G-A-C-G-T-C-G-T-D-D-D-D-A-C-G-A-C-G-T-C-G-T-D | SEQ ID NO:111 |
| A*C-G-A-C-G-T-C-G-T-C-G-A-A-G-A-C-G-A-C-G-T-C-G-T-D-D-T | SEQ ID NO:112 |
| T*C-G-A-C-G-T-C-G-T-C-G-A-A-G-A-C-G-T-C-G-T-C-G-T-D-D-T | SEQ ID NO:113 |
| C*C*A-C-G-A-C-G-T-C-G-T-C-G-A-A-G-A-C-G-A-C-G-T-C-G-T*G*G | SEQ ID NO:114 |
| T*C*C*A*D*G*A*C*G*T*T*T*T*T*G*A*T*G*T*T | SEQ ID NO:115 |
| T*C*C*A*T*G*A*C*G*T*T*D*T*T*G*A*T*G*T*T | SEQ ID NO:116 |
| T*C*C*A*J*G*A*C*G*T*T*T*T*T*G*A*T*G*T*T | SEQ ID NO:117 |
| T*C*C*A*T*G*A*C*G*T*T*J*T*T*G*A*T*G*T*T | SEQ ID NO:118 |
| T*C*C*A*T*G*A*C*G*T*T*T*T*T*G*A*T*G*T*T*cy3 | SEQ ID NO:119 |
| J*J*J*J*J*G*A*C*G*T*T*T*T*T*G*A*T*G*T*T | SEQ ID NO:120 |
| T*C*C*A*J*G*A*C*G*T*T*J*T*T*G*A*T*G*T*T | SEQ ID NO:121 |
| T*C*C*A*D*G*A*C*G*T*T*D*T*T*G*A*T*G*T*T | SEQ ID NO:122 |
| A-C-G-A-C-G-T-C-G-T-D-D-D-D-A-C-G-A-C-G-T-C-G-T-D-D-D-rU | SEQ ID NO:123 |
| A-C-G-A-C-G-T-C-G-T-D-D-D-D-A-C-G-A-C-G-T-C-G-T-D-D-D-rG | SEQ ID NO:124 |
| A-C-G-A-C-G-T-C-G-T-D-D-D-D-A-C-G-A-C-G-T-C-G-T-D-D-D-rA | SEQ ID NO:125 |
| D-D-D-A-C-G-A-C-G-T-C-G-T-D-D-D-D-A-C-G-A-C-G-T-C-G-T-D-D-D-rU | SEQ ID NO:126 |
| A-C-G-A-C-G-T-C-G-T-D-D-D-D-A-C-G-A-C-G-T-C-G-T-D-D-D-rA2-rA2-rA2-rA | SEQ ID NO:127 |
| T*C*G*A*T*G*A*C*G*T*T*C*C*T*G*A*C*G*T*T | SEQ ID NO:128 |
| T-T-T-A-C-G-A-C-G-T-C-G-T-D-D-D-D-A-C-G-A-C-G-T-C-G-T-D-D-D-rU | SEQ ID NO:129 |
| (T*C-G-A-C-G-T-C-G-T-)(vitE-)doub-teg | SEQ ID NO:130 |
| T*C*G*A*C-G*T*T*T*T*C-G*G*C*G*G*C*C-G*C*C*G | SEQ ID NO:131 |
| T*C*G*A*C-G*T*T*T*T*C-G*G*C*G*C*G*C-G*C*C*G | SEQ ID NO:132 |
| T*C-G*C-G*A*C-G*T*T*C-G*G*C*G*C-G*C*G*C*C*G | SEQ ID NO:133 |
| T*C*G*C-G*A*C*G*T*T*C-G*G*C*G*C*G*C*G*C*C*G | SEQ ID NO:134 |
| T*C*G*C-G*A*C*G*T*T*C*G*G*C-G*C*G*C*G*C*C*G | SEQ ID NO:135 |
| T*C*G*C-G*A*C*G*T*T*C*G*G*C*G*C-G*C*G*C*C*G | SEQ ID NO:136 |
| T*C*G*C*G*A*C-G*T*T*C*G*G*C*G*C-G*C*G*C*C*G | SEQ ID NO:137 |
| T*C*G*C*G*A*C-G*T*T*C*G*G*C-G*C*G*C*G*C*C*G | SEQ ID NO:138 |
| T*C*G*C-G*A*C*G*T*T*C-G*G*C*G*C-G*C*G*C*C*G | SEQ ID NO:139 |
| T*C*G*C*G*A*C-G*T*T*C-G*G*C*G*C-G*C*G*C*C*G | SEQ ID NO:140 |
| T*C*G*C*G*A*C-G*T*T*C*G*C*G*C-G*C*G*C*G | SEQ ID NO:141 |
| D*C*C*A*T*G*A*C*G*T*T*T*T*T*G*A*T*G*T*T | SEQ ID NO:142 |
| T*D*C*A*T*G*A*C*G*T*T*T*T*T*G*A*T*G*T*T | SEQ ID NO:143 |
| T*C*D*A*T*G*A*C*G*T*T*T*T*T*G*A*T*G*T*T | SEQ ID NO:144 |
| T*C*C*D*T*G*A*C*G*T*T*T*T*T*G*A*T*G*T*T | SEQ ID NO:145 |
| T*C*C*A*T*G*A*C*G*T*T*T*T*D*G*A*T*G*T*T | SEQ ID NO:146 |
| T*C*C*A*T*G*A*C*G*T*T*T*D*T*G*A*T*G*T*T | SEQ ID NO:147 |
| T*C*G*A*A*C-G*T*T*C*G*G*C*G*C*G*C*G*C*C*G | SEQ ID NO:148 |
| T*C*G*T*C*G*A*A*C-G*T*T*C*G*G*C*G*C*G*C*G*C*C*G | SEQ ID NO:149 |
| T*C*G*T*C*G*A*A*C-G*T*T*C*G*G*C*G*C*T*G*C*G*C*C*G | SEQ ID NO:150 |
| T*C*G*C*G*A*C-G*T*T*C*G*T*T*G*C*G*C*G*C*G*C*C*G | SEQ ID NO:151 |
| T*A*C*G*T*C-G*T*T*C*G*G*C*G*C*G*C*G*C*C*G | SEQ ID NO:152 |
| T*T*C*G*C*G*A*C-G*T*T*C*G*G*C*G*C*G*C*G*C*C*G | SEQ ID NO:153 |
| T*C*G*G*C*G*C*G*C*G*C*C-G*T*C*G*C*G*A*C*G*T | SEQ ID NO:154 |
| T*A*G*C-G*T*G*C-G*T*T*T*T*G*A*C-G*T*T*T*T*T*T*T | SEQ ID NO:155 |
| T*A*G*C-G*A*G*C-G*T*T*T*T*G*A*C-G*T*T*T*T*T*T*T | SEQ ID NO:156 |
| T*T*G*C-G*A*G*C-G*T*T*T*T*G*A*C-G*T*T*T*T*T*T*T | SEQ ID NO:157 |
| A*T*G*C-G*T*G*C-G*T*T*T*T*G*A*C-G*T*T*T*T*T*T*T | SEQ ID NO:158 |
| T*T*A*C-G*T*G*C-G*T*T*T*T*G*A*C-G*T*T*T*T*T*T*T | SEQ ID NO:159 |
| T*T*G*C-A*T*G*C-G*T*T*T*T*G*A*C-G*T*T*T*T*T*T*T | SEQ ID NO:160 |
| T*T*G*C-G*T*A*C-G*T*T*T*T*G*A*C-G*T*T*T*T*T*T*T | SEQ ID NO:161 |
| T*T*G*C-G*T*G*C-A*T*T*T*T*G*A*C-G*T*T*T*T*T*T*T | SEQ ID NO:162 |
| T*T*G*C-G*T*G*C-G*A*T*T*T*G*A*C-G*T*T*T*T*T*T*T | SEQ ID NO:163 |
| T*T*G*C-G*C*G*C-G*T*T*T*T*G*A*C-G*T*T*T*T*T*T*T | SEQ ID NO:164 |
| T*T*G*C-G*T*G*C-G*C*T*T*T*G*A*C-G*T*T*T*T*T*T*T | SEQ ID NO:165 |
| T*T*G*C-G*T*G*C-G*T*T*T*C*G*A*C-G*T*T*T*T*T*T*T | SEQ ID NO:166 |
| T*C*G*T*C-G*A*A*C*G*T*T*C-G*G*C*G*C*T*G*C*G*C*C*G | SEQ ID NO:167 |
| T*C*G*T*C-G*A*A*C*G*T*T*C-G*G*C-G*C*T*G*C*G*C*C*G | SEQ ID NO:168 |
| T*C*G*T*C-G*A*A*C*G*T*T*C-G*G*C*G*C*T*G*C-G*C*C*G | SEQ ID NO:169 |
| T*C*G*T*C*G*A*A*C-G*T*T*C*G*G*C-G*C*T*G*C*G*C*C*G | SEQ ID NO:170 |
| T*C*G*T*C-G*G*A*C*G*T*T*C-G*G*C*G*C*T*G*C*G*C*C*G | SEQ ID NO:171 |
| T*C*G*C*G*A*C-G*T*T*C*G*T*T*G*C-G*C*G*C*G*C*C*G | SEQ ID NO:172 |
| T*C*G*C-G*A*C*G*T*T*C-G*T*T*G*C-G*C*G*C*G*C*C*G | SEQ ID NO:173 |
| T*C-G*C*G*A*C*G*T*T*C-G*T*T*G*C*G*C-G*C*G*C*C*G | SEQ ID NO:174 |
| T*C*G*C*G*A*C-G*T*T*T*T*G*C*G*C-G*C*G*C | SEQ ID NO:175 |
| T*C*G*C*G*A*C-G*T*C*G*T*T*G*C-G*C*G*C*G*C*C*G | SEQ ID NO:176 |
| T*C*G*C*G*A*C-G*T*T*C*G*A*A*G*C-G*C*G*C*G*C*C*G | SEQ ID NO:177 |
| T*C*G*C*G*A*C-G*A*A*C*G*T*T*G*C-G*C*G*C*G*C*C*G | SEQ ID NO:178 |
| T-C-G-A-C-G-T-C-G-T-D-D-D-D-T-C-G-A-C-G-T-C-G-T-D-D-D | SEQ ID NO:179 |
| T*C*G*T*C*G*T*T*A*G*C*T*C*G*T*T*A*G*C*T*C*G*T*T | SEQ ID NO:180 |
| T*C*G*T*C*G*T*T*A*C*G*T*A*A*T*T*A*C*G*T*C*G*T*T | SEQ ID NO:181 |
| T*C*G*T*C*G*T*T*A*C*G*T*C*G*T*T*A*C*G*T*A*A*T*T | SEQ ID NO:182 |
| T*C*G*T*C*G*T*T*A*C*G*T*A*A*T*T*A*C*G*T*A*A*T*T | SEQ ID NO:183 |
| T*C*G*A*C*G*T*C*G-A-C*G*T*G*A*C*G*G*G | SEQ ID NO:184 |
| (T-C-G-A-C-G-T-C-G-T-T-)2doub-but | SEQ ID NO:185 |
| (T-C-G-A-C-G-T-C-G-T-T-)2doub-chol | SEQ ID NO:186 |
| (T-C-G-A-C-G-T-C-G-T-T-T-)2doub-chol | SEQ ID NO:187 |
| T-C-G-A-C-G-T-C-G-T-T-T-chol-T-T-C-G-A-C-G-T-C-G-T-T-but | SEQ ID NO:188 |
| T*C*G*C-G*A*C*G*T*T*C-G*G*C*G*C-G*C*T*G*C*C*G | SEQ ID NO:189 |
| T*C*G*C-G*A*C*G*T*T*C-G*G*C*G*C-G*T*C*G*C*C*G | SEQ ID NO:190 |
| T*C*G*C-G*A*C*G*T*T*C-G*G*C*G*G*C-T*C*G*C*C*G | SEQ ID NO:191 |
| T*C*G*C*G-A*C*G*T*T*C-G*G*C*G*C-G*T*C*G*C*C*G | SEQ ID NO:192 |
| T*C*G*C*G-A*C*G*T*T*C-G*G*C*G*G*C-T*C*G*C*C*G | SEQ ID NO:193 |
| T*C*G-C*G*A*C*G*T*T*C-G*G*C*G*C-G*T*C*G*C*C*G | SEQ ID NO:194 |
| T*C*G-C*G*A*C*G*T*T*C-G*G*C*G*G*C-T*C*G*C*C*G | SEQ ID NO:195 |
| (T-C-G-A-C-G-T-C-G-T-)(vitE-) | SEQ ID NO:196 |
| T*C-G*A*C-G*T*C-G*A*C*G*T*G*A*C*G*G*G | SEQ ID NO:197 |
| T*C*G*A*C*G*T*C*G*A*C*G*T*G*A*C*G*G*G | SEQ ID NO:198 |
| T*C*G*A*C*G*T*C*G*A*C*G*T*G*A*C*G*T*C | SEQ ID NO:199 |
| T*C*G*A*C*G*T*C*G*A*C*G*T*G*A*C*G | SEQ ID NO:200 |
| (T-C-G-A-C-G-T-C-G-A-)(vitE-) | SEQ ID NO:201 |
| T*C*G*T*C*G*T*T*A*C*G*T*A*A*C*T*A*C*G*T*C*G*T*T | SEQ ID NO:202 |
| T*C*G*T*C*G*T*T*A*C*G*T*A*A*C*G*A*C*G*T*C*G*T*T | SEQ ID NO:203 |
| T*C*G*T*C*G*T*T*A*C*G*T*A*A*C*G*A*C*G*A*C*G*T*T | SEQ ID NO:204 |
| T*C*G*T*C*G*T*T*A*G*C*T*A*A*T*T*A*G*C*T*C*G*T*T | SEQ ID NO:205 |
| T*C*G*T*C*G*T*T*A*C*G*T*A*A*T*T*A*G*C*T*C*G*T*T | SEQ ID NO:206 |
| C*C*C*A*T*G*A*C*G*T*T*C*C*T*G*A*C*G*T*T | SEQ ID NO:207 |
| G*C*C*A*T*G*A*C*G*T*T*C*C*T*G*A*C*G*T*T | SEQ ID NO:208 |
| A*C*C*A*T*G*A*C*G*T*T*C*C*T*G*A*C*G*T*T | SEQ ID NO:209 |
| T*G*G*A*T*G*A*C*G*T*T*C*C*T*G*A*C*G*T*T | SEQ ID NO:210 |
| T*T*T*A*T*G*A*C*G*T*T*C*C*T*G*A*C*G*T*T | SEQ ID NO:211 |
| T*A*A*A*T*G*A*C*G*T*T*C*C*T*G*A*C*G*T*T | SEQ ID NO:212 |
| C*C*A*T*G*A*C*G*T*T*C*C*T*G*A*C*G*T*T | SEQ ID NO:213 |
| C*A*T*G*A*C*G*T*T*C*C*T*G*A*C*G*T*T | SEQ ID NO:214 |
| A*T*G*A*C*G*T*T*C*C*T*G*A*C*G*T*T | SEQ ID NO:215 |
| T*G*A*C*G*T*T*C*C*T*G*A*C*G*T*T | SEQ ID NO:216 |
| T-C-G-A-C-G-T-C-G-A-D-D-D-D-T-C-G-A-C-G-T-C-G-A-chol | SEQ ID NO:217 |
| teg-iA-iG-iC-iT-iG-iC-iA-iG-iC-iT-D-D-D-D-T-C-G-A-C-G-T-C-G-A-chol | SEQ ID NO:218 |
| T*C-G*C-G*A*C-G*T*T*C-G*G*C-G*C-G*C-G*C*C-G | SEQ ID NO:219 |
| T*C-G*T*C-G*A*C-G*T*T*C-G*G*C*G*C-G*C*G*C*C*G | SEQ ID NO:220 |
| T*C-G*G*A*C-G*T*T*C-G*G*C*G*C-G*C*G*C*C*G | SEQ ID NO:221 |
| T*C-G*G*A*C-G*T*T*C-G*G*C*G*C*G*C*C*G | SEQ ID NO:222 |
| T*C-G*C-G*A*C-G*T*T*C-G*G*C*G*C*G*C*C*G | SEQ ID NO:223 |
| T*C-G*C-G*A*C-G*T*T*C-G*C-G*C-G*C-G*C-G | SEQ ID NO:224 |
| T*C-G*A*C-G*T*T*C-G*G*C*G*C-G*C*G*C*C*G | SEQ ID NO:225 |
| T*C-G*A*C-G*T*T*C-G*G*C*G*C*G*C*C*G | SEQ ID NO:226 |
| T*C-G*C-G*A*C-G*T*T*C-G*G*C*G*C*C*G | SEQ ID NO:227 |
| T*C-G*C-G*A*C-G*T*T*C-G*G*C*C*G | SEQ ID NO:228 |
| T*C-G*A*C-G*T*T*C-G*G*C*G*C*C*G | SEQ ID NO:229 |
| T*C-G*T*C-G*A*C-G*T*T*C-G*G*C*G-C*G*C*C*G | SEQ ID NO:230 |
| T*C-G*T*C-G*A*C-G*T*T*C-G*G*C-G*C*C*G | SEQ ID NO:231 |
| T*C-G*A*C-G*A*C-G*T*T*C-G*G*C*G*C-G*C*G*C*C*G | SEQ ID NO:232 |
| T*C-G*A*C-G*T*C-G*T*T*C-G*G*C*G*C-G*C*G*C*C*G | SEQ ID NO:233 |
| T*C-G*T*C-G*A*C-G*A*T*C-G*G*C*G*C-G*C*G*C*C*G | SEQ ID NO:234 |
| T*C-G*T*C-G*A*C-G*A*T*C-G*G*C*G-C*G*C*C*G | SEQ ID NO:235 |
| T*C-G*T*C-G*A*C-G*T*T*C-G*C*C*G*C-G*C*G*G*C*G | SEQ ID NO:236 |
| T*C-G*T*C-G*A*C-G*T*T*C-G*G*C*G*C*C-G*T*G*C*C*G | SEQ ID NO:237 |
| T*C-G*T*C-G*A*C-G*T*T*C-G*A*C*T*C-G*A*G*T*C*G | SEQ ID NO:238 |
| T*C-G*T*C-G*T*T*A*C-G*T*A*A*C-G*A*C*G*A*C-G*T*T | SEQ ID NO:239 |
| T*C*G*T*C-G*T*T*A*C-G*T*A*A*C-G*A*C*G*A*C*G*T*T | SEQ ID NO:240 |
| T*C*G*A*C*G*T*C*G*A*C*G*T*G*A*C*G*T*T | SEQ ID NO:241 |
| T*C*G*T*C*G*A*C*G*T*T*C*G*G*C*G*C*G*C*C*G | SEQ ID NO:242 |
| T*C*G*T*C*G*A*C*G*A*T*C*G*G*C*G*C*G*C*G*C*C*G | SEQ ID NO:243 |
| A-C-G-A-C-G-T-C-G-T-D-D-D-D-A-C-G-A-C-G-T-C-G-T-D-D-D-irU | SEQ ID NO:244 |
| T*C-G*T*C-G*A*C-G*A*T*C-G*G*C*G*C*C-G*T*G*C*C*G | SEQ ID NO:245 |
| T*C-G*T*C-G*A*C-G*A*C-G*G*C*G*C*C-G*T*G*C*C*G | SEQ ID NO:246 |
| T*C-G*T*C-G*A*C-G*A*C-G*C*G*C*C-G*T*G*C*G | SEQ ID NO:247 |
| T*C*G*T*C*G*A*C*G*A*T*C*G*G*C*G*C*C*G*T*G*C*C*G | SEQ ID NO:248 |
| T*C*G*T*C-G*A*C-G*A*T*C-G*G*C*G*C*C-G*T*G*C*C*G | SEQ ID NO:249 |
| T*C*G*T*C-G*A*C*G*A*T*C-G*G*C*G*C*C-G*T*G*C*C*G | SEQ ID NO:250 |
| T*C*G*T*C*G*A*C*G*A-T-C*G*G*C*G*C*C*G*T*G*C*C*G | SEQ ID NO:251 |
| T*C*G*T*C-G*A*C-G*A*T*C-G*G*C*G*C-G*C*G*C*C*G | SEQ ID NO:252 |
| T*C*G*T*C-G*A*C*G*A*T*C-G*G*C*G*C-G*C*G*C*C*G | SEQ ID NO:253 |
| T*C*G*T*C*G*A*C*G*A-T-C*G*G*C*G*C*G*C*G*C*C*G | SEQ ID NO:254 |
| T*C*G*A*C*G*T*C*G-A-C*G*T*G*A*C*G*T*T | SEQ ID NO:255 |
| T*C*G*A*C-G*T*C*G*A*C-G*T*G*A*C*G*T*T | SEQ ID NO:256 |
| T*C*G*A*C-G*T*C*G*A*C*G*T-G*A*C*G*T*T | SEQ ID NO:257 |
| T*C*G*T*C-G*A*C*G*A*C-G*T*G*T*C*G*A*T | SEQ ID NO:258 |
| T*C*G*A*C*G-T*C*G*A*C*G-T*G*A*C*G*T*T | SEQ ID NO:259 |
| T*C*G*A-C*G*T*C*G-A*C*G*T*G-A*C*G*T*T | SEQ ID NO:260 |
| T*C*G*T*C*G*A-C*G*A*T*C*G*G*C*G-C*C*G*T*G*C*C*G | SEQ ID NO:261 |
| T*C*G*T*C*G*A-C*G*A*C*G*G*C*G*C-C*G*T*G*C*C*G*T | SEQ ID NO:262 |
| T*C*G*T*C*G*A*C-G*A*C*G*G*C*G*C*C-G*T*G*C*C*G*T | SEQ ID NO:263 |
| T*C*G*T*C-G*A*C-G*A*T*C-G*G*C*G*C*C-G*T*G*C*C*G*T | SEQ ID NO:264 |
| T*C-G*T*C-G*A*C-G*T*T*C-G*G*C*G*C*C-G*T*G*C*C*G*T | SEQ ID NO:265 |
| T*C-G*T*C-G*A*C-G*T*C-G*G*C*G*C*C-G*T*G*C*C*G*T | SEQ ID NO:266 |
| T*C-G*T*C-G*A*C-G*C-G*G*C*G*C*C-G*T*G*C*C*G*T | SEQ ID NO:267 |
| T*C*G*T*C*G*A-C*G*C*G*G*C*G-C*C*G*T*G*C*C*G*T | SEQ ID NO:268 |
| T*C*G*T*C-G*A*C*G*A-A*G*T*C-G*A*C*G*A*T | SEQ ID NO:269 |
| T*C*G*T*C-G*A*C*G*A*G*A-A*T*C*G*T*C-G*A*C*G*A*T | SEQ ID NO:270 |
| T*C*G*T*C-G*T*A*C-G*G*C*G*C*C-G*T*G*C*C*G*T | SEQ ID NO:271 |
| T*C*G*T*C*G*A*C-G*A*T*C*G*G*C-G*C*C*G*T*G*C*C*G | SEQ ID NO:272 |
| T*C*G*T*C*G*A-C*G*A*T*C*G*G*C*G-C*C*G*T*G*C*C*G | SEQ ID NO:273 |
| T*C*G*T*C*G*A-C*G*A*T*C*G-G*C*G*C-C*G*T*G*C*C*G | SEQ ID NO:274 |
| T*C*G*T*C*G*A-C*G*A*C*G*G*C*G*C-C*G*T*G*C*C*G*T | SEQ ID NO:275 |
| T*C*G*T*C-G*A*C-G*A*T*C-G*G*C*G*C*C-G*T*G*C*C*G*T | SEQ ID NO:276 |
| T*C*G*T*C*G*A-C*G*A*T*C*G*G*C*G-C*C*G*T*G*C*C*G*T | SEQ ID NO:277 |
| T*C*G*T*C*G*A*C-G*A*C*G*G*C*G*C-C*G*T*G*C*C*G*T | SEQ ID NO:278 |
| T*C-G*T*C-G*A*C-G*T*T*C-G*G*C*G*C*C-G*T*G*C*C*G*T | SEQ ID NO:279 |
| T*C-G*T*C-G*A*C-G*T*C-G*G*C*G*C*C-G*T*G*C*C*G*T | SEQ ID NO:280 |
| T*C*G*T*C-G*A*C*G*A-A*G*T*C-G*A*C*G*A*T | SEQ ID NO:281 |
| T*C*G*T*C-G*A*C*G*A*G*A-A*T*C*G*T*C-G*A*C*G*A*T | SEQ ID NO:282 |
| T*C*G*T*C-G*A*C*G*A*C-G*T*G*T*C*G*A*T | SEQ ID NO:283 |
| T*C*G*A*C-G*T*C*G*A-A*G*A*C-G*T*C*G*A*T | SEQ ID NO:284 |
| T*C*G*A*C-G*T*C*G*A*G*A-A*T*C*G*A*C-G*T*C*G*A*T | SEQ ID NO:285 |
| T*C*G*T*C-G*A*C-G*A*C*G*G*C*G-A*A*G*C*C*G | SEQ ID NO:286 |
| T*C*G*T*C-G*A*C-G*A*C*G*G*C*G-A*A*G*C*C*G*T | SEQ ID NO:287 |
| T*C*G*T*C*G-A*C*G*A*C*G-T*G*T*C*G*A*T | SEQ ID NO:288 |
| T*C*G*T*C*G*A*C*G*A*C*G*T*G*T*C*G*A*T | SEQ ID NO:289 |
| T*C*G*A*C-G*T*C*G*A*C-G*T*G*A*C*G-T*T*G*T | SEQ ID NO:290 |
| T*C<G*T*C-G*A*C-G*A*T*C-G*G*C*G*C-G*C*G*C*C*G-but | SEQ ID NO:291 |
| T*C-G*T*C<G*A*C-G*A*T*C-G*G*C*G*C-G*C*G*C*C*G-but | SEQ ID NO:292 |
| T*C-G*T*C-G*A*C*G*A*T*C-G*G*C*G*C-G*C*G*C*C*G-iT | SEQ ID NO:293 |
| iT-T*C-G*T*C-G*A*C*G*A*T*C-G*G*C*G*C-G*C*G*C*C*G-iT | SEQ ID NO:294 |
| T*C-G*T*C-G*A*C-G*A*T*C-G*A*C*G*C-G*C*G*T*C*G | SEQ ID NO:295 |
| T*C-G*T*C-G*A*C-G*A*T*C-A*A*C*G*C-G*C*G*T*T*G | SEQ ID NO:296 |
| T*C-G*T*C-G*A*C-G*A*T*C-G*G*C*A*C-G*T*G*C*C*G | SEQ ID NO:297 |
| T*C-G*T*C-G*A*C-G*A*T*C-G*G*C*A*T-A*T*G*C*C*G | SEQ ID NO:298 |
| T*C-G*T*C-G*A*C-G*A*T*G-C*C*G*C*G-C*G*C*G*G*C | SEQ ID NO:299 |
| T*C*G*T*C*G*A*C*G*A*T*G*C*C*G*C*G*C*G*C*G*G*C | SEQ ID NO:300 |
| T*C-G*T*C*G*A*C*G*A*T*G*C*C*G*C*G*C*G*C*G*G*C | SEQ ID NO:301 |
| T*C*G*T*C-G*A*C*G*A*T*G*C*C*G*C*G*C*G*C*G*G*C | SEQ ID NO:302 |
| T*C-G*T*C*G*A*C*G*A*T*G*C*C*G*C*G*C*T*G*C*G*G*C | SEQ ID NO:303 |
| T*C-G*T*C*G*T*A*C*G*A*T*G*C*C*G*C*G*C*G*C*G*G*C | SEQ ID NO:304 |
| T*C-G*T*C*G*T*A*C*G*A*T*G*C*C*G*C*G*C*T*G*C*G*G*C | SEQ ID NO:305 |
| T*C*G*T*C*G*A*C*G*A*T-G*C*C*G*C*G*C*G*C*G*G*C | SEQ ID NO:306 |
| T*C*G*T*C*G*A*C*G*A*T-G-C*C*G*C*G*C*G*C*G*G*C | SEQ ID NO:307 |
| T*C*G*T*C-G*A*C*G*A*T*C*G*G*C*G*C*G*C*G*C*C*G-iT | SEQ ID NO:308 |
| T*C-G*T*C*G*A*C*G*A*T*C*G*G*C*G*C*G*C*G*C*C*G-iT | SEQ ID NO:309 |
| T*C*G*T*C*G*A*C*G*A*T*C-G*G*C*G*C*G*C*G*C*C*G-iT | SEQ ID NO:310 |
| T*C-G*T*G-C*A*C-G*A*T*C-G*G*C*G*C-G*C*G*C*C*G | SEQ ID NO:311 |
| T*Z-G*T*C-G*A*C-G*A*T*C-G*G*C*G*C-G*C*G*C*C*G | SEQ ID NO:312 |
| T*C-G*T*Z-G*A*C-G*A*T*C-G*G*C*G*C-G*C*G*C*C*G | SEQ ID NO:313 |
| T*C-G*T*C-G*A*Z-G*A*T*C-G*G*C*G*C-G*C*G*C*C*G | SEQ ID NO:314 |
| T*C-G*T*C-G*A*C-G*A*T*Z-G*G*C*G*C-G*C*G*C*C*G | SEQ ID NO:315 |
| T*C-G*A*C*G*T*C-G*A*C*G*T*C-G*A*C*G | SEQ ID NO:316 |
| T-C-G-A-C-G-T-C-G-A-C-G-T-C-G-A-C-G | SEQ ID NO:317 |
| T*C*G*A*C*G*T*C*G*A*C*G*T*C*G*A*C*G | SEQ ID NO:318 |
| T*C-G*T*C*G*A*C*G*T*T*C*G*G*C*G*C*C*G*T*G*C*C*G-iT | SEQ ID NO:319 |
| T*C*G*T*C-G*A*C*G*T*T*C*G*G*C*G*C*C*G*T*G*C*C*G-iT | SEQ ID NO:320 |
| T*C*G*T*C*G*A*C*G*T*T-C-G*G*C*G*C*C*G*T*G*C*C*G-iT | SEQ ID NO:321 |
| G*C*C*G*C*G-C*G*C*G*G-C*iT*iA*iG-iC*iA*iG-iC*iT*iG-iC*iT | SEQ ID NO:322 |
| C*G*G*C*G*C-G*C*G*C*C-G*iT*iA*iG-iC*iA*iG-iC*iT*iG-iC*iT | SEQ ID NO:323 |
| G*C*C*G*C*G*C*G*C*G*G*C*iT*iA*iG*iC*iA*iG-iC*iT*iG*iC*iT | SEQ ID NO:324 |
| C*G*G*C*G*C*G*C*G*C*C*G*iT*iA*iG*iC*iA*iG-iC*iT*iG*iC*iT | SEQ ID NO:325 |
| C*G*G*C*G*C*C-G*T*G*C*C*G*iT*iT*iG*iC*iA*iG-iC*iT*iG*iC*iT | SEQ ID NO:326 |
| G*C*C*G*T*G-C*C*G*C*G*G-C*iT*iT*iG*iC*iA*iG-iC*iT*iG*iC*iT | SEQ ID NO:327 |
| C*G*G*C*G*C*C*G*T*G*C*C*G*iT*iT*iG*iC*iA*iG-iC*iT*iG*iC*iT | SEQ ID NO:328 |
| G*C*C*G*T*G*C*C*G*C*G*G*C*iT*iT*iG*iC*iA*iG-iC*iT*iG*iC*iT | SEQ ID NO:329 |
| T*C*G*G*C*G*C-G*C*G*C*C-G*A*iT*iA*iG-iC*iA*iG-iC*iT*iG-iC*iT | SEQ ID NO:330 |
| T*C*G*G*C*G*C*G*C*G*C*C*G*A*iT*iA*iG*iC*iA*iG-iC*iT*iG*iC*iT | SEQ ID NO:331 |
| T*C*G*G*C*G*C*C-G*T*G*C*C*G*iT*iT*iG*iC*iA*iG-iC*iT*iG*iC*iT | SEQ ID NO:332 |
| T*C*G*G*C*G*C*C*G*T*G*C*C*G*iT*iT*iG*iC*iA*iG-iC*iT*iG*iC*iT | SEQ ID NO:333 |
| CGGCGCX₁GCGCCG | SEQ ID NO:334 |
| T*C_G*T*C_G*A*C_G*T*T*C_G*G*C*G*C_G*C*G*C*C*G | SEQ ID NO:335 |
| T*C*G*T*C*G*A*C*G*A*C*G*G*C*G*C*G*C*G*C*C*G | SEQ ID NO:336 |
| T*C*G*T*C*G*A*C*G*A*J*C*G*G*C*G*C*G*C*G*C*C*G | SEQ ID NO:337 |
| T*C*G*T*C*G*A*C*G*A*L*C*G*G*C*G*C*G*C*G*C*C*G | SEQ ID NO:338 |
| T*C*G*T*C*G*A*C*G*A*D*C*G*G*C*G*C*G*C*G*C*C*G | SEQ ID NO:339 |
| G*G*G-G-A-C-G-A-C-G-T-C-G-T-G-G*G*G*G*G*G | SEQ ID NO:340 |
| T*C-G-A-C-G-T-C-G-T-G-G*G*G*G | SEQ ID NO:341 |
| T*C*C*A*G*G*A*C*T*T*C*T*C*T*C*A | SEQ ID NO:342 |
| T*C*G*T*C*G*T*T*T*T*C*G*G*C*G*C*G*C*G*C*C*G | SEQ ID NO:343 |
| T*C* G*T*C-mG*mA*C*mG*mA*T*C*mG*mG*C*mG*C*mG*C*mG*C*C*3mG | SEQ ID NO:344 |
| T*C*mG*T*C*mG*mA*C*mG*mA*T*C*mG*mG*C*mG*C*mG*C*mG*C*C*3mG | SEQ ID NO:345 |
| T*C* G*T*C-mG*mA*C-mG*mA*T*C-mG*mG*C*mG*C-mG*C*mG*C*C*3mG | SEQ ID NO:346 |
| T*C-mG*T*C-mG*mA*C-mG*mA*T*C-mG*mG*C*mG*C-mG*C*mG*C*C*3mG | SEQ ID NO:347 |

Symbols used in table 1:

| | |
|---|---|
| * | Stabilized internucleotide linkage |
| - | Phosphodiester linkage |
| chol | Cholesterol |
| vitE | Vitamin E |
| biot | Biotin |
| teg | Triethylene glycol |
| dig | Digoxygenin |
| but | butyrate |
| J | 1,3-propane-diol |
| L | hexaethylene glycol |
| D | D spacer (1'2'-dideoxyribose, Glen Research, Sterling, VA) |
| mN | 2'-O-methyl nucleoside |
| iN | Inverse nucleotide (inverse orientation: 3' and 5' switched) |
| doub- | doubler |
| Z | 5-methyl-deoxycytidine |
| rN | ribonucleoside |
| Cy3 | Bis-hydroxypropyl-3,3,3',3'-tetramethyl-4,5-benzindocarbocyanine chloride (Glen Research) |

As discussed above, the measure of the duplex stability of a double stranded molecule is dependent on total strand concentration, base composition, temperature, pH and buffer salts. Under physiological conditions the formation of a duplex is preferred over unpaired stretches of bases. If more than one palindromic, complementarity containing, or duplex forming sequence is present in one molecule, potential aggregation of several molecules under physiological conditions will be preferred. The aggregation leads to a complex mixture of higher-ordered oligonucleotide structures which are difficult to analyze and also create a problem with respect to lot-to-lot consistency of final drug product dosing solutions. To prevent such aggregation, elevation of temperature, pH or reduction of buffer salts could be used. However, when the molecules are formulated for the intended treatment of animals or humans, elevation of temperature, pH or reduction of buffer salts can't be used, since physiological conditions (ambient temperature, physiological osmotic value, or neutral pH) have to be maintained. The invention also includes compositions for addressing these problems.

Thus, in some aspects, the disclosure relates to a composition of duplex forming oligonucleotides that are formulated in a manner to reduce *in vitro* aggregation, without inhibiting the *in vivo* self-assembly into concatamers. It has been discovered that a low salt buffer and a solute can be used to maintain the duplex forming oligonucleotides in a substantially homogenous, or non-aggregated state. In these pharmaceutical formulations the compounds are more useful and more practical for therapeutic development and are more acceptable to regulatory agencies because the structures are easier to analyze and it is simpler to produce more consistent lots for dosing. Such formulations are more useful for pharmaceutical dosing solutions because they avoid the complex concatamers that complicate drug analysis and reduce lot-to-lot consistency.

In some embodiments, molecules which contain two or more palindromic or duplex forming regions with significantly different melting temperatures are used. Under certain conditions the formation of the weaker duplex (i.e. the one with lower duplex stability) can be prevented, whereas formation of the stronger duplex is maintained, so that *in vitro,* the compounds will form duplexes instead of concatamers, but the *in vivo* activity can be maintained.

Thus, the immunostimulatory oligonucleotides of the invention can be used in compositions suitable for administration to a subject. The duplex forming oligonucleotides can be used in preparation of a dosing solution of oligonucleotides which comprise dissolving said oligonucleotides in a hypo-osmolal buffer, such as a low salt buffer, and adding a solute. A "solute" as used herein is an ingredient that, when added to the solution at the proper concentration, results in an approximately isotonic formulation in which the oligonucleotides are present in a substantially homogeneous form. In some embodiments the solute is an isotonic forming component. Examples of suitable solutes include but are not limited to alcohols such as amino acids and saccharides. Amino acids useful as solutes may have a hydrophobic side chain, such as isoleucine or charged side chain, such as lysine. In some embodiments the amino acid is glycine. Saccharides include but are not limited to dextrose, fructose, lactose, sucrose, ribose, arabinose or a disaccharide.

As used herein, the term "substantially homogeneous" refers to a solution in which the majority of the molecules are not present in high molecular weight concatamers. Thus, at least 50% of the molecules in the solution are not part of a high molecular weight concatamer. In other embodiments at least 40%, 30%, 20%, 10%, 5%, 2% or 1% of the molecules in the solution are not part of a high molecular weight concatamer. The oligonucleotides that are not part of the concatamer may be present in monomeric or dimeric form.

In some embodiments the composition includes oligonucleotides that all have the same sequence. Thus, multiple copies of the same oligonucleotide sequence are present in the composition. Alternatively, the composition can comprise a mixture of oligonucleotides of different sequences that have at least one duplex forming sequence in common with other oligonucleotides in the mixture. Typically a composition including different oligonucleotide sequences includes at least two different oligonucleotides having complementary duplex forming sequences such that the duplex forming sequences in the different oligonucleotides are complementary to one another and can form base pairs. The base pairings may be a perfect base paring or it may include imperfections, such as bulges or mismatches.

Of course, additional oligonucleotides that don't participate in the formation of the concatamer may be included in the composition whether the composition includes a single oligonucleotide with duplex forming sequences that are capable of base pairing with duplex forming sequences in the oligonucleotide of the same sequence or multiple oligonucleotide sequences.

The compositions of the invention are broadly applicable to the formulation of oligonucleotides. They are not limited to the formulation of immunostimulatory oligonucleotides. For instance, the compositions of the invention may be used to formulate therapeutic DNA, such as antisense oligonucleotides or RNA, such as siRNA oligonucleotides. In some embodiments the compositions are useful for formulating immunostimulatory oligonucleotides.

The immunostimulatory oligonucleotides generally have a length in the range of between 6 and 100 nucleotides. In some embodiments the length is in the range of 6-40, 13-100, 13-40, 13-30, 15-50, or 15-30 nucleotides or any integer range therebetween.

The terms "nucleic acid" and "oligonucleotide" are used interchangeably to mean multiple nucleotides (i.e., molecules including a sugar (e.g., ribose or deoxyribose) linked to a phosphate group and to an exchangeable organic base, which is either a substituted pyrimidine (e.g., cytosine (C), thymine (T) or uracil (U)) or a substituted purine (e.g., adenine (A) or guanine (G)). As used herein, the terms "nucleic acid" and "oligonucleotide" refer to oligoribonucleotides as well as oligodeoxyribonucleotides. The terms "nucleic acid" and "oligonucleotide" shall also include polynucleosides (i.e., a polynucleotide minus the phosphate) and any other organic base containing polymer. Nucleic acid molecules can be obtained from existing nucleic acid sources (e.g., genomic or cDNA), but are preferably synthetic (e.g., produced by nucleic acid synthesis).

The terms "nucleic acid" and "oligonucleotide" as used herein shall encompass nucleic acid molecules and oligonucleotides of the invention, as well as oligonucleotide analogs of the invention. The terms "oligodeoxynucleotide" and, equivalently, "ODN" as used herein shall encompass unmodified oligodeoxynucleotides of the invention as well as oligodeoxynucleotide analogs of the invention.

The terms "nucleic acid" and "oligonucleotide" also encompass nucleic acids or oligonucleotides with substitutions or modifications, such as in the bases and/or sugars. For example, they include nucleic acids having backbone sugars that are covalently attached to low molecular weight organic groups other than a hydroxyl group at the 2' position and other than a phosphate group or hydroxy group at the 5' position. Thus modified nucleic acids may include a 2'-O-alkylated ribose group. In addition, modified nucleic acids may include sugars such as arabinose or 2'-fluoroarabinose instead of ribose. Thus the nucleic acids may be heterogeneous in backbone composition thereby containing any possible combination of polymer units linked together such as peptide-nucleic acids (which have a peptide-like backbone with nucleic acid bases). In some embodiments the oligonucleotide may have one or more modifications, wherein each modification is located at a particular phosphodiester internucleoside bridge and/or at a particular β-D-ribose unit and/or at a particular natural nucleoside base position in comparison to an oligonucleotide of the same sequence which is composed of natural DNA. Other examples are described in more detail below.

The immunostimulatory oligonucleotides of the instant invention can encompass various chemical modifications and substitutions, in comparison to natural RNA and DNA, involving a phosphodiester internucleoside bridge, a β-D-ribose unit and/or a natural nucleoside base (such as adenine, guanine, cytosine, thymine, or uracil). Examples of chemical modifications are known to the skilled person and are described, for example, in Uhlmann E et al. (1990) Chem Rev 90:543; "Protocols for Oligonucleotides and Analogs" Synthesis and Properties & Synthesis and Analytical Techniques, S. Agrawal, Ed, Humana Press, Totowa, USA 1993; Crooke ST et al. (1996) Annu Rev Pharmacol Toxicol 36:107-29; and Hunziker J et al. (1995) Mod Synth Methods 7:331-417. An oligonucleotide according to the invention may have one or more modifications, wherein each modification is located at a particular phosphodiester internucleoside bridge and/or at a particular β-D-ribose unit and/or at a particular natural nucleoside base position in comparison to an oligonucleotide of the same sequence which is composed of natural DNA or RNA.

For example, the oligonucleotides may include one or more modifications and wherein each modification is independently selected from:
a) the replacement of a phosphodiester internucleoside bridge located at the 3' and/or the 5' end of a nucleoside by a modified internucleoside bridge,
b) the replacement of phosphodiester bridge located at the 3' and/or the 5' end of a nucleoside by a dephospho bridge,
c) the replacement of a sugar phosphate unit from the sugar phosphate backbone by another unit,
d) the replacement of a β-D-ribose unit by a modified sugar unit, and
e) the replacement of a natural nucleoside base by a modified nucleoside base.

More detailed examples for the chemical modification of an oligonucleotide are as follows.

The oligonucleotides may include modified internucleotide linkages, such as those described in a or b above. These modified linkages may be partially resistant to degradation (e.g., are stabilized). A stabilized oligonucleotide molecule shall mean an oligonucleotide that is relatively resistant to *in vivo* degradation (e.g., via an exo- or endo-nuclease) resulting form such modifications.

Oligonucleotides having phosphorothioate linkages, in some embodiments, may provide maximal activity and protect the oligonucleotide from degradation by intracellular exo- and endo-nucleases. A phosphodiester internucleoside bridge located at the 3' and/or the 5' end of a nucleoside can be replaced by a modified internucleoside bridge, wherein the modified internucleoside bridge is for example selected from phosphorothioate, phosphorodithioate, NR¹R²-phosphoramidate, boranophosphate, α-hydroxybenzyl phosphonate, phosphate-(C₁-C₂₁)-O-alkyl ester, phosphate-[(C₆-C₁₂)aryl-(C₁-C₂₁)-O-alkyl]ester, (C₁-C₈)alkylphosphonate and/or (C₆-C₁₂)arylphosphonate bridges, (C₇-C₁₂)-α-hydroxymethyl-aryl (e.g., disclosed in WO 95/01363), wherein (C₆-C₁₂)aryl, (C₆-C₂₀)aryl and (C₆-C₁₄)aryl are optionally substituted by halogen, alkyl, alkoxy, nitro, cyano, and where R¹ and R² are, independently of each other, hydrogen, (C₁-C₁₈)-alkyl, (C₆-C₂₀)-aryl, (C₆-C₁₄)-aryl-(C₁-C₈)-alkyl, preferably hydrogen, (C₁-C₈)-alkyl, preferably (C₁-C₄)-alkyl and/or methoxyethyl, or R¹ and R² form, together with the nitrogen atom carrying them, a 5-6-membered heterocyclic ring which can additionally contain a further heteroatom from the group O, S and N.

The replacement of a phosphodiester bridge located at the 3' and/or the 5' end of a nucleoside by a dephospho bridge (dephospho bridges are described, for example, in Uhlmann E and Peyman A in "Methods in Molecular Biology", Vol. 20, "Protocols for Oligonucleotides and Analogs", S. Agrawal, Ed., Humana Press, Totowa 1993, Chapter 16, pp. 355 ff), wherein a dephospho bridge is for example selected from the dephospho bridges formacetal, 3'-thioformacetal, methylhydroxylamine, oxime, methylenedimethyl-hydrazo, dimethylenesulfone and/or silyl groups.

A sugar phosphate unit (i.e., a β-D-ribose and phosphodiester internucleoside bridge together forming a sugar phosphate unit) from the sugar phosphate backbone (i.e., a sugar phosphate backbone is composed of sugar phosphate units) can be replaced by another unit, wherein the other unit is for example suitable to build up a "morpholino-derivative" oligomer (as described, for example, in Stirchak EP et al. (1989) Nucleic Acids Res 17:6129-41), that is, e.g., the replacement by a morpholino-derivative unit; or to build up a polyamide nucleic acid ("PNA"; as described for example, in Nielsen PE et al. (1994) Bioconjug Chem 5:3-7), that is, e.g., the replacement by a PNA backbone unit, e.g., by 2-aminoethylglycine. The oligonucleotide may have other carbohydrate backbone modifications and replacements, such as peptide nucleic acids with phosphate groups (PHONA), locked nucleic acids (LNA), and oligonucleotides having backbone sections with alkyl linkers or amino linkers. The alkyl linker may be branched or unbranched, substituted or unsubstituted, and chirally pure or a racemic mixture.

A β-ribose unit or a β-D-2'-deoxyribose unit can be replaced by a modified sugar unit, wherein the modified sugar unit is for example selected from β-D-ribose, α-D-2'-deoxyribose, L-2'-deoxyribose, 2'-F-2'-deoxyribose, 2'-F-arabinose, 2'-O-(C₁-C₆)alkyl-ribose, preferably 2'-O-(C₁-C₆)alkyl-ribose is 2'-O-methylribose, 2'-O-(C₂-C₆)alkenyl-ribose, 2'-[O-(C₁-C₆)alkyl-O-(C₁-C₆)alkyl]-ribose, 2'-NH₂-2'-deoxyribose, β-D-xylo-furanose, α-arabinofuranose, 2,4-dideoxy-β-D-erythro-hexo-pyranose, and carbocyclic (described, for example, in Froehler (1992) J Am Chem Soc 114:8320) and/or open-chain sugar analogs (described, for example, in Vandendriessche et al. (1993) Tetrahedron 49:7223) and/or bicyclosugar analogs (described, for example, in Tarkov M et al. (1993) Helv Chim Acta 76:481).

In some embodiments the sugar is 2'-O-methylribose, particularly for one or both nucleotides linked by a phosphodiester or phosphodiester-like internucleoside linkage.

The immunostimulatory nucleic acid molecules of the instant invention may include chimeric backbones. For purposes of the instant invention, a chimeric backbone refers to a partially stabilized backbone, wherein at least one internucleotide linkage is phosphodiester or phosphodiester-like, and wherein at least one other internucleotide linkage is a stabilized internucleotide linkage, wherein the at least one phosphodiester or phosphodiester-like linkage and the at least one stabilized linkage are different. Since boranophosphonate linkages have been reported to be stabilized relative to phosphodiester linkages, for purposes of the chimeric nature of the backbone, boranophosphonate linkages can be classified either as phosphodiester-like or as stabilized, depending on the context. For example, a chimeric backbone according to the instant invention could in one embodiment include at least one phosphodiester (phosphodiester or phosphodiester-like) linkage and at least one boranophosphonate (stabilized) linkage. In another embodiment a chimeric backbone according to the instant invention could include boranophosphonate (phosphodiester or phosphodiester-like) and phosphorothioate (stabilized) linkages. A "stabilized internucleotide linkage" shall mean an internucleotide linkage that is relatively resistant to *in vivo* degradation (e.g., via an exo- or endo-nuclease), compared to a phosphodiester internucleotide linkage. Preferred stabilized internucleotide linkages include, without limitation, phosphorothioate, phosphorodithioate, methylphosphonate, and methylphosphorothioate. Other stabilized internucleotide linkages include, without limitation: peptide, alkyl, dephospho, and others as described above.

In particular, phosphodiester or phosphodiester-like internucleotide linkages involve "internal dinucleotides". An internal dinucleotide in general shall mean any pair of adjacent nucleotides connected by an internucleotide linkage, in which neither nucleotide in the pair of nucleotides is a terminal nucleotide, i.e., neither nucleotide in the pair of nucleotides is a nucleotide defining the 5' or 3' end of the oligonucleotide. Thus a linear oligonucleotide that is n nucleotides long has a total of n-1 dinucleotides and only n-3 internal dinucleotides. Each internucleotide linkage in an internal dinucleotide is an internal internucleotide linkage. Thus a linear oligonucleotide that is n nucleotides long has a total of n-1 internucleotide linkages and only n-3 internal internucleotide linkages. The strategically placed phosphodiester or phosphodiester-like internucleotide linkages, therefore, refer to phosphodiester or phosphodiester-like internucleotide linkages positioned between any pair of nucleotides in the nucleic acid sequence. In some embodiments the phosphodiester or phosphodiester-like internucleotide linkages are not positioned between either pair of nucleotides closest to the 5' or 3' end.

A phosphodiester internucleotide linkage is the type of linkage characteristic of nucleic acids found in nature. The phosphodiester internucleotide linkage includes a phosphorus atom flanked by two bridging oxygen atoms and bound also by two additional oxygen atoms, one charged and the other uncharged. Phosphodiester internucleotide linkage is particularly preferred when it is important to reduce the tissue half-life of the oligonucleotide.

A phosphodiester-like internucleotide linkage is a phosphorus-containing bridging group that is chemically and/or diastereomerically similar to phosphodiester. Measures of similarity to phosphodiester include susceptibility to nuclease digestion and ability to activate RNase H. Thus for example phosphodiester, but not phosphorothioate, oligonucleotides are susceptible to nuclease digestion, while both phosphodiester and phosphorothioate oligonucleotides activate RNase H. In a preferred embodiment the phosphodiester-like internucleotide linkage is boranophosphate (or equivalently, boranophosphonate) linkage. U.S. Patent No. 5,177,198; U.S. Patent No. 5,859,231; U.S. Patent No. 6,160,109; U.S. Patent No. 6,207,819; Sergueev et al., (1998) J Am Chem Soc 120:9417-27. In another preferred embodiment the phosphodiester-like internucleotide linkage is diasteromerically pure Rp phosphorothioate. It is believed that diasteromerically pure Rp phosphorothioate is more susceptible to nuclease digestion and is better at activating RNase H than mixed or diastereomerically pure Sp phosphorothioate. Stereoisomers of CpG oligonucleotides are the subject of co-pending U.S. patent application 09/361,575 filed July 27, 1999, and published PCT application PCT/US99/17100 (WO 00/06588). It is to be noted that for purposes of the instant invention, the term "phosphodiester-like internucleotide linkage" specifically excludes phosphorodithioate and methylphosphonate internucleotide linkages.

Modified backbones such as phosphorothioates may be synthesized using automated techniques employing either phosphoramidate or H-phosphonate chemistries. Aryl- and alkyl-phosphonates can be made, e.g., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described. Uhlmann E et al. (1990) Chem Rev 90:544; Goodchild J (1990) Bioconjugate Chem 1:165. Methods for preparing chimeric oligonucleotides are also known. For instance patents issued to Uhlmann et al have described such techniques.

Mixed backbone modified oligonucleotides may be synthesized using a commercially available DNA synthesizer and standard phosphoramidite chemistry. (F. E. Eckstein, "Oligonucleotides and Analogues - A Practical Approach" IRL Press, Oxford, UK, 1991, and M. D. Matteucci and M. H. Caruthers, Tetrahedron Lett. 21, 719 (1980)). After coupling, PS linkages are introduced by sulfurization using the Beaucage reagent (R. P. Iyer, W. Egan, J. B. Regan and S. L. Beaucage, J. Am. Chem. Soc. 112, 1253 (1990)) (0.075 M in acetonitrile) or phenyl acetyl disulfide (PADS) followed by capping with acetic anhydride, 2,6-lutidine in tetrahydrofurane (1:1:8; v:v:v) and *N*-methylimidazole (16 % in tetrahydrofurane). This capping step is performed after the sulfurization reaction to minimize formation of undesired phosphodiester (PO) linkages at positions where a phosphorothioate linkage should be located. In the case of the introduction of a phosphodiester linkage, e.g. at a CpG dinucleotide, the intermediate phosphorous-III is oxidized by treatment with a solution of iodine in water/pyridine. After cleavage from the solid support and final deprotection by treatment with concentrated ammonia (15 hrs at 50°C), the ODN are analyzed by HPLC on a GenPak™ Fax column (Millipore-Waters) using a NaCl-gradient (e.g. buffer A: 10 mM NaH₂PO₄ in acetonitrile/water = 1:4/v:v pH 6.8; buffer B: 10 mM NaH₂PO₄, 1.5 M NaCl in acetonitrile/water = 1:4/v:v; 5 to 60 % B in 30 minutes at 1 ml/min) or by capillary gel electrophoresis. The ODN can be purified by HPLC or by FPLC on a Source High Performance column (Amersham Pharmacia). HPLC-homogeneous fractions are combined and desalted *via* a C18 column or by ultrafiltration. The ODN was analyzed by MALDI-TOF mass spectrometry to confirm the calculated mass.

The nucleic acids of the invention can also include other modifications. These include nonionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. Nucleic acids which contain diol, such as tetraethyleneglycol or hexaethylene glycol, at either or both termini have also been shown to be substantially resistant to nuclease degradation.

Nucleic acids also include substituted purines and pyrimidines such as C-5 propyne pyrimidine and 7-deaza-7-substituted purine modified bases. Wagner RW et al. (1996) Nat Biotechnol 14:840-4. Purines and pyrimidines include but are not limited to adenine, cytosine, guanine, thymine, and uracil and other naturally and non-naturally occurring nucleobases, substituted and unsubstituted aromatic moieties.

A modified base is any base which is chemically distinct from the naturally occurring bases typically found in DNA and RNA such as T, C, G, A, and U, but which share basic chemical structures with these naturally occurring bases. The modified nucleoside base may be, for example, selected from hypoxanthine, uracil, dihydrouracil, pseudouracil, 2-thiouracil, 4-thiouracil, 5-aminouracil, 5-(C₁-C₆)-alkyluracil, 5-(C₂-C₆)-alkenyluracil, 5-(C₂-C₆)-alkynyluracil, 5-(hydroxymethyl)uracil, 5-chlorouracil, 5-fluorouracil, 5-bromouracil, 5-hydroxycytosine, 5-(C₁-C₆)-alkylcytosine, 5-(C₂-C₆)-alkenylcytosine, 5-(C₂-C₆)-alkynylcytosine, 5-chlorocytosine, 5-fluorocytosine, 5-bromocytosine, N²-dimethylguanine, 2,4-diamino-purine, 8-azapurine, a substituted 7-deazapurine, preferably 7-deaza-7-substituted and/or 7-deaza-8-substituted purine, 5-hydroxymethylcytosine, N4-alkylcytosine, e.g., N4-ethylcytosine, 5-hydroxydeoxycytidine, 5-hydroxymethyldeoxycytidine, N4-alkyldeoxycytidine, e.g., N4-ethyldeoxycytidine, 6-thiodeoxyguanosine, and deoxyribonucleosides of nitropyrrole, C5-propynylpyrimidine, and diaminopurine e.g., 2,6-diaminopurine, inosine, 5-methylcytosine, 2-aminopurine, 2-amino-6-chloropurine, hypoxanthine or other modifications of a natural nucleoside bases. This list is meant to be exemplary and is not to be interpreted to be limiting.

In particular formulas described herein modified bases may be incorporated. For instance a cytosine may be replaced with a modified cytosine. A modified cytosine as used herein is a naturally occurring or non-naturally occurring pyrimidine base analog of cytosine which can replace this base without impairing the immunostimulatory activity of the oligonucleotide. Modified cytosines include but are not limited to 5-substituted cytosines (e.g., 5-methyl-cytosine, 5-fluoro-cytosine, 5-chloro-cytosine, 5-bromo-cytosine, 5-iodo-cytosine, 5-hydroxy-cytosine, 5-hydroxymethyl-cytosine, 5-difluoromethyl-cytosine, and unsubstituted or substituted 5-alkynyl-cytosine), 6-substituted cytosines (e.g., 6-hydroxy-cytosine), N4-substituted cytosines (e.g., N4-ethyl-cytosine), 5-aza-cytosine, 2-mercapto-cytosine, isocytosine, pseudo-isocytosine, cytosine analogs with condensed ring systems (e.g., N,N'-propylene cytosine or phenoxazine), and uracil and its derivatives (e.g., 5-fluoro-uracil, 5-bromo-uracil, 5-bromovinyl-uracil, 4-thio-uracil, 5-hydroxy-uracil, 5-propynyl-uracil). Some of the preferred cytosines include 5-methyl-cytosine, 5-fluoro-cytosine, 5-hydroxy-cytosine, 5-hydroxymethyl-cytosine, and N4-ethyl-cytosine. In another embodiment of the invention, the cytosine base is substituted by a universal base (e.g., 3-nitropyrrole, P-base), an aromatic ring system (e.g., fluorobenzene or difluorobenzene) or a hydrogen atom (dSpacer).

A guanine may be replaced with a modified guanine base. A modified guanine as used herein is a naturally occurring or non-naturally occurring purine base analog of guanine which can replace this base without impairing the immunostimulatory activity of the oligonucleotide. Modified guanines include but are not limited to 7-deazaguanine, 7-deaza-7-substituted guanine (such as 7-deaza-7-(C2-C6)alkynylguanine), 7-deaza-8-substituted guanine, hypoxanthine, N2-substituted guanines (e.g., N2-methyl-guanine), 5-amino-3-methyl-3H,6H-thiazolo[4,5-d]pyrimidine-2,7-dione, 2,6-diaminopurine, 2-aminopurine, purine, indole, adenine, substituted adenines (e.g., N6-methyl-adenine, 8-oxo-adenine), 8-substituted guanine (e.g., 8-hydroxyguanine and 8-bromoguanine), and 6-thioguanine. In another embodiment of the invention, the guanine base is substituted by a universal base (e.g., 4-methyl-indole, 5-nitro-indole, and K-base), an aromatic ring system (e.g., benzimidazole or dichloro- benzimidazole, 1-methyl-1H-[1,2,4]triazole-3-carboxylic acid amide) or a hydrogen atom (dSpacer).

The immunostimulatory oligonucleotides may also contain one or more unusual linkages between the nucleotide or nucleotide-analog moieties. The usual internucleoside linkage is the 3'5'-linkage. All other linkages are considered as unusual internucleoside linkages, such as 2'5'-, 5'5'-, 3'3'-, 2'2'-, and 2'3'-linkages. Thereby, the nomenclature 2' to 5' is chosen according to the carbon atom of ribose. However, if unnatural sugar moieties are employed, such as ring-expanded sugar analogs (e.g., hexanose, cylohexene, or pyranose) or bi- or tricyclic sugar analogs, then this nomenclature changes according to the nomenclature of the monomer. In 3'-deoxy-β-D-ribopyranose analogs (also called p-DNA) the mononucleotides are connected, for example, via a 4'2'-linkage.

In principle, linkages between different parts of an oligonucleotide or between different oligonucleotides, respectively, can occur via all parts of the molecule, as long as this does not negatively interfere with the recognition by its receptor. According to the nature of the nucleic acid, the linkage can involve the sugar moiety (Su), the heterocyclic nucleobase (Ba) or the phosphate backbone (Ph). Thus, linkages of the type Su-Su, Su-Ph, Su-Ba, Ba-Ba, Ba-Su, Ba-Ph, Ph-Ph, Ph-Su, and Ph-Ba are possible. If the oligonucleotides are further modified by certain non-nucleotidic substituents, the linkage can also occur via the modified parts of the oligonucleotides. These modifications include also modified nucleic acids, e.g., PNA, LNA, or morpholino oligonucleotide analogs.

The linkages are preferably composed of C, H, N, O, S, B, P, and halogen, containing 3 to 300 atoms. An example with 3 atoms is an acetal linkage (ODN1-3'-O-CH₂-O-3'-ODN2; Froehler and Matteucci) connecting e.g. the 3'-hydroxy group of one nucleotide to the 3'-hydroxy group of a second oligonucleotide. An example with about 300 atoms is PEG-40 (tetraconta polyethyleneglycol). Preferred linkages are phosphodiester, phosphorothioate, methylphosphonate, phosphoramidate, boranophosphonate, amide, ether, thioether, acetal, thioacetal, urea, thiourea, sulfonamide, Schiff base, and disulfide linkages. Another possibility is the use of the Solulink BioConjugation System (TriLink BioTechnologies, San Diego, CA).

The immunostimulatory oligonucleotides of the invention can also be conjugated to a lipophilic group. A "lipophilic group" as used herein is a chemical functional group with a chemical affinity for lipid or nonpolar molecules. In some embodiments the lipophilic group is cholesterol.

The immunostimulatory oligonucleotides of the instant invention are useful for inducing a Th1-like immune response. They are believed to be of particular use in any condition calling for prolonged or repeated administration of immunostimulatory oligonucleotide for any purpose. Accordingly, the immunostimulatory oligonucleotides of the instant invention are useful as adjuvants for vaccination, and they are useful for treating diseases including cancer, infectious disease, allergy, and asthma.

Cancer is a disease which involves the uncontrolled growth (i.e., division) of cells. Some of the known mechanisms which contribute to the uncontrolled proliferation of cancer cells include growth factor independence, failure to detect genomic mutation, and inappropriate cell signaling. The ability of cancer cells to ignore normal growth controls may result in an increased rate of proliferation. Although the causes of cancer have not been firmly established, there are some factors known to contribute, or at least predispose a subject, to cancer. Such factors include particular genetic mutations (e.g., BRCA gene mutation for breast cancer, APC for colon cancer), exposure to suspected cancer-causing agents, or carcinogens (e.g., asbestos, UV radiation) and familial disposition for particular cancers such as breast cancer.

The cancer may be a malignant or non-malignant cancer. Cancers or tumors include but are not limited to biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g. small cell and non-small cell); melanoma; neuroblastomas; oral cancer; ovarian cancer; pancreas cancer; prostate cancer; rectal cancer; sarcomas; skin cancer; testicular cancer; thyroid cancer; and renal cancer, as well as other carcinomas and sarcomas. In one embodiment the cancer is hairy cell leukemia, chronic myelogenous leukemia, cutaneous T-cell leukemia, multiple myeloma, follicular lymphoma, malignant melanoma, squamous cell carcinoma, renal cell carcinoma, prostate carcinoma, bladder cell carcinoma, or colon carcinoma.

A subject having a cancer is a subject that has detectable cancerous cells. A "subject in need" of cancer treatment is a subject that has detectable cancerous cells or is a subject at risk of developing a cancer.

A subject at risk of developing a cancer is one who has a higher than normal probability of developing cancer. These subjects include, for instance, subjects having a genetic abnormality that has been demonstrated to be associated with a higher likelihood of developing a cancer, subjects having a familial disposition to cancer, subjects exposed to cancer causing agents (i.e., carcinogens) such as tobacco, asbestos, or other chemical toxins, and subjects previously treated for cancer and in apparent remission.

The CpG immunostimulatory oligonucleotides may also be administered in conjunction with a traditional anti-cancer treatment. "Traditional anti-cancer treatment" as used herein, refers to cancer medicaments, radiation and surgical procedures. As used herein, a "cancer medicament" refers to an agent which is administered to a subject for the purpose of treating a cancer. As used herein, "treating cancer" includes preventing the development of a cancer, reducing the symptoms of cancer, and/or inhibiting the growth of an established cancer. In other aspects, the cancer medicament is administered to a subject at risk of developing a cancer for the purpose of reducing the risk of developing the cancer. Various types of medicaments for the treatment of cancer are described herein. For the purpose of this specification, cancer medicaments are classified as chemotherapeutic agents, immunotherapeutic agents, cancer vaccines, hormone therapy, and biological response modifiers. In some embodiments the anti-cancer medicament can be linked to the immunostimulatory oligonucleotide.

Additionally, the methods of the disclosure are intended to embrace the use of more than one cancer medicament along with the CpG immunostimulatory oligonucleotides. As an example, where appropriate, the CpG immunostimulatory oligonucleotides may be administered with both a chemotherapeutic agent and an immunotherapeutic agent or an anti-angiogenic agent. The combination of the P-Class CpG ODN and an antiangiogenic agent may include multiple combinations with one or more other anti-cancer therapies. Alternatively, the cancer medicament may embrace an immunotherapeutic agent and a cancer vaccine, or a chemotherapeutic agent and a cancer vaccine, or a chemotherapeutic agent, an immunotherapeutic agent and a cancer vaccine all administered to one subject for the purpose of treating a subject having a cancer or at risk of developing a cancer. In some embodiments the treatment further comprises an antibody.

The chemotherapeutic agent may be selected from the group consisting of methotrexate, vincristine, adriamycin, cisplatin, non-sugar containing chloroethylnitrosoureas, 5-fluorouracil, mitomycin C, bleomycin, doxorubicin, dacarbazine, taxol, fragyline, Meglamine GLA, valrubicin, carmustaine and poliferposan, MMI270, BAY 12-9566, RAS farnesyl transferase inhibitor, farnesyl transferase inhibitor, MMP, MTA/LY231514, LY264618/Lometexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Paclitaxel, Taxol/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara/Fludarabine, Pharmarubicin/Epirubicin, DepoCyt, ZD1839, LU 79553/Bis-Naphtalimide, LU 103793/Dolastain, Caetyx/liposomal doxorubicin, Gemzar/Gemcitabine, ZD 0473/Anormed, YM 116, lodine seeds, CDK4 and CDK2 inhibitors, PARP inhibitors, D4809/Dexifosamide, Ifes/Mesnex/Ifosamide, Vumon/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, cisplatin, Cytarabine HCl, Dactinomycin, Daunorubicin HCl, Estramustine phosphate sodium, Etoposide (VP16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analog), Lomustine (CCNU), Mechlorethamine HCl (nitrogen mustard), Mercaptopurine, Mesna, Mitotane (o.p'-DDD), Mitoxantrone HCl, Octreotide, Plicamycin, Procarbazine HCl, Streptozocin, Tamoxifen citrate, Thioguanine, Thiotepa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26), and Vindesine sulfate, but it is not so limited.

The immunotherapeutic or anti-angiogenic agent may be selected from the group consisting of Rituxan, Ributaxin, Herceptin, Quadramet, Panorex, IDEC-Y2B8, BEC2, C225, Oncolym, SMART M195, ATRAGEN, Ovarex, Bexxar, LDP-03, ior t6, MDX-210, MDX-11, MDX-22, OV103, 3622W94, anti-VEGF, anti-CTLA-4, Avastin, anti-EGFR, Iressa, Zenapax, MDX-220, MDX-447, MELIMMUNE-2, MELIMMUNE-1, CEACIDE, Pretarget, NovoMAb-G2, TNT, Gliomab-H, GNI-250, EMD-72000, LymphoCide, CMA 676, Monopharm-C, 4B5, ior egf.r3, ior c5, BABS, anti-FLK-2, MDX-260, ANA Ab, SMART 1D10 Ab, SMART ABL 364 Ab, and ImmuRAIT-CEA, but it is not so limited.

The cancer vaccine may be selected from the group consisting of EGF, Anti-idiotypic cancer vaccines, Gp75 antigen, GMK melanoma vaccine, MGV ganglioside conjugate vaccine, Her2/neu, Ovarex, M-Vax, O-Vax, L-Vax, STn-KHL theratope, BLP25 (MUC-1), liposomal idiotypic vaccine, Melacine, peptide or recombinant protein antigen vaccines, toxin/antigen vaccines, MVA-based vaccine, PACIS, BCG vaccine, TA-HPV, TA-CIN, DISC-virus, conjugates of one or more tumor-associated antigens with other immune stimulatory proteins or molecules, and ImmuCyst/TheraCys, but it is not so limited.

The use of CpG immunostimulatory oligonucleotides in conjunction with immunotherapeutic agents such as monoclonal antibodies is able to increase long-term survival through a number of mechanisms including significant enhancement of ADCC (as discussed above), activation of NK cells and an increase in IFN-α levels. The nucleic acids when used in combination with monoclonal antibodies serve to reduce the dose of the antibody required to achieve a biological result.

In the instances when the CpG oligonucleotide is administered with an antigen, the subject may be exposed to the antigen. As used herein, the term "exposed to" refers to either the active step of contacting the subject with an antigen or the passive exposure of the subject to the antigen *in vivo*. Methods for the active exposure of a subject to an antigen are well-known in the art. In general, an antigen is administered directly to the subject by any means such as intravenous, intramuscular, oral, transdermal, mucosal, intranasal, intratracheal, or subcutaneous administration. The antigen can be administered systemically or locally. Methods for administering the antigen and the CpG immunostimulatory oligonucleotide are described in more detail below. A subject is passively exposed to an antigen if an antigen becomes available for exposure to the immune cells in the body. A subject may be passively exposed to an antigen, for instance, by entry of a foreign pathogen into the body or by the development of a tumor cell expressing a foreign antigen on its surface.

The methods in which a subject is passively exposed to an antigen can be particularly dependent on timing of administration of the CpG immunostimulatory oligonucleotide. For instance, in a subject at risk of developing a cancer or an infectious disease or an allergic or asthmatic response, the subject may be administered the CpG immunostimulatory oligonucleotide on a regular basis when that risk is greatest, e.g., during allergy season or after exposure to a cancer causing agent. Additionally the CpG immunostimulatory oligonucleotide may be administered to travelers before they travel to foreign lands where they are at risk of exposure to infectious agents. Likewise the CpG immunostimulatory oligonucleotide may be administered to soldiers or civilians at risk of exposure to biowarfare to induce a systemic or mucosal immune response to the antigen when and if the subject is exposed to it.

An antigen as used herein is a molecule capable of provoking an immune response. Antigens include but are not limited to cells, cell extracts, proteins, polypeptides, peptides, polysaccharides, polysaccharide conjugates, peptide and non-peptide mimics of polysaccharides and other molecules, small molecules, lipids, glycolipids, carbohydrates, viruses and viral extracts and multicellular organisms such as parasites and allergens. The term antigen broadly includes any type of molecule which is recognized by a host immune system as being foreign. Antigens include but are not limited to cancer antigens, microbial antigens, and allergens.

A cancer antigen as used herein is a compound, such as a peptide or protein, associated with a tumor or cancer cell surface and which is capable of provoking an immune response when expressed on the surface of an antigen presenting cell in the context of an MHC molecule. Cancer antigens can be prepared from cancer cells either by preparing crude extracts of cancer cells, for example, as described in Cohen PA et al. (1994) Cancer Res 54:1055-8, by partially purifying the antigens, by recombinant technology, or by de novo synthesis of known antigens. Cancer antigens include but are not limited to antigens that are recombinantly expressed, an immunogenic portion thereof, or a whole tumor or cancer cell. Such antigens can be isolated or prepared recombinantly or by any other means known in the art.

As used herein, the terms "cancer antigen" and "tumor antigen" are used interchangeably to refer to antigens which are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent (i.e., not expressed) in normal cells, those that are expressed only at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), or fusion proteins resulting from internal deletions or chromosomal translocations. Still other cancer antigens can be encoded by viral genes such as those carried on RNA and DNA tumor viruses.

Symptoms of asthma include recurrent episodes of wheezing, breathlessness, and chest tightness, and coughing, resulting from airflow obstruction. Airway inflammation associated with asthma can be detected through observation of a number of physiological changes, such as, denudation of airway epithelium, collagen deposition beneath basement membrane, edema, mast cell activation, inflammatory cell infiltration, including neutrophils, eosinophils, and lymphocytes. As a result of the airway inflammation, asthma patients often experience airway hyper-responsiveness, airflow limitation, respiratory symptoms, and disease chronicity. Airflow limitations include acute bronchoconstriction, airway edema, mucous plug formation, and airway remodeling, features which often lead to bronchial obstruction. In some cases of asthma, subbasement membrane fibrosis may occur, leading to persistent abnormalities in lung function.

Research over the past several years has revealed that asthma likely results from complex interactions among inflammatory cells, mediators, and other cells and tissues resident in the airway. Mast cells, eosinophils, epithelial cells, macrophage, and activated T-cells all play an important role in the inflammatory process associated with asthma (Djukanovic et al., Am. Rev. Respir. Dis; 142:434-457; 1990). It is believed that these cells can influence airway function through secretion of preformed and newly synthesized mediators which can act directly or indirectly on the local tissue. It has also been recognized that subpopulations of T-lymphocytes (Th2) play an important role in regulating allergic inflammation in the airway by releasing selective cytokines and establishing disease chronicity (Robinson, et al. N. Engl. J. Med.; 326:298-304; 1992).

Asthma is a complex disorder which arises at different stages in development and can be classified based on the degree of symptoms of acute, sub acute or chronic. An acute inflammatory response is associated with an early recruitment of cells into the airway. The sub acute inflammatory response involves the recruitment of cells as well as the activation of resident cells causing a more persistent pattern of inflammation. Chronic inflammatory response is characterized by a persistent level of cell damage and an ongoing repair process, which may result in permanent abnormalities in the airway.

A "subject having asthma" is a subject that has a disorder of the respiratory system characterized by inflammation, narrowing of the airways and increased reactivity of the airways to inhaled agents. Asthma is frequently, although not exclusively associated with atopic or allergic symptoms. An initiator is a composition or environmental condition which triggers asthma. Initiators include, but are not limited to, allergens, cold temperatures, exercise, viral infections, SO₂.

The oligonucleotides are also useful for redirecting an immune response from a Th2 immune response to a Th1 immune response. This results in the production of a relatively balanced Th1/Th2 environment. Redirection of an immune response from a Th2 to a Th1 immune response can be assessed by measuring the levels of cytokines produced in response to the nucleic acid (e.g., by inducing monocytic cells and other cells to produce Th1 cytokines, including IFN-α). The redirection or rebalance of the immune response from a Th2 to a Th1 response is particularly useful for the treatment of asthma. For instance, an effective amount for treating asthma can be that amount useful for redirecting a Th2 type of immune response that is associated with asthma to a Th1 type of response or a balanced Th1/Th2 environment. Th2 cytokines, especially IL-4 and IL-5 are elevated in the airways of asthmatic subjects. The CpG immunostimulatory oligonucleotides described herein cause an increase in Th1 cytokines which helps to rebalance the immune system, preventing or reducing the adverse effects associated with a predominately Th2 immune response.

Redirection of an immune response from a Th2 to a Th1 immune response can also be assessed by measuring the levels of specific isotypes of immunoglobulin. For example, in mice IgG2a is associated with a Th1 immune response, and IgG1 and IgE are associated with a Th2 immune response.

As used herein, "asthma exacerbated by viral infection" refers to the increase in asthma symptoms and/or symptom severity during or following a viral infection. Viral respiratory infections can exacerbate the symptoms and/or development of asthma, especially in young children, by increasing the amount of T_{H}2 cytokines present. Respiratory viral infections caused by rhinoviruses, coronaviruses, influenza, parainfluenza and respiratory syncytial viruses (RSVs) are common triggers of asthma attacks and can cause increased wheezing and symptoms in asthma patients.

The immunostimulatory oligonucleotides of the instant invention can be used to treat airway remodeling in asthma or allergy patients. Remodeling of structural and functional tissues in the lungs is a significant morbidity factor for chronic asthmatics. As used herein, "airway remodeling" refers to the changes in lung tissue that occur with the chronic inflammation present in the lungs of chronic asthmatics. These changes can include increased collagen deposition and airway smooth muscle bulk, mast cell and goblet cell hyperplasia and epithelial cell hypertrophy. As a result of such changes the structure of the airway walls can change, causing blockage that in some cases cannot be completely reversed with treatment. Instead of or in addition to asthma a subject in need of treatment for airway remodeling may have chronic obstructive pulmonary disease or is a smoker. In some embodiments the subject is free of symptoms of asthma.

The immunostimulatory oligonucleotides of the instant invention can be used to treat allergy. An "allergy" refers to acquired hypersensitivity to a substance (allergen). Allergic conditions include but are not limited to eczema, "allergic rhinitis" or coryza, hay fever, conjunctivitis, ocular allergy, bronchial asthma, urticaria (hives) and food allergies, and other atopic conditions atopic dermatitis; anaphylaxis; drug allergy; angioedema; and allergic conjunctivitis. Allergic diseases in dogs include but are not limited to seasonal dermatitis; perennial dermatitis; rhinitis: conjunctivitis; allergic asthma; and drug reactions. Allergic diseases in cats include but are not limited to dermatitis and respiratory disorders; and food allergens. Allergic diseases in horses include but are not limited to respiratory disorders such as "heaves" and dermatitis. Allergic diseases in non-human primates include but are not limited to allergic asthma, ocular allergy, and allergic dermatitis.

Allergy is a disease associated with the production of antibodies from a particular class of immunoglobulin, IgE, against allergens. The development of an IgE-mediated response to common aeroallergens is also a factor which indicates predisposition towards the development of asthma. If an allergen encounters a specific IgE which is bound to an Fc IgE receptor on the surface of a basophil (circulating in the blood) or mast cell (dispersed throughout solid tissue), the cell becomes activated, resulting in the production and release of mediators such as histamine, scrotonin, and lipid mediators. Allergic diseases include but are not limited to rhinitis (hay fever) asthma, urticaria and atopic dermatitis.

A subject having an allergy is a subject that is currently experiencing or has previously experienced an allergic reaction in response to an allergen.

A subject at risk of developing an allergy or asthma is a subject that has been identified as having an allergy or asthma in the past but who is not currently experiencing the active disease as well as a subject that is considered to be at risk of developing asthma or allergy because of genetic or environmental factors. A subject at risk of developing allergy or asthma can also include a subject who has any risk of exposure to an allergen or a risk of developing asthma, i.e. someone who has suffered from an asthmatic attack previously or has a predisposition to asthmatic attacks. For instance, a subject at risk may be a subject who is planning to travel to an area where a particular type of allergen or asthmatic initiator is found or it may even be any subject living in an area where an allergen has been identified. If the subject develops allergic responses to a particular antigen and the subject may be exposed to the antigen, i.e., during pollen season, then that subject is at risk of exposure to the antigen.

Currently, allergic diseases are generally treated by the injection of small doses of antigen followed by subsequent increasing dosage of antigen. It is believed that this procedure induces tolerization to the allergen to prevent further allergic reactions. These methods, however, can take several years to be effective and are associated with the risk of side effects such as anaphylactic shock. The methods of the disclosure can be combined with traditional allergy treatments to increase their effectiveness and greatly decrease the time it takes for the effects to present in patients.

The symptoms of the allergic reaction vary, depending on the location within the body where the IgE reacts with the antigen. If the reaction occurs along the respiratory epithelium the symptoms are sneezing, coughing and asthmatic reactions. If the interaction occurs in the digestive tract, as in the case of food allergies, abdominal pain and diarrhea are common. Systematic reactions, for example following a bee sting, can be severe and often life threatening.

Delayed type hypersensitivity, also known as type IV allergy reaction is an allergic reaction characterized by a delay period of at least 12 hours from invasion of the antigen into the allergic subject until appearance of the inflammatory or immune reaction. The T lymphocytes (sensitized T lymphocytes) of individuals in an allergic condition react with the antigen, triggering the T lymphocytes to release lymphokines (macrophage migration inhibitory factor (MIF), macrophage activating factor (MAF), mitogenic factor (MF), skin-reactive factor (SRF), chemotactic factor, neovascularization-accelerating factor, etc.), which function as inflammation mediators, and the biological activity of these lymphokines, together with the direct and indirect effects of locally appearing lymphocytes and other inflammatory immune cells, give rise to the type IV allergy reaction. Delayed allergy reactions include tuberculin type reaction, homograft rejection reaction, cell-dependent type protective reaction, contact dermatitis hypersensitivity reaction, and the like, which are known to be most strongly suppressed by steroidal agents. Consequently, steroidal agents are effective against diseases which are caused by delayed allergy reactions. Long-term use of steroidal agents at concentrations currently being used can, however, lead to the serious side-effect known as steroid dependence. The methods of the disclosure solve some of these problems, by providing for lower and fewer doses to be administered.

Immediate hypersensitivity (or anaphylactic response) is a form of allergic reaction which develops very quickly, i.e. within seconds or minutes of exposure of the patient to the causative allergen, and it is mediated by IgE antibodies made by B lymphocytes. In nonallergic patients, there is no IgE antibody of clinical relevance; but, in a person suffering with allergic diseases, IgE antibody mediates immediate hypersensitivity by sensitizing mast cells which are abundant in the skin, lymphoid organs, in the membranes of the eye, nose and mouth, and in the respiratory tract and intestines.

Mast cells have surface receptors for IgE, and the IgE antibodies in allergy-suffering patients become bound to them. As discussed briefly above, when the bound IgE is subsequently contacted by the appropriate allergen, the mast cell is caused to degranulate and to release various substances called bioactive mediators, such as histamine, into the surrounding tissue. It is the biologic activity of these substances which is responsible for the clinical symptoms typical of immediate hypersensitivity; namely, contraction of smooth muscle in the airways or the intestine, the dilation of small blood vessels and the increase in their permeability to water and plasma proteins, the secretion of thick sticky mucus, and in the skin, redness, swelling and the stimulation of nerve endings that results in itching or pain.

The list of allergens is enormous and can include pollens, insect venoms, animal dander, dust, fungal spores and drugs (e.g., penicillin). Examples of natural, animal and plant allergens include but are not limited to proteins specific to the following genuses: *Canine (Canis familiaris); Dermatophagoides* (e.g., *Dermatophagoides farinae*); *Felis* (*Felis domesticus*); *Ambrosia* (*Ambrosia artemiisfolia*; *Lolium* (e.g., *Lolium perenne* or *Lolium multiflorum*); *Cryptomeria* (*Cryptomeria japonica*); *Alternaria* (*Alternaria alternata*); *Alder*; *Alnus* (*Alnus gultinoasa*); *Betula* (*Betula verrucosa*); *Quercus* (*Quercus alba*); *Olea* (*Olea europa*); *Artemisia* (*Artemisia vulgaris*); *Plantago* (e.g., *Plantago lanceolata*); *Parietaria* (e.g., *Parietaria officinalis* or *Parietaria judaica*); *Blattella* (e.g., *Blattella germanica*); *Apis* (e.g., *Apis multiflorum*); *Cupressus* (e.g., *Cupressus sempervirens, Cupressus arizonica* and *Cupressus macrocarpa*); *Juniperus* (e.g., *Juniperus sabinoides, Juniperus virginiana, Juniperus communis* and *Juniperus ashei*); *Thuya* (e.g., *Thuya orientalis*); *Chamaecyparis* (e.g., *Chamaecyparis obtusa*); *Periplaneta* (e.g., *Periplaneta americana*); *Agropyron* (e.g., *Agropyron repens*); *Secale* (e.g., *Secale cereale*); *Triticum* (e.g., *Triticum aestivum*); *Dactylis* (e.g., *Dactylis glomerata*); *Festuca* (e.g., *Festuca elatior*); *Poa* (e.g., *Poa pratensis* or *Poa compressa*); *Avena* (e.g., *Avena sativa*); *Holcus* (e.g., *Holcus lanatus*); *Anthoxanthum* (e.g., *Anthoxanthum odoratum*); *Arrhenatherum* (e.g., *Arrhenatherum elatius*); *Agrostis* (e.g., *Agrostis alba*); *Phleum* (e.g., *Phleum pratense*); *Phalaris* (e.g., *Phalaris arundinacea*); *Paspalum* (e.g., *Paspalum notatum*); *Sorghum* (e.g., *Sorghum halepensis*); and *Bromus* (e.g., *Bromus inermis*).

The allergen may be substantially purified. The term substantially purified as used herein refers to an antigen, i.e., a polypeptide which is substantially free of other proteins, lipids, carbohydrates or other materials with which it is naturally associated. One skilled in the art can purify polypeptide antigens using standard techniques for protein purification. The substantially pure polypeptide will often yield a single major band on a non-reducing polyacrylamide gel. In the case of partially glycosylated polypeptides or those that have several start codons, there may be several bands on a non-reducing polyacrylamide gel, but these will form a distinctive pattern for that polypeptide. The purity of the polypeptide antigen may also be determined by amino-terminal amino acid sequence analysis. Other types of antigens such as polysaccharides, small molecule, mimics, etc., are included within the invention and may optionally be substantially pure.

CpG immunostimulatory oligonucleotides can be combined with an additional immune modulator such as an adjuvant to modulate an immune response. The CpG immunostimulatory oligonucleotide and other therapeutic agent may be administered simultaneously or sequentially. When the other therapeutic agents are administered simultaneously they can be administered in the same or separate formulations, but are administered at the same time. The other therapeutic agents are administered sequentially with one another and with CpG immunostimulatory oligonucleotide, when the administration of the other therapeutic agents and the CpG immunostimulatory oligonucleotide is temporally separated. More specifically, the CpG immunostimulatory oligonucleotide can be administered before or after administration of (or exposure to) at least one other therapeutic agent. The separation in time between the administration of these compounds may be a matter of minutes or it may be longer. Other therapeutic agents include but are not limited to adjuvants, cytokines, antibodies, antigens, etc.

The compositions of the invention may also be administered with non-nucleic acid adjuvants. A non-nucleic acid adjuvant is any molecule or compound except for the CpG immunostimulatory oligonucleotides described herein which can stimulate the humoral and/or cellular immune response. Non-nucleic acid adjuvants include, for instance, adjuvants that create a depot effect, immune stimulating adjuvants, and adjuvants that create a depot effect and stimulate the immune system.

The CpG immunostimulatory oligonucleotides are also useful as mucosal adjuvants. It has previously been discovered that both systemic and mucosal immunity are induced by mucosal delivery of CpG nucleic acids. Thus, the oligonucleotides may be administered in combination with other mucosal adjuvants.

Immune responses can also be induced or augmented by the co-administration or colinear expression of cytokines (Bueler & Mulligan, 1996; Chow et al., 1997; Geissler et al., 1997; Iwasaki et al., 1997; Kim et al., 1997) or co-stimulatory molecules such as B7 (Iwasaki et al., 1997; Tsuji et al., 1997) with the CpG immunostimulatory oligonucleotides. The term cytokine is used as a generic name for a diverse group of soluble proteins and peptides which act as humoral regulators at nano- to picomolar concentrations and which, either under normal or pathological conditions, modulate the functional activities of individual cells and tissues. These proteins also mediate interactions between cells directly and regulate processes taking place in the extracellular environment. Examples of cytokines include, but are not limited to interleukin-1 (IL-1), IL-2, IL-3, IL-4, IL-5, IL-6, IL-7, IL-10, IL-12, IL-15, IL-18, granulocyte-macrophage colony stimulating factor (GM-CSF), granulocyte colony stimulating factor (G-CSF), IFN-γ, IFN-α, IFN-β, tumor necrosis factor (TNF), TGF-β, Flt-3 ligand, and CD40 ligand. In addition to cytokines the CpG oligonucleotides may be used in combination with antibodies against certain cytokines, such as anti-IL-10 and anti-TGF-β, as well as cyclooxygenase inhibitors, i.e., COX-1 and COX-2 inhibitors.

The CpG immunostimulatory oligonucleotides are also useful for treating and preventing inflammatory disorders. As used herein, the term "inflammatory disorder" refers to a condition associated with an antigen-nonspecific reaction of the innate immune system that involves accumulation and activation of leukocytes and plasma proteins at a site of infection, toxin exposure, or cell injury. Cytokines that are characteristic of inflammation include tumor necrosis factor (TNF-α), interleukin 1 (IL-1), IL-6, IL-12, interferon alpha (IFN-α), interferon beta (IFN-β), and chemokines. Inflammatory disorders include, for example asthma, allergy, allergic rhinitis cardiovascular disease, chronic obstructive pulmonary disease (COPD), bronchiectasis, chronic cholecystitis, tuberculosis, Hashimoto's thyroiditis, sepsis, arcoidosis, silicosis and other pneumoconioses, and an implanted foreign body in a wound, but are not so limited.

The CpG immunostimulatory oligonucleotides are also useful for treating and preventing autoimmune diseases. Autoimmune disease is a class of diseases in which a subject's own antibodies react with host tissue or in which immune effector T cells are autoreactive to endogenous self peptides and cause destruction of tissue. Thus an immune response is mounted against a subject's own antigens, referred to as self antigens. Autoimmune diseases include but are not limited to rheumatoid arthritis, psoriasis, inflammatory bowel diseases, ulcerative colitis, Crohn's disease, multiple sclerosis, systemic lupus erythematosus (SLE), autoimmune encephalomyelitis, myasthenia gravis (MG), Hashimoto's thyroiditis, Goodpasture's syndrome, pemphigus (e.g., pemphigus vulgaris), Grave's disease, autoimmune hemolytic anemia, autoimmune thrombocytopenic purpura, scleroderma with anti-collagen antibodies, mixed connective tissue disease, polymyositis, pernicious anemia, idiopathic Addison's disease, autoimmune-associated infertility, glomerulonephritis (e.g., crescentic glomerulonephritis, proliferative glomerulonephritis), bullous pemphigoid, Sjögren's syndrome, insulin resistance, and autoimmune diabetes mellitus.

A "self-antigen" as used herein refers to an antigen of a normal host tissue. Normal host tissue does not include cancer cells. Thus an immune response mounted against a self-antigen, in the context of an autoimmune disease, is an undesirable immune response and contributes to destruction and damage of normal tissue, whereas an immune response mounted against a cancer antigen is a desirable immune response and contributes to the destruction of the tumor or cancer. Thus, in some aspects of the invention aimed at treating autoimmune disorders it is not recommended that the CpG immunostimulatory oligonucleotides be administered with self antigens, particularly those that are the targets of the autoimmune disorder.

In other instances, the CpG immunostimulatory oligonucleotides may be delivered with low doses of self-antigens. A number of animal studies have demonstrated that mucosal administration of low doses of antigen can result in a state of immune hyporesponsiveness or "tolerance." The active mechanism appears to be a cytokine-mediated immune deviation away from a Th1 towards a predominantly Th2 and Th3 (i.e., TGF-β dominated) response. The active suppression with low dose antigen delivery can also suppress an unrelated immune response (bystander suppression) which is of considerable interest in the therapy of autoimmune diseases, for example, rheumatoid arthritis and SLE. Bystander suppression involves the secretion of Th1-counter-regulatory, suppressor cytokines in the local environment where proinflammatory and Th1 cytokines are released in either an antigen-specific or antigen-nonspecific manner. "Tolerance" as used herein is used to refer to this phenomenon. Indeed, oral tolerance has been effective in the treatment of a number of autoimmune diseases in animals including: experimental autoimmune encephalomyelitis (EAE), experimental autoimmune myasthenia gravis, collagen-induced arthritis (CIA), and insulin-dependent diabetes mellitus. In these models, the prevention and suppression of autoimmune disease is associated with a shift in antigen-specific humoral and cellular responses from a Th1 to Th2/Th3 response.

The disclosure also includes methods for inducing antigen non-specific innate immune activation and broad spectrum resistance to infectious challenge using the CpG immunostimulatory oligonucleotides. The term innate immune activation as used herein refers to the activation of immune cells other than memory B cells and for instance can include the activation of monocytes, neutrophils, macrophages, dendritic cells, NK cells, and/or other immune cells that can respond in an antigen-independent fashion. A broad spectrum resistance to infectious challenge is induced because the immune cells are in active form and are primed to respond to any invading compound or microorganism. The cells do not have to be specifically primed against a particular antigen. This is particularly useful in biowarfare, and the other circumstances described above such as travelers.

In other embodiments the oligonucleotide is delivered to the subject in an effective amount to induce cytokine or chemokine expression. Optionally the cytokine or chemokine is selected from the group consisting of IL-6, TNFα, IFN-α, IFN-γ and IP-10. In other embodiments the oligonucleotide is delivered to the subject in an effective amount to shift the immune response to a Th1 biased response form a Th2 biased response.

The immunostimulatory oligonucleotides of the instant invention can be used to treat or prevent infectious disease. An "infectious disease" as used herein, refers to a disorder arising from the invasion of a host, superficially, locally, or systemically, by an infectious organism. Infectious organisms include bacteria, viruses, fungi, and parasites. Accordingly, "infectious disease" includes bacterial infections, viral infections, fungal infections and parasitic infections.

Bacteria are unicellular organisms which multiply asexually by binary fission. They are classified and named based on their morphology, staining reactions, nutrition and metabolic requirements, antigenic structure, chemical composition, and genetic homology. Bacteria can be classified into three groups based on their morphological forms, spherical (coccus), straight-rod (bacillus) and curved or spiral rod (vibrio, campylobacter, spirillum, and spirochaete). Bacteria are also more commonly characterized based on their staining reactions into two classes of organisms, gram-positive and gram-negative. Gram refers to the method of staining which is commonly performed in microbiology labs. Gram-positive organisms retain the stain following the staining procedure and appear a deep violet color. Gram-negative organisms do not retain the stain but take up the counter-stain and thus appear pink. U.S. Non-Provisional Patent Application No. 09/801,839, filed March 8, 2001, lists a number of bacteria, the infections of which the present invention intends to prevent and treat.

Gram positive bacteria include, but are not limited to, *Pasteurella* species, *Staphylococci* species, and *Streptococcus* species. Gram negative bacteria include, but are not limited to, *Escherichia coli, Pseudomonas* species, and *Salmonella* species. Specific examples of infectious bacteria include but are not limited to, *Helicobacter pyloris, Borrelia burgdorferi, Legionella pneumophilia, Mycobacteria* sps (e.g., *M. tuberculosis, M. avium, M. intracellulare, M. kansasii, M. gordonae*)*, Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae,* pathogenic *Campylobacter sp., Enterococcus sp., Haemophilus influenzae, Bacillus anthracis, Corynebacterium diphtheriae, Corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema pallidum, Treponema pertenue, Leptospira, Rickettsia,* and *Actinomyces israelii.*

Examples of fungi include *Cryptococcus neoformans, Histoplasma capsulatum, Coccidioides immitis, Blastomyces dermatitidis, Chlamydia trachomatis, Candida albicans.*

Other infectious organisms (i.e., protists) include *Plasmodium spp.* such as *Plasmodium falciparum, Plasmodium malariae, Plasmodium ovale,* and *Plasmodium vivax* and *Toxoplasma gondii.* Blood-borne and/or tissues parasites include *Plasmodium* spp., *Babesia microti, Babesia divergens, Leishmania tropica, Leishmania* spp., *Leishmania braziliensis, Leishmania donovani, Trypanosoma gambiense* and *Trypanosoma rhodesiense* (African sleeping sickness), *Trypanosoma cruzi* (Chagas' disease), and *Toxoplasma gondii.* Parasites are organisms which depend upon other organisms in order to survive and thus must enter, or infect, another organism to continue their life cycle. The infected organism, i.e., the host, provides both nutrition and habitat to the parasite. Although in its broadest sense the term parasite can include all infectious agents (i.e., bacteria, viruses, fungi, protozoa and helminths), generally speaking, the term is used to refer solely to protozoa, helminths, and ectoparasitic arthropods (e.g., ticks, mites, etc.). Protozoa are single celled organisms which can replicate both intracellularly and extracellularly, particularly in the blood, intestinal tract or the extracellular matrix of tissues. Helminths are multicellular organisms which almost always are extracellular (the exception being *Trichinella* spp.). Helminths normally require exit from a primary host and transmission into a secondary host in order to replicate. In contrast to these aforementioned classes, ectoparasitic arthropods form a parasitic relationship with the external surface of the host body.

Other medically relevant microorganisms have been described extensively in the literature, e.g., see C.G.A Thomas, Medical Microbiology, Bailliere Tindall, Great Britain 1983.

The oligonucleotides of the invention may be administered to a subject with an anti-microbial agent. An anti-microbial agent, as used herein, refers to a naturally-occurring or synthetic compound which is capable of killing or inhibiting infectious microorganisms. The type of anti-microbial agent useful according to the invention will depend upon the type of microorganism with which the subject is infected or at risk of becoming infected. Anti-microbial agents include but are not limited to anti-bacterial agents, anti-viral agents, anti-fungal agents and anti-parasitic agents. Phrases such as "anti-infective agent", "anti-bacterial agent", "anti-viral agent", "anti-fungal agent", "anti-parasitic agent" and "parasiticide" have well-established meanings to those of ordinary skill in the art and are defined in standard medical texts. Briefly, anti-bacterial agents kill or inhibit bacteria, and include antibiotics as well as other synthetic or natural compounds having similar functions. Antibiotics are low molecular weight molecules which are produced as secondary metabolites by cells, such as microorganisms. In general, antibiotics interfere with one or more bacterial functions or structures which are specific for the microorganism and which are not present in host cells. Anti-viral agents can be isolated from natural sources or synthesized and are useful for killing or inhibiting viruses. Anti-fungal agents are used to treat superficial fungal infections as well as opportunistic and primary systemic fungal infections. Anti-parasitic agents kill or inhibit parasites.

Examples of anti-parasitic agents, also referred to as parasiticides useful for human administration include but are not limited to albendazole, amphotericin B, benznidazole, bithionol, chloroquine HCl, chloroquine phosphate, clindamycin, dehydroemetine, diethylcarbamazine, diloxanide furoate, eflornithine, furazolidaone, glucocorticoids, halofantrine, iodoquinol, ivermectin, mebendazole, mefloquine, meglumine antimoniate, melarsoprol, metrifonate, metronidazole, niclosamide, nifurtimox, oxamniquine, paromomycin, pentamidine isethionate, piperazine, praziquantel, primaquine phosphate, proguanil, pyrantel pamoate, pyrimethanmine-sulfonamides, pyrimethanmine-sulfadoxine, quinacrine HCl, quinine sulfate, quinidine gluconate, spiramycin, stibogluconate sodium (sodium antimony gluconate), suramin, tetracycline, doxycycline, thiabendazole, tinidazole, trimethroprim-sulfamethoxazole, and tryparsamide some of which are used alone or in combination with others.

Antibacterial agents kill or inhibit the growth or function of bacteria. A large class of antibacterial agents is antibiotics. Antibiotics, which are effective for killing or inhibiting a wide range of bacteria, are referred to as broad spectrum antibiotics. Other types of antibiotics are predominantly effective against the bacteria of the class gram-positive or gram-negative. These types of antibiotics are referred to as narrow spectrum antibiotics. Other antibiotics which are effective against a single organism or disease and not against other types of bacteria are referred to as limited spectrum antibiotics. Antibacterial agents are sometimes classified based on their primary mode of action. In general, antibacterial agents are cell wall synthesis inhibitors, cell membrane inhibitors, protein synthesis inhibitors, nucleic acid synthesis or functional inhibitors, and competitive inhibitors.

Anti-fungal agents are useful for the treatment and prevention of infective fungi. Anti-fungal agents are sometimes classified by their mechanism of action. Some anti-fungal agents function as cell wall inhibitors by inhibiting glucose synthase. These include, but are not limited to, basiungin/ECB. Other anti-fungal agents function by destabilizing membrane integrity. These include, but are not limited to, imidazoles, such as clotrimazole, sertaconzole, fluconazole, itraconazole, ketoconazole, miconazole, and voriconacole, as well as FK 463, amphotericin B, BAY 38-9502, MK 991, pradimicin, UK 292, butenafine, and terbinafine. Other anti-fungal agents function by breaking down chitin (e.g., chitinase) or immunosuppression (501 cream).

In some embodiments the anti-microbial treatment can include a microbial antigen. A microbial antigen is an antigen of a microorganism and includes but is not limited to viruses, bacteria, parasites, and fungi. Such antigens include the intact microorganism as well as natural isolates and fragments or derivatives thereof and also synthetic compounds which are identical to or similar to natural microorganism antigens and induce an immune response specific for that microorganism. A compound is similar to a natural microorganism antigen if it induces an immune response (humoral and/or cellular) to a natural microorganism antigen. Such antigens are used routinely in the art and are well known to those of ordinary skill in the art.

The immunostimulatory oligonucleotides of the instant invention can be used to treat or prevent viral infection. Viruses are small infectious agents which generally contain a nucleic acid core and a protein coat, but are not independently living organisms. Viruses can also take the form of infectious nucleic acids lacking a protein. A virus cannot survive in the absence of a living cell within which it can replicate. Viruses enter specific living cells either by endocytosis or direct injection of DNA (phage) and multiply, causing disease. The multiplied virus can then be released and infect additional cells. Some viruses are DNA-containing viruses and others are RNA-containing viruses.

Examples of viruses that have been found in humans include but are not limited to: *Retroviridae* (e.g., human immunodeficiency viruses, such as HIV-1 (also referred to as HTLV-III, LAV or HTLV-III/LAV, or HIV-III; and other isolates, such as HIV-LP; *Picornaviridae* (e.g., polio viruses, hepatitis A virus; enteroviruses, human Coxsackie viruses, rhinoviruses, echoviruses); *Calciviridae* (e.g., strains that cause gastroenteritis); *Togaviridae* (e.g., equine encephalitis viruses, rubella viruses); *Flaviviridae* (e.g., dengue viruses, encephalitis viruses, yellow fever viruses); *Coronaviridae* (e.g., coronaviruses); *Rhabdoviridae* (e.g., vesicular stomatitis viruses, rabies viruses); *Filoviridae* (e.g., ebola viruses); *Paramyxoviridae* (e.g., parainfluenza viruses, mumps virus, measles virus, respiratory syncytial virus); *Orthomyxoviridae* (e.g., influenza viruses); *Bunyaviridae* (e.g., Hantaan viruses, bunya viruses, phleboviruses and Nairo viruses); Arena viridae (hemorrhagic fever viruses); *Reoviridae* (e.g., reoviruses, orbiviurses and rotaviruses); *Bornaviridae*; *Hepadnaviridae* (Hepatitis B virus); *Parvoviridae* (parvoviruses); *Papovaviridae* (papilloma viruses, polyoma viruses); *Adenoviridae* (most adenoviruses); *Herpesviridae* (herpes simplex virus (HSV) 1 and 2, varicella zoster virus, cytomegalovirus (CMV), herpes virus; *Poxviridae* (variola viruses, vaccinia viruses, pox viruses); and *Iridoviridae* (e.g., African swine fever virus); and unclassified viruses (e.g., the agent of delta hepatitis (thought to be a defective satellite of hepatitis B virus), Hepatitis C; Norwalk and related viruses, and astroviruses).

The immunostimulatory oligonucleotides of the instant invention can be administered concurrently with a traditional antiviral treatment. In some embodiments the anti-viral agent and the CpG oligonucleotide of the invention are linked. Antiviral agents are compounds which prevent infection of cells by viruses or replication of the virus within the cell. There are many fewer antiviral drugs than antibacterial drugs because the process of viral replication is so closely related to DNA replication within the host cell, that non-specific antiviral agents would often be toxic to the host. There are several stages within the process of viral infection which can be blocked or inhibited by antiviral agents. These stages include, attachment of the virus to the host cell (immunoglobulin or binding peptides), uncoating of the virus (e.g., amantadine), synthesis or translation of viral mRNA (e.g., interferon), replication of viral RNA or DNA (e.g., nucleoside analogs), maturation of new virus proteins (e.g., protease inhibitors), and budding and release of the virus.

Nucleotide analogs are synthetic compounds which are similar to nucleotides, but which have an incomplete or abnormal deoxyribose or ribose group. Once the nucleotide analogs are in the cell, they are phosphorylated, producing the triphosphate form which competes with normal nucleotides for incorporation into the viral DNA or RNA. Once the triphosphate form of the nucleotide analog is incorporated into the growing nucleic acid chain, it causes irreversible association with the viral polymerase and thus chain termination. Nucleotide analogs include, but are not limited to, acyclovir (used for the treatment of herpes simplex virus and varicella-zoster virus), gancyclovir (useful for the treatment of cytomegalovirus), idoxuridine, ribavirin (useful for the treatment of respiratory syncitial virus), dideoxyinosine, dideoxycytidine, zidovudine (azidothymidine), imiquimod, and resiquimod.

The interferons are cytokines which are secreted by virus-infected cells as well as immune cells. The interferons function by binding to specific receptors on cells adjacent to the infected cells, causing the change in the cell which protects it from infection by the virus. α and β-interferon also induce the expression of Class I and Class II MHC molecules on the surface of infected cells, resulting in increased antigen presentation for host immune cell recognition. α and β-interferons are available as recombinant forms and have been used for the treatment of chronic hepatitis B and C infection. At the dosages which are effective for anti-viral therapy, interferons have severe side effects such as fever, malaise and weight loss.

Other anti-viral agents useful in the invention include but are not limited to immunoglobulins, amantadine, interferons, nucleoside analogs, and protease inhibitors. Specific examples of anti-virals include but are not limited to Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscarnet Sodium; Fosfonet Sodium; Ganciclovir; Ganciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; and Zinviroxime.

The CpG immunostimulatory oligonucleotides may be directly administered to the subject or may be administered in conjunction with a nucleic acid delivery complex. A nucleic acid delivery complex shall mean a nucleic acid molecule associated with (e.g., ionically or covalently bound to; or encapsulated within) a targeting means (e.g., a molecule that results in higher affinity binding to target cell). Examples of nucleic acid delivery complexes include nucleic acids associated with a sterol (e.g., cholesterol), a lipid (e.g., a cationic lipid, virosome or liposome), or a target cell specific binding agent (e.g., a ligand recognized by target cell specific receptor). Preferred complexes may be sufficiently stable *in vivo* to prevent significant uncoupling prior to internalization by the target cell. However, the complex can be cleavable under appropriate conditions within the cell so that the oligonucleotide is released in a functional form.

The CpG immunostimulatory oligonucleotide and/or the antigen and/or other therapeutics may be administered alone (e.g., in saline or buffer) or using any delivery vehicles known in the art. For instance the following delivery vehicles have been described: Cochleates; Emulsomes; ISCOMs; Liposomes; Live bacterial vectors (e.g., *Salmonella, Escherichia coli,* bacillus Calmette-Guérin, *Shigella, Lactobacillus*); Live viral vectors (e.g., Vaccinia, adenovirus, Herpes Simplex); Microspheres; Nucleic acid vaccines; Polymers (e.g., carboxymethylcellulose, chitosan); Polymer rings; Sodium Fluoride; Transgenic plants; Virosomes; Virus-like particles. Other delivery vehicles are known in the art.

The term "effective amount" refers generally to the amount necessary or sufficient to realize a desired biologic effect. For example, an effective amount of a CpG immunostimulatory oligonucleotide administered with an antigen for inducing mucosal immunity is that amount necessary to cause the development of IgA in response to an antigen upon exposure to the antigen, whereas that amount required for inducing systemic immunity is that amount necessary to cause the development of IgG in response to an antigen upon exposure to the antigen. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is entirely effective to treat the particular subject. The effective amount for any particular application can vary depending on such factors as the disease or condition being treated, the particular CpG immunostimulatory oligonucleotide being administered the size of the subject, or the severity of the disease or condition. One of ordinary skill in the art can empirically determine the effective amount of a particular CpG immunostimulatory oligonucleotide and/or antigen and/or other therapeutic agent without necessitating undue experimentation.

Subject doses of the compounds described herein for mucosal or local delivery typically range from about 10 µg to 10 g per administration, which depending on the application could be given daily, weekly, or monthly and any other amount of time therebetween or as otherwise required. More typically mucosal or local doses range from about 1 mg to 500 mg per administration, and most typically from about 1 mg to 100 mg, with 2 - 4 administrations being spaced days or weeks apart. More typically, immune stimulant doses range from 10 µg to 100 mg per administration, and most typically 100 µg to 10 mg, with daily or weekly administrations. Subject doses of the compounds described herein for parenteral delivery (ie, SC or IM) for the purpose of inducing an antigen-specific immune response, wherein the compounds are delivered with an antigen but not another therapeutic agent are typically in about the same dose range or somewhat lower than the effective mucosal dose (i.e., IN, sublingual, oral, intravaginal, rectal, etc) for vaccine adjuvant or immune stimulant applications. Doses of the compounds described herein for parenteral delivery for the purpose of inducing an innate immune response or for increasing ADCC or for inducing an antigen specific immune response when the CpG immunostimulatory oligonucleotides are administered in combination with other therapeutic agents or in specialized delivery vehicles typically range from about 100 µg to 10 g per administration, which depending on the application could be given daily, weekly, or monthly and any other amount of time therebetween or as otherwise required. More typically parenteral doses for these purposes range from about 1 mg to 5 g per administration, and most typically from about 1 mg to 1 g, with 2 - 4 administrations being spaced days or weeks apart. In some embodiments, however, parenteral doses for these purposes may be used in a range of 5 to 10,000 times higher than the typical doses described above. Depending on the application, the duration of dosing required may only be one or a few doses, or for treatment of chronic conditions, the dosing could be for many years on a daily, weekly, or monthly basis.

For any compound described herein the therapeutically effective amount can be initially determined from animal models. A therapeutically effective dose can also be determined from human data for other CpG oligonucleotides which have been tested in humans (human clinical trials are ongoing) and for compounds which are known to exhibit similar pharmacological activities, such as other adjuvants, e.g., LT and other antigens for vaccination purposes. Higher doses may be required for parenteral administration. The applied dose can be adjusted based on the relative bioavailability, body mass, and potency of the administered compound. Adjusting the dose to achieve maximal efficacy based on the methods described above and other methods as are well-known in the art is well within the capabilities of the ordinarily skilled artisan.

The formulations of the invention are administered in pharmaceutically acceptable solutions, which may routinely contain pharmaceutically acceptable concentrations of salt, buffering agents, preservatives, compatible carriers, adjuvants, and optionally other therapeutic ingredients. Some formulations may contain an alcohol, for example a saccharide (e.g. dextrose), or an amino acid.

For use in therapy, an effective amount of the CpG immunostimulatory oligonucleotide and/or other therapeutics can be administered to a subject by any mode that delivers the compound to the desired surface, e.g., local, mucosal, systemic. Administering the pharmaceutical composition of the present invention may be accomplished by any means known to the skilled artisan. Preferred routes of administration include but are not limited to oral, parenteral, intravenous, intramuscular, subcutaneous, intralesional, intratumoral, intranasal, sublingual, intratracheal, inhalation, ocular, vaginal, and rectal.

For oral administration, the compounds (i.e., CpG immunostimulatory oligonucleotides, antigens and/or other therapeutic agents) can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers for neutralizing internal acid conditions or may be administered without any carriers.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

The compounds may be administered by inhalation to pulmonary tract, especially the bronchi and more particularly into the alveoli of the deep lung, using standard inhalation devices. The compounds may be delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. An inhalation apparatus may be used to deliver the compounds to a subject. An inhalation apparatus, as used herein, is any device for administering an aerosol, such as dry powdered form of the compounds. This type of equipment is well known in the art and has been described in detail, such as that description found in Remington: The Science and Practice of Pharmacy, 19th Edition, 1995, Mac Publishing Company, Easton, Pennsylvania, pages 1676-1692. Many U.S. patents also describe inhalation devices, such as U.S. Pat. No. 6,116,237.

"Powder" as used herein refers to a composition that consists of finely dispersed solid particles. Preferably the compounds are relatively free flowing and capable of being dispersed in an inhalation device and subsequently inhaled by a subject so that the compounds reach the lungs to permit penetration into the alveoli. A "dry powder" refers to a powder composition that has a moisture content such that the particles are readily dispersible in an inhalation device to form an aerosol. The moisture content is generally below about 10% by weight (% w) water, and in some embodiments is below about 5% w and preferably less than about 3% w. The powder may be formulated with polymers or optionally may be formulated with other materials such as liposomes, albumin and/or other carriers.

Aerosol dosage and delivery systems may be selected for a particular therapeutic application by one of skill in the art, such as described, for example in Gonda, I. "Aerosols for delivery of therapeutic and diagnostic agents to the respiratory tract," in Critical Reviews in Therapeutic Drug Carrier Systems, 6:273-313 (1990), and in Moren, "Aerosol dosage forms and formulations," in Aerosols in Medicine. Principles, Diagnosis and Therapy, Moren, et al., Eds., Elsevier, Amsterdam, 1985.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, e.g., sterile pyrogen-free water, before use.

The compounds may also be formulated in rectal or vaginal compositions such as suppositories or retention enemas, e.g., containing conventional suppository bases such as cocoa butter or other glycerides.

In addition to the formulations described previously, the compounds may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may include suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R (1990) Science 249:1527-33.

The CpG immunostimulatory oligonucleotides and optionally other therapeutics and/or antigens may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions of the invention contain an effective amount of a CpG immunostimulatory oligonucleotide and optionally antigens and/or other therapeutic agents optionally included in a pharmaceutically-acceptable carrier. The term pharmaceutically-acceptable carrier means one or more compatible solid or liquid filler, diluents or encapsulating substances which are suitable for administration to a human or other vertebrate animal. The term carrier denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The present invention is further illustrated by the following Examples, which in no way should be construed as further limiting.

### EXAMPLES

### Introduction:

The new P-Class CpG oligonucleotides of this invention are a subset of the previously described C-Class CpG oligonucleotides. The improved P-Class ODN differs from those by virtue of the surprisingly high IFN-α and Th1 and Th1-like cytokine and chemokine secreting activity *in vitro* as well as *in vivo*. They are also superior to the previously described C-Classes in their potency to induce other cytokine/chemokine production and cellular activation. Agrawal et al. have described antisense oligonucleotides having a sequence which is complementary to a nucleic acid target sequence plus a 3'-stem-loop structure, latter making the ODN stable to nuclease degradation. The 3'-stem-loop structure of Agrawal et al. is not part of the recognition sequence. Our double palindrome P-Class CpG ODN are partially self complementary at the two palindromes. Although the 3' palindromes in these ODN are potentially capable of forming a 3' end hairpin structure, it is believed that the concatamer formation is the more likely active structure in cells, and that the ability of a possible 3' hairpin to enhance stability against nucleases does not make a major contribution to the unique ODN activity.

The new compounds of this invention can be used to treat diseases in which Th1-like immune stimulation or immune modulation would be of advantage. Application areas are particularly infectious diseases, cancer, asthma and allergies. Although treatment of viral diseases, such as Hepatitis B and C, Cytomegalovirus (CMV), Papilloma Virus, HIV and Herpes simplex viruses (HSV) are of particular interest, the compounds of the present invention can be used to treat most diseases caused by any pathogens, including Leishmania, Listeria and Anthrax. Besides induction of secretion of IFN-alpha and IFN-gamma, the new ODN can be used as vaccine adjuvants when a Th1 immune response is required. The compounds of the present invention can be used for prophylaxis and therapy, either as a stand alone therapy or in combination with other therapeutics or medical devices.

So far, no compositions have been described that differentially resolve duplex or aggregate formation of oligonucleotides with complementary base sequences. The chemical species described previously for prevention of aggregate formation were limited to monosaccharides and polyalcohols. No combination of more than one chemical additive of one or more than one chemical species has been described for prevention of oligonucleotide duplex or aggregate formation. The following examples illustrate novel oligonucleotides and oligonucleotide formulations.

### Materials and Methods

### Oligodeoxynucleotides (ODNs)

All ODNs were purchased from Biospring (Frankfurt, Germany) or Sigma-Ark (Darmstadt, Germany), and were controlled for identity and purity by Coley Pharmaceutical GmbH (Langenfeld, Germany). ODNs were dissolved in 0.5x Tris-EDTA (5mM Tris Base, 0.5 mM EDTA, pH 8.0) and stored at -20o C. For incubation of cells, ODNs were diluted in phosphate-buffered saline (Sigma, Germany). All dilutions were carried out using pyrogen-free reagents.

### Cell purification

Peripheral blood buffy coat preparations from healthy male and female human donors were obtained from the Blood Bank of the University of Düsseldorf (Germany) and from these, peripheral blood mononuclear cells (PBMC) were purified by centrifugation over Ficoll®-Hypaque (Sigma). The purified PBMC were either used freshly (for most assays) or were suspended in freezing medium and stored at -70°C. When required, aliquots of these cells were thawed, washed and resuspended in RPMI 1640 culture medium (BioWhittaker, Belgium) supplemented with 5% (v/v) heat inactivated human AB serum (BioWhittaker, Belgium) or 10% (v/v) heat inactivated FCS, 2mM L-glutamine (BioWhittaker), 100U/ml penicillin and 100µg/ml streptomycin (Invitrogen (Karlsruhe, Germany)).

### Cytokine induction in PBMC

Human peripheral blood mononuclear cells were incubated with CpG ODN for 48 hours at concentrations indicated. IFN-α secretion by human PBMC was measured.

### Cytokine detection

Fresh PBMC were seeded on 96 well round-bottom plates at 5x10⁶/ml, and incubated with ODN in the concentrations as indicated in a humidified incubator at 37°C. Culture supernatants were collected and, if not used immediately, frozen at -20°C until required. Amounts of cytokines in the supernatants were assessed using commercially available ELISA Kits or in-house ELISAs developed using commercially available antibodies.

### Size Exclusion-Chromatography (SEC)

Size exclusion chromatography (SEC) was performed using a Superdex® 75 10/300 Column (#17-5174-01; GE Healthcare, Munich, Germany) at a Flow Rate of 0.4 - 1 mL/min. Sample buffer and running buffer were identical. The buffer consisted of 10mM phosphate buffer (pH 7.5) supplemented with 20mM sodium chloride and the indicated percentage (w/w) of chemical additive. Chromatography was performed at ambient temperature.

All test-ODN were injected in concentrations of 1mg/mL to 30 mg/mL in the respective running buffer. Before administration to the HPLC, the samples were heated to 95°C for 5 minutes and allowed to cool to room temperature. This was performed to allow for similar incubation conditions for the samples before chromatography. Injection volume was 0.1 - 5 µl, depending on sample concentration.

A reference standard was used to determine the run times of monomers of different lengths. A dimer-forming ODN was used to determine the run time of an ODN-dimer. For each buffer condition, the run times obtained with the standard ODN were compared to the runtimes of the test-ODN and the percent values of test-ODN-monomer and test-ODN-dimer were calculated by area under the curve integrations.

No aggregates were observed at the investigated conditions for these buffers, except for dextrose which showed no aggregates below 10 mg/ml.

The SEC analyses of the fractions were carried out using a 1100 Series Binary Pump (G1312A), 1100 Series Well-Plate Sampler (G1367A), 1100 Series Micro Vacuum-Degasser (G1379A) and a 1100 Series MWD (G1365B) (all instruments manufactured by Agilent Technologies, Boeblingen, Germany) or using a Beckman HPLC system (System Gold® 126 Solvent Module, System Gold® 508 Autosampler, and System Gold® 168 Detector, manufactured by BeckmanCoulter GmbH, Krefeld, Germany).

Data Analysis was performed by integration of the resulting OD 260 signals using the instrument software.

| Chromatographic Conditions: | |
|---|---|
| Column: | Superdex 75 10/300 |
| Column Temp.: | 25°C |
| Autosampler Temp.: | 25°C |
| Mobile Phase: | 10mM Phosphate buffer pH 7.5; 20mM NaCl + additive |
| Detection Wavelength: | 260 nm |
| Flow Rate: | 0.4 - 1 mL/min |
| Injection Volume: | 0.1 - 5 µL |
| Run Time: | 45 minutes |

| Gradient: | | | |
|---|---|---|---|
| Time | Flow | %A | %B |
| Initial | 0.7-1 | 100 | 0 |
| 45 min | 0.7-1 | 100 | 0 |

### Plasma Cytokine Detection

BALB/c mice (n=5) were injected subcutaneously (SC) or intravenously (IV) with the indicated concentration of ODN or 100µl PBS. Plasma samples were collected at 3 hours post-injection and used for assay of cytokine/chemokine by ELISA.

### Vaccine Studies

Female BALB/c mice (n=10/gp) were immunized with HBsAg (1 µg) alone or with ODN (10 µg). Animals were bled at 4 weeks post-immunization and HBsAg specific total IgG titers were determined by end point ELISA.

For the comparison of different routes of injection ODN (5 mg/ml) in PBS or 5% buffered dextrose solution were heated at 95°C for 5 minutes and then cooled down to room temperature. BALB/c mice (n=5/gp) were injected IV with 500 µg of ODN, bled at 3 hours injections and plasma tested for IFN-alpha.

**Table 2: Sample Oligonucleotides**

| **Sequence** | **Oligo type** | **SEQ ID NO** |
|---|---|---|
| T*C*C*A*G*G*A*C*T*T*C*T*C*T*C*A*G*G*T*T | Non-CpG control | SEQ ID NO:12 |
| T*C*G*T*C*G*T*T*T*T*G*T*C*G*T*T*T*T*G*T*C*G*T*T | B-class | SEQ ID NO:336 |
| 5' T*C*C*T*G*G*C*G*G*G*G*A*A*G*T 3' | S-class | SEQ ID NO:337 |
| T*C*G*T*C*G*T*T*T*T*G*A*C*G*T*T*T*T*G*T*C*G*T*T: | B-class | SEQ ID NO:338 |
| *G*C*T*G*C*T*T*T*T*G*T*G*C*T*T*T*T*G*T*G*C*T*T | Non-CpG control | SEQ ID NO:339 |
| G*G*G-G-A-C-G-A-C-G-T-C-G-T-G-G*G*G*G*G*G: | A-class | SEQ ID NO:340 |
| T*C*G*T*C*G*T*T*T*T*C*G*G*C*G*G*C*C*G*C*C*G | C-class | SEQ ID NO:341 |
| T*C*G*C*G*A*C*G*T*T*C*G*G*C*G*C*G*C*G*C*C*G | C-class | SEQ ID NO:342 |
| T*C*G*T*C*G*T*T*T*T*T*C*G*G*T*T*T*T*C*G*T*T*T*T | B-class | SEQ ID NO:343 |
| 5' T*C-G-A-C-G-T-C-G-T-G-G*G*G*G 3' | A-class | SEQ ID NO:344 |
| T*C*G*T*C-G*T*T*T*T*A*C-G*G*C*G*C*C-G*T*G*C*C*G | C-class | SEQ ID NO:345 |
| T*C*G*T*C_G*T*T*T*T*A*C_G*G*C*G*C*C_G*T*G*C*C*G | C-class | SEQ ID NO:346 |
| T*C-G*C-G*A*C-G*T*T*C-G*G*C*G*C-G*C*G*C*C*G | P-Class | SEQ ID NO:133 |
| T*T*T*C_G*T*C_G*T*T*T*C_G*T*C_G*T*T | B-class | SEQ ID NO:347 |
| T*C-G*T*C-G*A*C-G*T*T*C-G*G*C*G*C-G*C*G*C*C*G | P-Class | SEQ ID NO:220 |
| T*C-G*T*C-G*T*C-G*T*T*C-G*G*C*G*C*-G*C*G*C*C*G | | SEQ ID NO:348 |
| T*C-G*A*C-G*T*T*C-G*G*C*G*C*C*G | | SEQ ID NO:230 |
| T*C-G*T*C-G*A*C-G*T*T*C-G*G*C-G*C*C*G | P-Class | SEQ ID NO:231 |
| T*C-G*T*C-G*A*C-G*A*T*C-G*G*C*G*C-G*C*G*C*C*G | P-Class | SEQ ID NO:234 |
| T*C-G*T*C-G*A*C-G*T*T*C-G*G*C*G*C*C-G*T*G*C*C*G | P-Class | SEQ ID NO:237 |
| T*C-G*T*C-G*A*C-G*T*T*C-G*A*C*T*C-G*A*G*T*C*G | P-Class | SEQ ID NO:238 |
| T*C-G*T*C-G*A*C-G*A*T*C-G*G*C*G*C*C-G*T*G*C*C*G | P-Class | SEQ ID NO:245 |
| T*C-G*T*C-G*A*C-G*A*C-G*G*C*G*C*C-G*T*G*C*C*G | P-Class | SEQ ID NO:246 |
| T*C-G*T*C-G*A*C-G*A*C-G*C*G*C*C-G*T*G*C*G | P-Class | SEQ ID NO:247 |
| T*C*G*T*C*G*A*C*G*A*T*C*G*G*C*G*C*C*G*T*G*C*C*G | P-Class | SEQ ID NO:248 |
| T*C*G*T*C-G*A*C-G*A*T*C-G*G*C*G*C*C-G*T*G*C*C*G | P-Class | SEQ ID NO:249 |
| T*C*G*T*C-G*A*C*G*A*T*C-G*G*C*G*C*C-G*T*G*C*C*G | P-Class | SEQ ID NO:250 |
| T*C*G*T*C*G*A*C*G*A-T-C*G*G*C*G*C*C*G*T*G*C*C*G | P-Class | SEQ ID NO:251 |
| T*C*G*T*C-G*A*C-G*A*T*C-G*G*C*G*C-G*C*G*C*C*G | P-Class | SEQ ID NO:252 |
| T*C*G*T*C-G*A*C*G*A*T*C-G*G*C*G*C-G*C*G*C*C*G | P-Class | SEQ ID NO:253 |
| T*C*G*T*C*G*A*C*G*A-T-C*G*G*C*G*C*G*C*G*C*C*G | P-Class | SEQ ID NO:254 |
| T*C*G*A*C-G*T*C*G*A*C*G*T-G*A*C*G*T*T | C-class | SEQ ID NO:257 |

### Example 1: Induction of IFN-α by P-Class (double-palindromic) CpG oligonucleotides in human PBMC

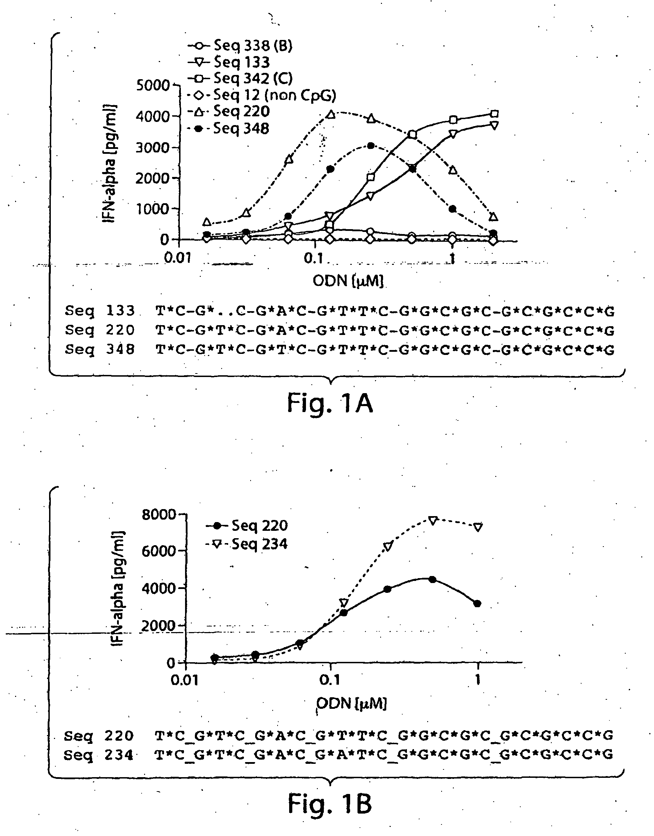

Double-palindromic ODN were observed to be more potent IFN-α inducers than C-Class ODN in the studies performed. Figure 1 shows graphs illustrating the induction of IFN-α with various ODN, including B-, C-, and P-Class as well as non-CpG controls. In the P-Class ODN in some cases the 5' bases were part of the palindromic region, as in SEQ ID NO:234; however the 5' base or bases did not have to be part of the 5' palindromic region, as in SEQ ID NO:220. The ODN retained activity when the spacer between palindromes was as short as 1 nucleotide. For example, the spacer in SEQ ID NO:234 is 'T'. D-Spacers and non-nucleotide spacers were also effective.

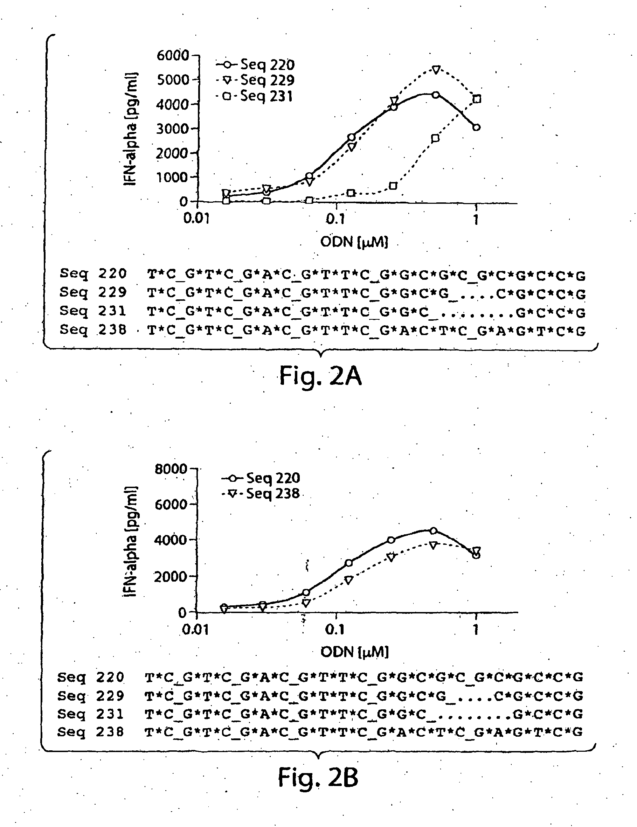

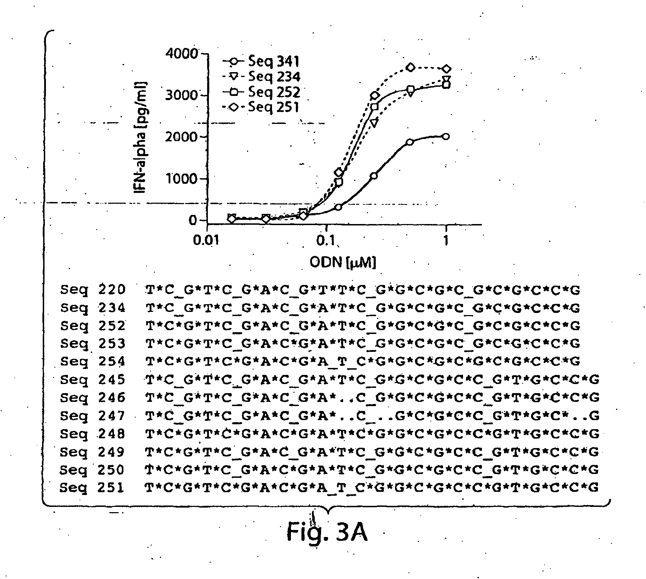

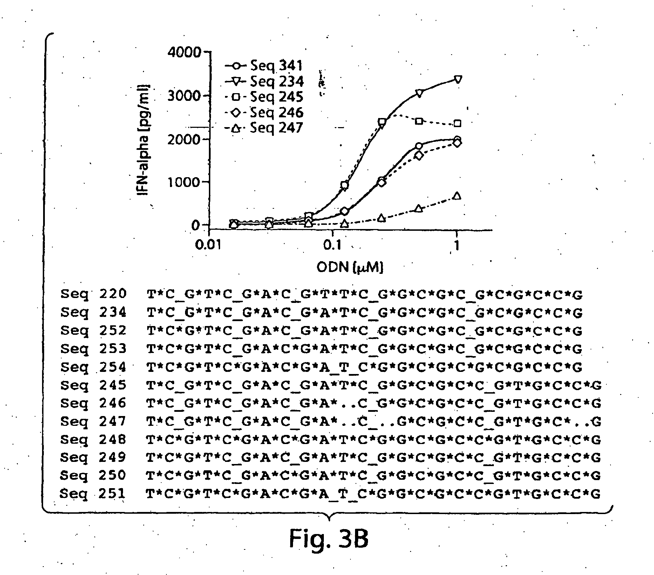

The ODN were most effective when the length of the 3' palindromic region was at least 10 nucleotides. The 3' palindrome could contain A/T base pairings and still be effective. Figure 2 shows graphs illustrating the induction of IFN-α with various P-Class ODN. In addition, ODN with an imperfect palindrome/complementarity-containing region were able to induce IFN-α provided they were long enough. In the experiments performed, such ODN were effective if the complementarity-containing region was 10 nucleotides or greater, as in SEQ ID NO:247 (Figure 3).

### Example 2: P-Class CpG ODN induce plasma cytokine and chemokine production superior to conventional C-Classes.

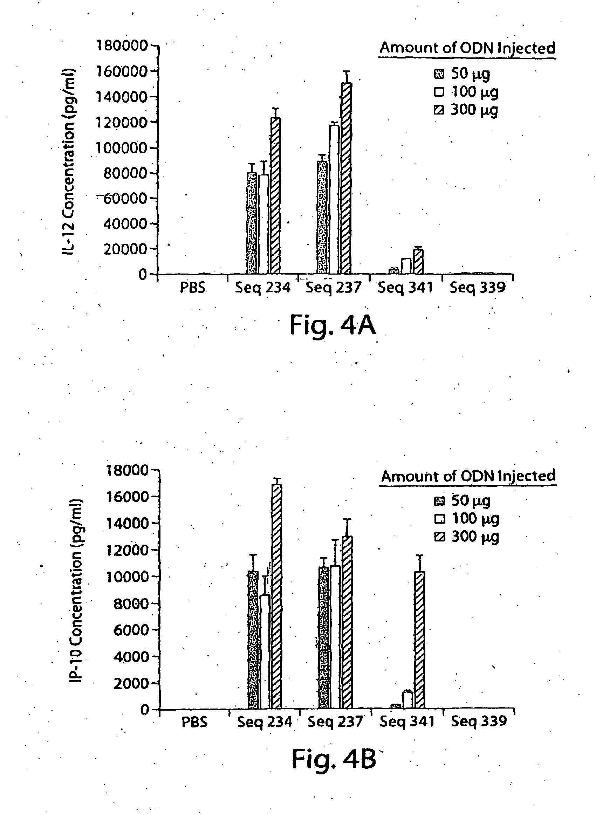

Conventional C-Class ODN such as SEQ ID NO:341 have been shown to stimulate Th1-like cytokine and chemokine responses *in vivo* in mice. Comparison of IL-12 and IP-10 production in plasma upon subcutaneous (SC) application revealed a dose-dependent IL-12 and IP-10 response for P-Class CpG ODN SEQ ID NOs:234 and 237 that was significantly higher compared to SEQ ID NO:341 (Figure 4). Non-CpG control SEQ ID NO:339 did not induce any cytokine or chemokine production.

### Example 3: P-Class CpG ODN stimulate strongest IFN-α production upon in vivo administration.

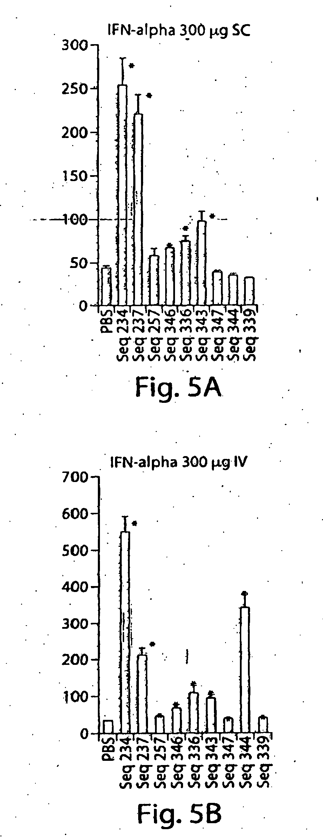

As demonstrated in Example 1, P-Class CpG ODN produced the highest IFN-α production upon stimulation of human PBMC when compared to other CpG ODN classes such as the C- and B-Classes. A similar observation was made when CpG ODN of different classes were injected in mice and IFN-α plasma levels were measured. Compared to the other classes (SEQ ID NO:344: A-Class; SEQ ID NOs:336, 347, 343: B-Class; SEQ ID NOs:346, 257: C-Class) P-Class ODN stimulated the strongest IFN-α production upon SC as well as intravenous (IV) administration (Figure 5). Non-CpG control SEQ ID NO:339 did not induce any IFN-α production.

### Example 4: P-Class CpG ODN stimulate strong adaptive immune responses.

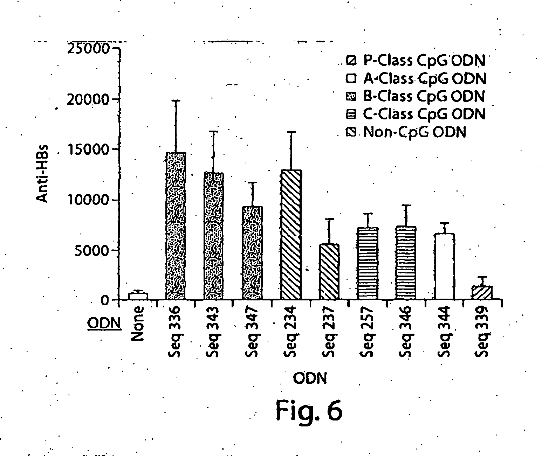

CpG ODN stimulate innate and adaptive immune responses. P-Class ODN SEQ ID NOs:234 and 237 stimulated strong antibody (Ab) responses when added to HbsAg, with P-Class SEQ ID NO:234 stimulating the strongest Ab responses when compared to C- and A-Classes (Figure 6). The Ab response was even similar to B-Class ODN that are known to stimulate strongest Ab responses *in vivo*.

### Example 5: Concatamer formation and formulation of P-Class CpG ODN

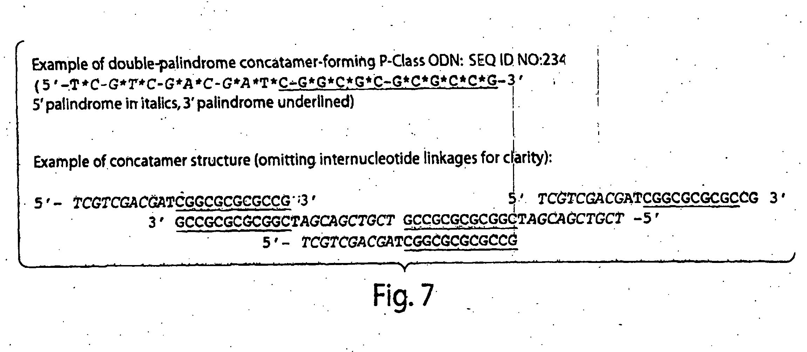

In the design of the double-palindrome P-Class CpG ODN, the length of the two palindromes and the GC content were important determinants of biological activity. Destruction of either palindrome was found to reduce the biologic activity of the ODN. Lengths between 10 and 16 bases showed the highest biological activity, although ODN between 8 and 20 or even 4 and 30 bases in length showed activity. In some instances palindromes have at least 50% GC content, more preferred at least 70%, and most preferred 100%. However, palindromes with 0-49% GC content can also be effective, especially if there are base or sugar modifications or alterations to one or more internucleotide linkages that stabilize dimer formation. Figure 7 shows an example of a double-palindrome concatamer-forming P-Class ODN (SEQ ID NO:234).

Formulation of the immunostimulatory ODN can affect the extent of concatamer formation. Formulation of the immunostimulatory ODN in 5% dextrose avoided the formation of concatamers within the final drug product, which will simplify the drug development process while still allowing the cell uptake and formation of the concatamer structures with full biologic activity within immune cells. Although formulations that avoid the formation of higher ordered structures in the vialed product have certain practical advantages, the ODN can be given in any pharmacologic formulation.

SEQ ID NO:234, containing two palindromic regions, is described to show high IFN-α inducing activity; however, Size Exclusion Chromatography (SEC) revealed the presence of large aggregates in PBS solution, preventing exact characterization of the molecule in this solution. Replacing sodium chloride or other buffer salts with a variety of chemicals can prevent the aggregation of this and similar molecules. 5% (w/v) dextrose solution in water is a solution of clinical acceptance, and served as a starting point for the analysis. To reduce salt content but largely maintain osmotic strength, 130 mM NaCl in PBS solution was substituted with 5% (w/w) of chemical species as different as sugars, alcohols and amino acids.

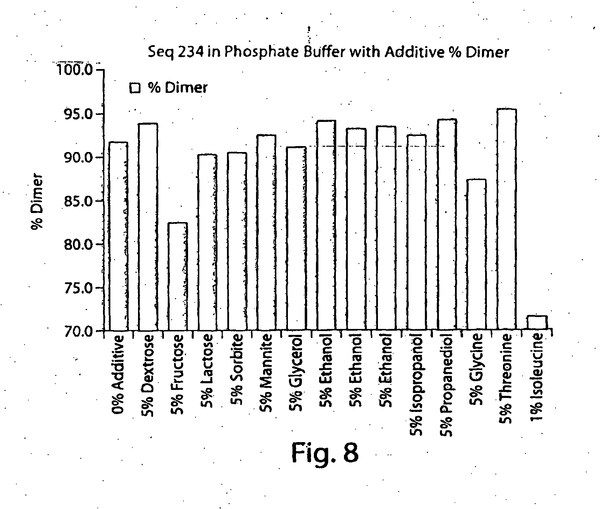

Figure 8 shows duplex formation by SEQ ID NO:234 in various 5% solutions as measured by exclusion chromatography (SEC). Surprisingly, duplex formation by some types of palindromes was prevented, whereas duplex formation by others was maintained. By performing SEC in the respective buffers, the prevention of duplex formation at the AT-rich palindrome in SEQ ID NOs:234 and 237 was observed, as well as a destabilization of the duplex at the GC-rich palindrome of NOs:234 and 341. It was concluded that for molecules which contain two or more duplex forming regions with significantly different duplex stability, formation of the duplex with lower stability could be prevented, whereas formation of the high stability duplexes was maintained in these solutions. This observation argued for duplex stability reduction as the major effect responsible for this phenomenon. In Figure 8 the bars represent % values of peak area for the dimer peak. The percentage of monomeric ODN can be calculated using the formula: 100 - (% dimer peak area). No aggregates were observed for these buffers.

Table 3 shows data demonstrating that SEQ ID NO:234 showing the prevention of the formation of the duplex with lower stability in both dextrose buffer and glycine buffer, but not in 1XPBS. Dextrose buffer: 5% Dextrose, 20mM NaCl, 10mM Phosphate-buffer pH ∼7.2. Glycine buffer: 5% Glycine, 20mM NaCl, 10mM Phosphate-buffer pH ∼7.2. ODN concentration was 5x10⁶ M (0.04 mg/ml). Wavelength (1): 260 nm. The temperature range was 20°C to 90°C *. Melting temperatures for Tm1 from Dextrose and Glycine buffers were below 25°C and could not be determined using the experimental temperature range.

**Table 3**

| | **1xPBS** | | **Dextrose-Buffer** | | **Glycine-Buffer** | |
|---|---|---|---|---|---|---|
| | Tm 1 | Tm 2 | Tm 1 | Tm 2 | Tm 1 | Tm 2 |
| **Seq. 234** | 33.9°C | 65.7°C | *) | 53.6°C | *) | 53.7°C |

The assays indicate that this effect was not only due to the reduction in sodium chloride, but at least some of the tested chemical species (i.e. saccharides (dextrose; fructose); disaccharides (lactose); monoalcohols (ethanol; isopropanol); diols (propandiol); polyalcohols (glycerol, mannitol, sorbitol) and amino acids (isoleucine; glycine; threonine) caused an additional reduction in duplex stability. Isoleucine in particular reduced formation of the low melting duplex at concentrations as low as 1% w/w. This effect could be further enhanced by combining addition of a low percentage of isoleucine with other chemical additives of the described species. Other amino acids with hydrophobic side chains should be similarly effective, such as e.g. valine, leucine, phenylalanine, praline, tyrosine and tryptophan, or amino acids with charged side chains, such as glutamate, aspartate, and lysine.

The unexpected effects described above allowed resolution of aggregate formation of duplex forming oligonucleotides, resulting in homogenous solution properties. Surprisingly, the investigated CpG ODNs retained their immune stimulatory activity when applied with the new formulations. This allows molecules which contain two or more duplex forming regions with similar duplex stability to be formulated so as to completely suppress hybridization, resulting in drug product dosing solutions in which the oligonucleotide is present in its monomeric form. In addition, G-tetrad formation of oligonucleotides was decreased by the new type of formulation. Using the formulation described herein, homogeneous dosing solutions have been observed for oligonucleotides containing G-tetrad forming sequences, such as for A-Class CpG SEQ ID NO:344 or S-Class CpG SEQ ID NO:337, which also contains one palindrome, indicating that compositions containing one or more of the above mentioned chemical species can be used to prevent aggregate formation at these base stretches.

### Example 6: P-Class ODN formulated in 5% dextrose that avoids the formation of concatamers within the final drug product stimulate strongest Th1-like cytokine and chemokine responses either upon SC and IV induction.

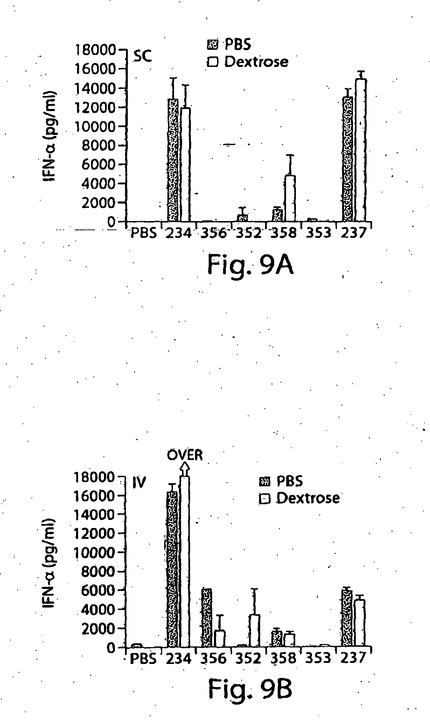

Differences exist between the stimulation of Th1-like cytokines upon *in vivo* administration via different routes. For example, A-Class ODN such as SEQ ID NOs:344 and 340 stimulated significant IFN-α in plasma only upon IV administration, whereas IFN-α levels were low or near background upon SC injection. In contrast, P-Class ODN stimulated the highest IFN-α levels upon administration by both routes, IV and SC (Figure 9). In addition, formulation of P-Class ODN SEQ ID NO:234 in 5% dextrose stimulated a stronger IFN-α response compared to formulation in PBS upon IV injection.

### Example 7. P-class ODN with a linker between the TLR activation domain and the duplex forming region can stimulate increased INF-α secretion.

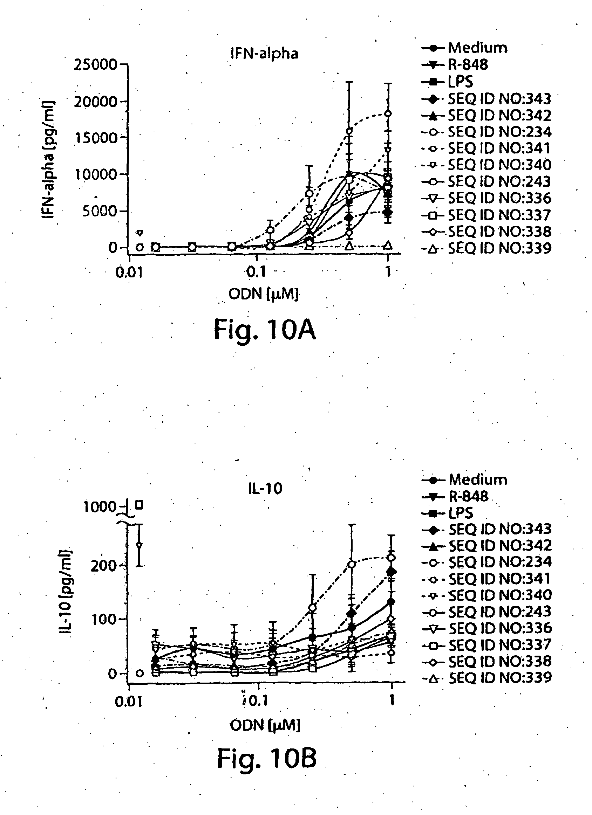

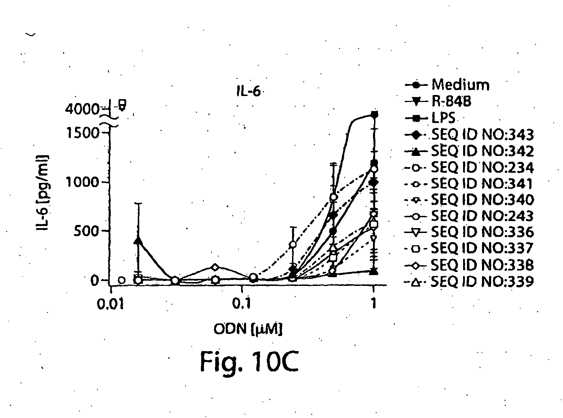

In order to determine the effect of various linkers on the immune stimulatory activity of P-class ODN, an abasic spacer (D-spacer), 1,3-propane-diol, hexaethylene glycolwere incorporated into P-class ODN SEQ ID NO:243. PBMC were incubated with the ODN at concentrations between 0.1 µM and 1 µM for 48 hours and an ELISA assay was performed on the supernatants. The inclusion of hexaethylene glycolresulted in a slight decrease in potency (Figure 10). The inclusion of 1,2-propane-diol or an abasic spacer lead to a slight decrease in potency but an increase in IFN-α production over non CpG control (SEQ ID NO:343), A class (SEQ ID NO:340 and 341) and C class (SEQ ID NO:343) as measured by an increase in IFN-α production (Figure 10a). All modifications resulted in a decreased in IL-10 (Figure 10b) and IL-6 (Figure 10c) production.

### Example 8. P-class ODN with 2'-O-methyl modification stimulate increased IFN-α secretion

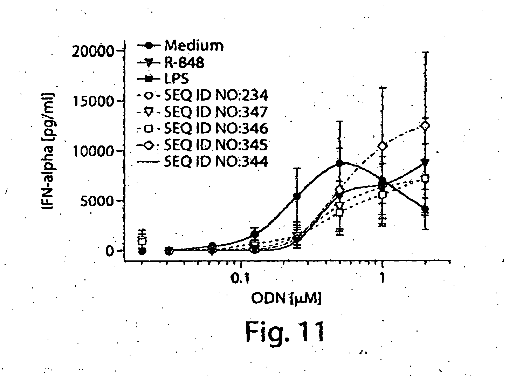

Modifications of ribose and deoxyribose in oligonucleotides can influence the activity of the oligonucleotide. In order to determine the effect of modified sugar residues on the activity of P-class ODN, O-methyl groups were added to the 2' position of the sugar moieties at various places in the sequence (see Table 1). Human PBMCs were incubated with the ODN for 48 hours at ODN concentrations ranging from 0.01 to 1 µM. The supernatants were analyzed by ELISA. As shown in Figure 11, all the modified ODN (SEQ ID NO:344-347) showed a slight decrease in potency but an elevated induction of IFN- α production compared to unmodified control of the same base sequence (SEQ ID NO:234).

## Claims

1. An immunostimulatory oligonucleotide comprising:
a 5' TLR activation domain and at least two palindromic regions, one palindromic region being a 5' palindromic region of at least 6 nucleotides in length and connected to a 3' palindromic region of at least 8 nucleotides in length through a spacer,
wherein the oligonucleotide includes at least one unmethylated CpG dinucleotide, wherein the oligonucleotide has the formula 5' XP₁SP₂T 3', wherein X is the TLR activation domain, P₁ is a palindrome, S is a spacer, P₂ is a palindrome, and
T is a 3' tail of 0-100 nucleotides in length, wherein S is a nucleic acid having a length of 1-50 nucleotides,
wherein a "palindrome" or "palindromic region" is a nucleic acid sequence which is its own perfect reverse complement.

2. The immunostimulatory oligonucleotide of claim 1, wherein X is TCG, TTCG, TTTCG, TYpR, TTYpR, TTTYpR, UCG, UUCG, UUUCG, TTT, or TTTT.

3. The immunostimulatory oligonucleotide of claim 1, wherein the two palindromic regions are connected by a spacer which is a nucleic acid having a length of 1 nucleotide.

4. An immunostimulatory oligonucleotide comprising:
a 5' TLR activation domain and at least two palindromic regions, one palindromic region being a 5' palindromic region of at least 6 nucleotides in length and connected to a 3' palindromic region of at least 8 nucleotides in length through a spacer,
wherein the oligonucleotide includes at least one unmethylated CpG dinucleotide, wherein the oligonucleotide has the formula 5' XP₁SP₂T 3', wherein X is the TLR activation domain, P₁ is a palindrome, S is a spacer, P₂ is a palindrome, and T is a 3' tail of 0-100 nucleotides in length, wherein S is a non-nucleotide spacer.

5. The immunostimulatory oligonucleotide of claim 4, wherein the non-nucleotide spacer is a D-spacer.

6. The immunostimulatory oligonucleotide of claim 4, wherein the non-nucleotide spacer is a linker selected from the group consisting of diols, cholesterol, nitroindol, triethylene glycol and hexaethylene glycol.

7. An immunostimulatory oligonucleotide comprising:
a 5' TLR activation domain and at least two complementarity-containing regions, a 5' and a 3' complementarity-containing region, each complementarity-containing region being at least 8 nucleotides in length and connected to one another through a spacer,
wherein the oligonucleotide includes at least one unmethylated CpG dinucleotide,
and wherein at least one of the complementarity-containing regions is not a perfect palindrome, and wherein the TLR activation domain is immediately 5' to the 5' complementarity-containing region,
wherein the oligonucleotide has the formula 5' XNSPT 3' or the formula 5' XPSNT 3', wherein X is the TLR activation domain, N is a non-perfect palindrome, P is a palindrome, S is a spacer, and T is a 3' tail of 0-100 nucleotides in length.

8. The immunostimulatory oligonucleotide of any one of claims 1 or 7, wherein at least one of the immunostimulatory oligonucleotides comprises a 2' modified sugar residue.

9. The immunostimulatory oligonucleotide of claim 8, wherein the 2' modified sugar residue is a 2'-O-methyl modified sugar residue.

10. A composition comprising:
a mixture of duplex forming oligonucleotides of any one of the preceding claims, formulated in a low salt buffer and including a solute.

11. An oligonucleotide of any one of claims 1-9 or a composition of claim 10 for use in the treatment of cancer.

12. Use of an oligonucleotide of any one of claims 1-9 or a composition of claim 10 in the manufacture of a medicament for use in the treatment of cancer.

13. An oligonucleotide of any one of claims 1-9 or a composition of claim 10 for use in the treatment of asthma.

14. An oligonucleotide of any one of claims 1-9 or a composition of claim 10 for use in the treatment of allergy.

15. An oligonucleotide of any one of claims 1-9 or a composition of claim 10 for use in the treatment of an infectious disease.

16. An oligonucleotide of any one of claims 1-9 or a composition of claim 10 for modulating an immune response in a subject.

17. The oligonucleotide or composition for use according to claim 16, wherein the oligonucleotide or composition is for delivery to the subject in an effective amount to induce cytokine and/or chemokine expression.

18. The oligonucleotide or composition for use according to claim 17, wherein the cytokine and/or chemokine is selected from the group consisting of IFN-α, IFN-β, IL-28, IL-29, IFN-ω, TNF-α, IL-10, IL-6, IFN-γ, IP-10, MCP-1, IL-3 and IL-12.

## Patentansprüche

1. Immunstimulatorisches Oligonucleotid, umfassend:
eine 5'-TLR-Aktivierungsdomäne und mindestens zwei Palindromregionen, wobei eine Palindromregion eine 5'-Palindromregion mit einer Länge von mindestens 6 Nucleotiden ist und mit einer 3'-Palindromregion mit einer Länge von mindestens 8 Nucleotiden durch einen Spacer verbunden ist,
wobei das Oligonucleotid mindestens ein unmethyliertes CpG-Dinucleotid umfasst,
wobei das Oligonucleotid die Formel 5' XP₁SP₂T 3' aufweist, wobei X die TLR-Aktivierungsdomäne ist, P₁ ein Palindrom ist, S ein Spacer ist, P₂ ein Palindrom ist und T ein 3'-Ende mit einer Länge von 0 - 100 Nucleotiden ist, wobei S eine Nucleinsäure mit einer Länge von 1 - 50 Nucleotiden ist,
wobei ein "Palindrom" oder eine "Palindromregion" eine Nucleinsäuresequenz ist, die ihr eigenes perfektes reverses Komplement ist.

2. Immunstimulatorisches Oligonucleotid nach Anspruch 1, wobei X TCG, TTCG, TTTCG, TYpR, TTYpR, TTTYpR, UCG, UUCG, UUUCG, TTT oder TTTT ist.

3. Immunstimulatorisches Oligonucleotid nach Anspruch 1, wobei die beiden Palindromregionen durch einen Spacer, der eine Nucleinsäure mit einer Länge von 1 Nucleotid ist, verbunden sind.

4. Immunstimulatorisches Oligonucleotid, umfassend:
eine 5'-TLR-Aktivierungsdomäne und mindestens zwei Palindromregionen, wobei eine Palindromregion eine 5'-Palindromregion mit einer Länge von mindestens 6 Nucleotiden ist und mit einer 3'-Palindromregion mit einer Länge von mindestens 8 Nucleotiden durch einen Spacer verbunden ist,
wobei das Oligonucleotid mindestens ein unmethyliertes CpG-Dinucleotid umfasst,
wobei das Oligonucleotid die Formel 5' XP₁SP₂T 3' aufweist, wobei X die TLR-Aktivierungsdomäne ist, P₁ ein Palindrom ist, S ein Spacer ist, P₂ ein Palindrom ist und T ein 3'-Ende mit einer Länge von 0 - 100 Nucleotiden ist, wobei S ein Nicht-Nucleotid-Spacer ist.

5. Immunstimulatorisches Oligonucleotid nach Anspruch 4, wobei der Nicht-Nucleotid-Spacer ein D-Spacer ist.

6. Immunstimulatorisches Oligonucleotid nach Anspruch 4, wobei der Nicht-Nucleotid-Spacer ein Linker, ausgewählt aus der Gruppe, bestehend aus Diolen, Cholesterol, Nitroindol, Triethylenglycol und Hexaethylenglycol, ist.

7. Immunstimulatorisches Oligonucleotid, umfassend:
eine 5'-TLR-Aktivierungsdomäne und mindestens zwei Komplementarität enthaltende Regionen, eine 5'- und eine 3'-Komplementarität enthaltende Region, wobei jede Komplementarität enthaltende Region eine Länge von mindestens 8 Nucleotiden hat und sie miteinander durch einen Spacer verbunden sind,
wobei das Oligonucleotid mindestens ein unmethyliertes CpG-Dinucleotid umfasst,
und wobei mindestens eine der Komplementarität enthaltenden Regionen kein perfektes Palindrom ist, und wobei die TLR-Aktivierungsdomäne unmittelbar 5' zu der 5'-Komplementarität enthaltenden Region ist,
wobei das Oligonucleotid die Formel 5' XNSPT 3' oder die Formel 5' XPSNT 3' aufweist,
wobei X die TLR-Aktivierungsdomäne ist, N ein nicht perfektes Palindrom ist, P ein Palindrom ist, S ein Spacer ist und T ein 3'-Ende mit einer Länge von 0 - 100 Nucleotiden ist.

8. Immunstimulatorisches Oligonucleotid nach einem der Ansprüche 1 oder 7, wobei mindestens eines der immunstimulatorischen Oligonucleotide einen 2'-modifizierten Zuckerrest umfasst.

9. Immunstimulatorisches Oligonucleotid nach Anspruch 8, wobei der 2'-modifizierte Zuckerrest ein 2'-O-Methyl-modifizierter Zuckerrest ist.

10. Zusammensetzung, umfassend:
ein Gemisch aus Duplex-bildenden Oligonucleotiden nach einem der vorstehenden Ansprüche, formuliert in einem salzarmen Puffer und umfassend ein Solut.

11. Oligonucleotid nach einem der Ansprüche 1 - 9 oder Zusammensetzung nach Anspruch 10 zur Verwendung bei der Behandlung von Krebs.

12. Verwendung eines Oligonucleotids nach einem der Ansprüche 1 - 9 oder einer Zusammensetzung nach Anspruch 10 bei der Herstellung eines Medikaments zur Verwendung bei der Behandlung von Krebs.

13. Oligonucleotid nach einem der Ansprüche 1 - 9 oder Zusammensetzung nach Anspruch 10 zur Verwendung bei der Behandlung von Asthma.

14. Oligonucleotid nach einem der Ansprüche 1 - 9 oder Zusammensetzung nach Anspruch 10 zur Verwendung bei der Behandlung einer Allergie.

15. Oligonucleotid nach einem der Ansprüche 1 - 9 oder Zusammensetzung nach Anspruch 10 zur Verwendung bei der Behandlung einer Infektionskrankheit.

16. Oligonucleotid nach einem der Ansprüche 1 - 9 oder Zusammensetzung nach Anspruch 10 zum Modulieren einer Immunantwort bei einem Probanden.

17. Oligonucleotid oder Zusammensetzung zur Verwendung nach Anspruch 16, wobei das Oligonucleotid oder die Zusammensetzung der Abgabe an den Probanden in einer wirksamen Menge, um die Zytokin- und/oder Chemokinexpression zu induzieren, dient.

18. Oligonucleotid oder Zusammensetzung zur Verwendung nach Anspruch 17, wobei das Zytokin und/oder Chemokin aus der Gruppe ausgewählt ist, bestehend aus IFN-α, IFN-β, IL-28, IL-29, IFN-ω, TNF-α, IL-10, IL-6, IFN-γ, IP-10, MCP-1, IL-3 und IL-12.

## Revendications

1. Oligonucléotide immunostimulateur comprenant:
un domaine d'activation de TLR5' et au moins deux régions palindromiques, une région palindromique étant une région palindromique 5' d'au moins 6 nucléotides en longueur et reliée à une région palindromique 3' d'au moins 8 nucléotides en longueur par l'intermédiaire d'un segment espaceur,
où l'oligonucléotide comprend au moins un dinucléotide CpG non méthylé, où l'oligonucléotide est de formule 5' XP₁SP₂T 3', dans laquelle X est le domaine d'activation de TLR, P₁ est un palindrome, S est un segment espaceur, P₂ est un palindrome, et T est une queue 3' de 0 à 100 nucléotides en longueur, dans laquelle S est un acide nucléique possédant une longueur de 1 à 50 nucléotides,
dans lequel un "palindrome" ou une "région palindromique" est une séquence d'acides nucléiques qui est son propre complément inverse parfait.

2. Oligonucléotide immunostimulateur selon la revendication 1, dans lequel X est TCG, TTCG, TTTCG, TYpR, TTYpR, TTTYpR, UCG, UUCG, UUUCG, TTT, ou TTTT.

3. Oligonucléotide immunostimulateur selon la revendication 1, dans lequel les deux régions palindromiques sont reliées par un segment espaceur qui est un acide nucléique possédant une longueur de 1 nucléotide.

4. Oligonucléotide immunostimulateur comprenant:
un domaine d'activation de TLR5' et au moins deux régions palindromiques, une région palindromique étant une région palindromique 5' d'au moins 6 nucléotides en longueur et reliée à une région palindromique 3' d'au moins 8 nucléotides en longueur par l'intermédiaire d'un segment espaceur,
où l'oligonucléotide comprend au moins un dinucléotide CpG non méthylé, où l'oligonucléotide est de formule 5' XP₁SP₂T 3', dans laquelle X est le domaine d'activation de TLR, P₁ est un palindrome, S est un segment espaceur, P₂ est un palindrome, et T est une queue 3' de 0 à 100 nucléotides en longueur, dans laquelle S est un segment espaceur non nucléotidique.

5. Oligonucléotide immunostimulateur selon la revendication 4, dans lequel le segment espaceur non nucléotidique est un segment espaceur D.

6. Oligonucléotide immunostimulateur selon la revendication 4, dans lequel le segment espaceur non nucléotidique est une séquence de liaison choisie dans le groupe constitué de diols, cholestérol, nitro-indol, triéthylène glycol et hexaéthylène glycol.

7. Oligonucléotide immunostimulateur comprenant:
un domaine d'activation de TLR 5' et au moins deux régions contenant une complémentarité, une région contenant une complémentarité 5' et une 3', chaque région contenant une complémentarité ayant une longueur d'au moins 8 nucléotides et étant reliée à l'autre par l'intermédiaire d'un segment espaceur,
où l'oligonucléotide comprend au moins un dinucléotide CpG non méthylé,
et dans lequel au moins l'une des régions contenant une complémentarité n'est pas un parfait palindrome, et dans lequel le domaine d'activation de TLR est immédiatement 5' par rapport à la région contenant une complémentarité 5',
où l'oligonucléotide est de formule 5' XNSPT 3' ou de formule 5' XPSNT 3', dans laquelle X est le domaine d'activation de TLR, N est un palindrome non parfait, P est un palindrome, S est un segment espaceur, et T est une queue 3' de 0 à 100 nucléotides en longueur.

8. Oligonucléotide immunostimulateur selon l'une quelconque des revendications 1 ou 7, dans lequel au moins l'un des oligonucléotides immunostimulateurs comprend un résidu sucre modifié 2'.

9. Oligonucléotide immunostimulateur selon la revendication 8, dans lequel le résidu sucre modifié 2' est un résidu sucre modifié par 2'-O-méthyle.

10. Composition comprenant:
un mélange de duplex formant des oligonucléotides selon l'une quelconque des revendications précédentes, formulé dans un tampon à faible teneur en sel et comprenant un soluté.

11. Oligonucléotide selon l'une quelconque des revendications 1 à 9 ou composition selon la revendication 10 utilisable pour le traitement du cancer.

12. Utilisation d'un oligonucléotide selon l'une quelconque des revendications 1 à 9 ou composition selon la revendication 10 dans la fabrication d'un médicament utilisable pour le traitement du cancer.

13. Oligonucléotide selon l'une quelconque des revendications 1 à 9 ou composition selon la revendication 10 pour le traitement de l'asthme.

14. Oligonucléotide selon l'une quelconque des revendications 1 à 9 ou composition selon la revendication 10 pour le traitement d'une allergie.

15. Oligonucléotide selon l'une quelconque des revendications 1 à 9 ou composition selon la revendication 10 pour le traitement d'une maladie infectieuse.

16. Oligonucléotide selon l'une quelconque des revendications 1 à 9 ou composition selon la revendication 10 pour moduler une réponse immunitaire chez un sujet.

17. Oligonucléotide ou composition utilisable selon la revendication 16, où l'oligonucléotide ou la composition est destiné à une administration chez le sujet en une quantité efficace pour induire l'expression de cytokine et/ou de chimiokine.

18. Oligonucléotide ou composition utilisable selon la revendication 17, dans lequel la cytokine et/ou la chimiokine sont choisies dans le groupe constitué de IFN-'α, IFN-β, IL-28, IL-29, IFN-ω, TNF-α, IL-10, IL-6, IFN-γ, IP-10, MCP-1, IL-3 et IL-12.


### SEQUENCE LISTING

<110> Coley Pharmaceutical Group, Inc. Coley Pharmaceutical Group, GmbH
<120> Compositions and Methods for Oligonucleotide Formulations
<130> C1041.70046WO00
<140> Not yet assigned
   <141> 2007-02-15
<150> 60/773,505 <151> 2006-02-15
<150> 60/858,240 <151> 2006-11-09
<160> 348
<170> PatentIn version 3.3
<210> 1
   <211> 27
   <212> DNA
   <213> Artificial sequence
<220>
   <223> Synthetic oligonucleotide
<400> 1
   tcgtcgacga ttttacgacg tcgtttt 27
<210> 2
   <211> 27
   <212> DNA
   <213> Artificial sequence
<220>
   <223> Synthetic oligonucleotide
<400> 2
   tcgtcgacga ttttacgacg tcgtttt 27
<210> 3
   <211> 20
   <212> DNA
   <213> Artificial sequence
<220>
   <223> Synthetic oligonucleotide
<400> 3
   tcgtcgacga acgacgtcgt 20
<210> 4
   <211> 27
   <212> DNA
   <213> Artificial sequence
<220>
   <223> Synthetic oligonucleotide
<400> 4
   tcgtcgacga tttttcgtcg acgattt 27
<210> 5
   <211> 27
   <212> DNA
   <213> Artificial sequence
<220>
   <223> Synthetic oligonucleotide
<400> 5
   tcgtcgacga tttttcgtcg acgattt 27
<210> 6
   <211> 20
   <212> DNA
   <213> Artificial sequence
<220>
   <223> Synthetic oligonucleotide
<400> 6
   tcgtcgacga tcgtcgacga 20
<210> 7
   <211> 20
   <212> DNA
   <213> Artificial sequence
<220>
   <223> Synthetic oligonucleotide
<400> 7
   cgcgcgcgcg cgcgcgcgcg 20
<210> 8
   <211> 20
   <212> DNA
   <213> Artificial sequence
<220>
   <223> Synthetic oligonucleotide
<400> 8
   gagaacgctc gaccttcgat 20
<210> 9
   <211> 19
   <212> DNA
   <213> Artificial sequence
<220>
   <223> Synthetic oligonucleotide
<400> 9
   agctccatgg tgctcactg 19
<210> 10
   <211> 18
   <212> DNA
   <213> Artificial sequence
<220>
   <223> Synthetic oligonucleotide
<400> 10
   tctcccagcg tgcgccat 18
<210> 11
   <211> 20
   <212> DNA
   <213> Artificial sequence
<220> .
   <223> Synthetic oligonucleotide
<400> 11
   tccatgacgt tcctgacgtt 20
<210> 12
   <211> 20
   <212> DNA
   <213> Artificial sequence
<220>
   <223> Synthetic oligonucleotide
<400> 12
   tccaggactt ctctcaggtt 20
<210> 13
   <211> 20
   <212> DNA
   <213> Artificial sequence
<220>
   <223> Synthetic oligonucleotide
<400> 13
   tccacgacgt tttcgacgtt 20
<210> 14
   <211> 24
   <212> DNA
   <213> Artificial sequence
<220>
   <223> Synthetic oligonucleotide
<400> 14
   tcgtcgtttt gacgttttga cgtt 24
<210> 15
   <211> 22
   <212> DNA
   <213> Artificial sequence
<220>
   <223> Synthetic oligonucleotide
<400> 15
   tcctgacgtt cggcgcgcgc cc 22
<210> 16
   <211> 27
   <212> DNA
   <213> Artificial sequence
<220>
   <223> Synthetic oligonucleotide
<400> 16
   tcgcgtgcgt tttgtcgttt tgacgtt 27
<210> 17
   <211> 22
   <212> DNA
   <213> Artificial sequence
<220>
   <223> Synthetic oligonucleotide
<400> 17
   tcgcgacgtt cggcgcgcgc cg 22
<210> 18
   <211> 20
   <212> DNA
   <213> Artificial sequence
<220>
   <223> Synthetic oligonucleotide
<400> 18
   ccggccggcc ggccggccgg 20
<210> 19
   <211> 20
   <212> DNA
   <213> Artificial sequence
<220>
   <223> Synthetic oligonucleotide
<400> 19
   cgcgcgcgcg cgcgcgcgcg 20
<210> 20
   <211> 24
   <212> DNA
   <213> Artificial sequence
<220>
   <223> Synthetic oligonucleotide
<400> 20
   tccaggactt ctctcaggtt tttt 24
<210> 21
   <211> 21
   <212> DNA
   <213> Artificial sequence
<220>
   <223> Synthetic oligonucleotide
<400> 21
   gtgctcgagg atgcgcttcg c 21
<210> 22
   <211> 21
   <212> DNA
   <213> Artificial sequence
<220>
   <223> Synthetic oligonucleotide
<400> 22
   gccgaggtcc atgtcgtacg c 21
<210> 23
   <211> 27
   <212> DNA
   <213> Artificial sequence
<220>
   <223> Synthetic oligonucleotide
<400> 23
   tcgcgtgcgt tttgtcgttt tgacgtt 27
<210> 24
   <211> 30
   <212> DNA
   <213> Artificial sequence
<220>
   <223> Synthetic oligonucleotide
<400> 24
   accgataccg gtgccggtga cggcaccacg 30
<210> 25
   <211> 30
   <212> DNA
   <213> Artificial sequence
<220>
   <223> Synthetic oligonucleotide
<400> 25
   accgataacg ttgccggtga cggcaccacg 30
<210> 26
   <211> 30
   <212> DNA
   <213> Artificial sequence
<220>
   <223> Synthetic oligonucleotide
<400> 26
   accgatgacg tcgccggtga cggcaccacg 30
<210> 27
   <211> 24
   <212> DNA
   <213> Artificial sequence
<220>
   <223> Synthetic oligonucleotide
<400> 27
   cggcgcgcgc cgcggcgcgc gccg 24
<210> 28
   <211> 23
   <212> DNA
   <213> Artificial sequence
<220>
   <223> Synthetic oligonucleotide
<400> 28
   tcgatcgttt ttcgtgcgtt ttt 23
<210> 29
   <211> 23
   <212> DNA
   <213> Artificial sequence
<220>
   <223> Synthetic oligonucleotide
<400> 29
   tcgtccagga cttctctcag gtt 23
<210> 30
   <211> 26
   <212> DNA
   <213> Artificial sequence
<220>
   <223> Synthetic oligonucleotide
<400> 30
   tcgtcgtcca ggacttctct caggtt 26
<210> 31
   <211> 22
   <212> DNA
   <213> Artificial sequence
<220>
   <223> Synthetic oligonucleotide
<400> 31
   tcgtgacggg cggcgcgcgc cc 22
<210> 32
   <211> 23
   <212> DNA
   <213> Artificial sequence
<220>
   <223> Synthetic oligonucleotide
<220>
   <221> misc_feature
   <222> (11)..(11)
   <223> n is a, c, g, or t
<400> 32
   acgacgtcgt ncggcggccg ccg 23
<210> 33
   <211> 27
   <212> DNA
   <213> Artificial sequence
<220>
   <223> Synthetic oligonucleotide
<400> 33
   ggggacgacg tcgtgcggcg gccgccg 27
<210> 34
   <211> 27
   <212> DNA
   <213> Artificial sequence
<220>
   <223> Synthetic oligonucleotide
<400> 34
   ggggacgacg tcgtgcggcg gccgccg 27
<210> 35
   <211> 29
   <212> DNA
   <213> Artificial sequence
<220>
   <223> Synthetic oligonucleotide
<400> 35
   ccacgacgtc gtcgaagacg acgtcgtgg 29
<210> 36
   <211> 24
   <212> DNA
   <213> Artificial sequence
<220>
   <223> Synthetic oligonucleotide
<400> 36
   ctgcagctgc agctgcagct gcag 24
<210> 37
   <211> 24
   <212> DNA
   <213> Artificial sequence
<220>
   <223> Synthetic oligonucleotide
<400> 37
   cggccgctgc agcggccgct gcag 24
<210> 38
   <211> 18
   <212> DNA
   <213> Artificial sequence
<220>
   <223> Synthetic oligonucleotide
<400> 38
   catgacgttt ttgatgtt 18
<210> 39
   <211> 17
   <212> DNA
   <213> Artificial sequence
<220>
   <223> Synthetic oligonucleotide
<400> 39
   atgacgtttt tgatgtt 17
<210> 40
   <211> 16
   <212> DNA
   <213> Artificial sequence
<220>
   <223> Synthetic oligonucleotide
<400> 40
   tgacgttttt gatgtt 16
<210> 41
   <211> 19
   <212> DNA
   <213> Artificial sequence
<220>
   <223> Synthetic oligonucleotide
<400> 41
   atgacgtttt tgatgttgt 19
<210> 42
   <211> 20
   <212> DNA
   <213> Artificial sequence
<220>
   <223> Synthetic oligonucleotide
<400> 42
   tccatgacgt ttttgatgtt 20
<210> 43
   <211> 20
   <212> DNA
   <213> Artificial sequence
<220>
   <223> Synthetic oligonucleotide
<400> 43
   tccatgacgt ttttgatgtt 20
<210> 44
   <211> 20
   <212> DNA
   <213> Artificial sequence
<220>
   <223> Synthetic oligonucleotide
<400> 44
   tccatgacgt ttttgatgtt 20
<210> 45
   <211> 20
   <212> DNA
   <213> Artificial sequence
<220>
   <223> Synthetic oligonucleotide
<400> 45
   tccatgacgt ttttgatgtt 20
<210> 46
   <211> 20
   <212> DNA
   <213> Artificial sequence
<220>
   <223> Synthetic oligonucleotide
<400> 46
   tccatgacgt ttttgatgtt 20
<210> 47
   <211> 19
   <212> DNA
   <213> Artificial sequence
<220>
   <223> Synthetic oligonucleotide
<400> 47
   atgacgtttt tgatgttgt 19
<210> 48
   <211> 19
   <212> DNA
   <213> Artificial sequence
<220>
   <223> Synthetic oligonucleotide
<400> 48
   atgacgtttt tgatgttgt 19
<210> 49
   <211> 19
   <212> DNA
   <213> Artificial sequence
<220>
   <223> Synthetic oligonucleotide
<400> 49
   atgacgtttt tgatgttgt 19
<210> 50
   <211> 19
   <212> DNA
   <213> Artificial sequence
<220>
   <223> Synthetic oligonucleotide
<400> 50
   atgacgtttt tgatgttgt 19
<210> 51
   <211> 20
   <212> DNA
   <213> Artificial sequence
<220>
   <223> Synthetic oligonucleotide
<400> 51
   tccatgcgtt tttgaatgtt 20
<210> 52
   <211> 20
   <212> DNA
   <213> Artificial sequence
<220>
   <223> Synthetic oligonucleotide
<400> 52
   tccatgacgt ctttgatgtc 20
<210> 53
   <211> 21
   <212> DNA
   <213> Artificial sequence
<220>
   <223> Synthetic oligonucleotide
<400> 53
   acgacgtcgt tccgacgtcg t 21
<210> 54
   <211> 27
   <212> DNA
   <213> Artificial sequence
<220>
   <223> Synthetic oligonucleotide
<220>
   <221> misc_feature
   <222> (25)..(27)
   <223> where n is D-spacer
<400> 54
   acgacgtcgt ggccacgacg tcgtnnn 27
<210> 55
   <211> 27
   <212> DNA
   <213> Artificial sequence
<220>
   <223> Synthetic oligonucleotide
<220>
   <221> misc_feature
   <222> (11)..(14)
   <223> where n is d-spacer
<220>
   <221> misc_feature
   <222> (25)..(27)
   <223> where n is d-spacer
<400> 55
   acgacgtcgt nnnnacgacg tcgtnnn 27
<210> 56
   <211> 30
   <212> DNA
   <213> Artificial sequence
<220>
   <223> Synthetic oligonucleotide
<220>
   <221> misc_feature
   <222> (1)..(3)
   <223> where n is d-spacer
<220>
   <221> misc_feature
   <222> (14)..(17)
   <223> where n is d-spacer
<220>
   <221> misc_feature
   <222> (28)..(30)
   <223> where n is d-spacer
<400> 56
   nnnacgacgt cgtnnnnacg acgtcgtnnn 30
<210> 57
   <211> 27
   <212> DNA
   <213> Artificial sequence
<220>
   <223> Synthetic oligonucleotide
<220>
   <221> misc_feature
   <222> (1)..(3)
   <223> where n is d-spacer
<220>
   <221> misc_feature
   <222> (14)..(17)
   <223> where n is d-spacer
<400> 57
   nnnacgacgt cgtnnnnacg acgtcgt 27
<210> 58
   <211> 30
   <212> DNA
   <213> Artificial sequence
<220>
   <223> Synthetic oligonucleotide
<400> 58
   gggacgacgt cgtggccacg acgtcgtccc 30
<210> 59
   <211> 16
   <212> DNA
   <213> Artificial sequence
<220>
   <223> Synthetic oligonucleotide
<400> 59
   cccacgacgt cgtggg 16
<210> 60
   <211> 18
   <212> DNA
   <213> Artificial sequence
<220>
   <223> Synthetic oligonucleotide
<400> 60
   ccccacgacg tcgtgggg 18
<210> 61
   <211> 23
   <212> DNA
   <213> Artificial sequence
<220>
   <223> Synthetic oligonucleotide
<400> 61
   tcgatcgttt ttcgtgcgtt ttt 23
<210> 62
   <211> 23
   <212> DNA
   <213> Artificial sequence
<220>
   <223> Synthetic oligonucleotide
<400> 62
   tcgatcgttt ttcgtgcgtt ttt 23
<210> 63
   <211> 23
   <212> DNA
   <213> Artificial sequence
<220>
   <223> Synthetic oligonucleotide
<400> 63
   tcgatcgttt ttcgtgcgtt ttt 23
<210> 64
   <211> 23
   <212> DNA
   <213> Artificial sequence
<220>
   <223> Synthetic oligonucleotide
<400> 64
   tcgatcgttt ttcgtgcgtt ttt 23
<210> 65
   <211> 17
   <212> DNA
   <213> Artificial sequence
<220>
   <223> Synthetic oligonucleotide
<400> 65
   atgacgtttt tgacgtt 17
<210> 66
   <211> 17
   <212> DNA
   <213> Artificial sequence
<220>
   <223> Synthetic oligonucleotide
<400> 66
   acgacgtttt tgatgtt 17
<210> 67
   <211> 17
   <212> DNA
   <213> Artificial sequence
<220>
   <223> Synthetic oligonucleotide
<400> 67
   acgacgtttt cgacgtt 17
<210> 68
   <211> 17
   <212> DNA
   <213> Artificial sequence
<220>
   <223> Synthetic oligonucleotide
<400> 68
   atgatgtttt tgatgtt 17
<210> 69
   <211> 16
   <212> DNA
   <213> Artificial sequence
<220>
   <223> Synthetic oligonucleotide
<400> 69
   atgacgtttt gatgtt 16
<210> 70
   <211> 17
   <212> DNA
   <213> Artificial sequence
<220>
   <223> Synthetic oligonucleotide
<400> 70
   atgacgtttg tgatgtt 17
<210> 71
   <211> 17
   <212> DNA
   <213> Artificial sequence
<220>
   <223> Synthetic oligonucleotide
<400> 71
   ttgacgtttt tgatgtt 17
<210> 72
   <211> 17
   <212> DNA
   <213> Artificial sequence
<220>
   <223> Synthetic oligonucleotide
<400> 72
   atgatgtttt tgatgtt 17
<210> 73
   <211> 17
   <212> DNA
   <213> Artificial sequence
<220>
   <223> Synthetic oligonucleotide
<400> 73
   atgagctttt gtatgtt 17
<210> 74
   <211> 22
   <212> DNA
   <213> Artificial sequence
<220>
   <223> Synthetic oligonucleotide
<400> 74
   tcgacgtttt cggcggccgc cg 22
<210> 75
   <211> 24
   <212> DNA
   <213> Artificial sequence
<220>
   <223> Synthetic oligonucleotide
<400> 75
   tcctgacgtt ttcggcggcc gccg 24
<210> 76
   <211> 22
   <212> DNA
   <213> Artificial sequence
<220>
   <223> Synthetic oligonucleotide
<400> 76
   tcctgacgtt cggcggccgc cg 22
<210> 77
   <211> 23
   <212> DNA
   <213> Artificial sequence
<220>
   <223> Synthetic oligonucleotide
<400> 77
   tccatgacgt tcggcgcgcg ccc 23
<210> 78
   <211> 21
   <212> DNA
   <213> Artificial sequence
<220>
   <223> Synthetic oligonucleotide
<400> 78
   tcctgacgtt cggcgcgcgc c 21
<210> 79
   <211> 22
   <212> DNA
   <213> Artificial sequence
<220>
   <223> Synthetic oligonucleotide
<400> 79
   tcgacgtttt cggcgcgcgc cg 22
<210> 80
   <211> 22
   <212> DNA
   <213> Artificial sequence
<220>
   <223> Synthetic oligonucleotide
<400> 80
   tcgacgtttt cggcggccgc cg 22
<210> 81
   <211> 24
   <212> DNA
   <213> Artificial sequence
<220>
   <223> Synthetic oligonucleotide
<400> 81
   tcgacgtcga cgttagggtt aggg 24
<210> 82
   <211> 21
   <212> DNA
   <213> Artificial sequence
<220>
   <223> Synthetic oligonucleotide
<400> 82
   acgacgtcgt tagggttagg g 21
<210> 83
   <211> 12
   <212> DNA
   <213> Artificial sequence
<220>
   <223> Synthetic oligonucleotide
<400> 83
   gtcggcgttg ac 12
<210> 84
   <211> 25
   <212> DNA
   <213> Artificial sequence
<220>
   <223> Synthetic oligonucleotide
<220>
   <221> misc_feature
   <222> (13)..(16)
   <223> where n is d-spacer
<400> 84
   acgacgtcgt cgnnnncggc cgccg 25
<210> 85
   <211> 25
   <212> DNA
   <213> Artificial sequence
<220>
   <223> Synthetic oligonucleotide
<220>
   <221> misc_feature
   <222> (13)..(16)
   <223> where n is d-spacer
<400> 85
   acgacgtcgt cgnnnncggc cgccg 25
<210> 86
   <211> 18
   <212> DNA
   <213> Artificial sequence
<220>
   <223> Synthetic oligonucleotide
<400> 86
   tcgtcgacga cgtcgtcg 18
<210> 87
   <211> 22
   <212> DNA
   <213> Artificial sequence
<220>
   <223> Synthetic oligonucleotide
<220>
   <221> misc_feature
   <222> (19) .. (22)
   <223> where n is d-spacer
<400> 87
   tcgtcgacga cgtcgtcgnn nn 22
<210> 88
   <211> 24
   <212> DNA
   <213> Artificial sequence
<220>
   <223> Synthetic oligonucleotide
<400> 88
   acgacgtcgt ttttacgacg tcgt 24
<210> 89
   <211> 27
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<400> 89
   acgacgtcgt nnnnacgacg tcgtnnn 27
<210> 90
   <211> 30
   <212> DNA
   <213> Artificial sequence
<220>
   <223> Synthetic oligonucleotide
<220>
   <221> misc_feature
   <222> (1) .. (3)
   <223> where n is d-spacer
<220>
   <221> misc_feature
   <222> (14) .. (17)
   <223> where n is d-spacer
<220>
   <221> misc_feature
   <222> (28) .. (30)
   <223> where n is d-spacer
<400> 90
   nnnacgacgt cgtnnnnacg acgtcgtnnn 30
<210> 91
   <211> 27
   <212> DNA
   <213> Artificial sequence
<220>
   <223> Synthetic oligonucleotide
<220>
   <221> misc_feature
   <222> (25) .. (27)
   <223> where n is d-spacer
<400> 91
   acgacgtcgt ttttacgacg tcgtnnn 27
<210> 92
   <211> 27
   <212> DNA
   <213> Artificial sequence
<220>
   <223> Synthetic oligonucleotide
<400> 92
   acgacgtcgt ttttacgacg tcgtttt 27
<210> 93
   <211> 27
   <212> DNA
   <213> Artificial sequence
<220>
   <223> Synthetic oligonucleotide
<400> 93
   acgacgtcgt ttttacgacg tcgtttt 27
<210> 94
   <211> 24
   <212> DNA
   <213> Artificial sequence
<220>
   <223> Synthetic oligonucleotide
<400> 94
   acgacgtcgt ttttacgacg tcgt 24
<210> 95
   <211> 24
   <212> DNA
   <213> Artificial sequence
<220>
   <223> Synthetic oligonucleotide
<220>
   <221> misc_feature
   <222> (11) .. (14)
   <223> where n is d-spacer
<400> 95
   acgacgtcgt nnnnacgacg tcgt 24
<210> 96
   <211> 20
   <212> DNA
   <213> Artificial sequence
<220>
   <223> Synthetic oligonucleotide
<400> 96
   acgacgtcgt acgacgtcgt 20
<210> 97
   <211> 20
   <212> DNA
   <213> Artificial sequence
<220>
   <223> Synthetic oligonucleotide
<400> 97
   acgacgtcgt acgacgtcgt 20
<210> 98
   <211> 25
   <212> DNA
   <213> Artificial sequence
<220>
   <223> Synthetic oligonucleotide
<220>
   <221> misc_feature
   <222> (9) .. (12)
   <223> where n is d-spacer
<220>
   <221> misc_feature
   <222> (13) .. (13)
   <223> n is a, c, g, or t
<220>
   <221> misc_feature
   <222> (23) .. (25)
   <223> where 6n is d-spacer
<400> 98
   cgacgtcgtn nnnacgacgt cgnnn 25
<210> 99
   <211> 25
   <212> DNA
   <213> Artificial sequence
<220>
   <223> Synthetic oligonucleotide
<220>
   <221> misc_feature
   <222> (9) .. (12)
   <223> where n is d-spacer
<220>
   <221> misc_feature
   <222> (13) .. (13)
   <223> n is a, c, g, or t
<220>
   <221> misc_feature
   <222> (23) .. (25)
   <223> where n is d-spacer
<400> 99
   acgacgtcgn nnncgacgtc gtnnn 25
<210> 100
   <211> 23
   <212> DNA
   <213> Artificial sequence
<220>
   <223> Synthetic oligonucleotide
<220>
   <221> misc_feature
   <222> (8) .. (11)
   <223> where n is d-spacer
<220>
   <221> misc_feature
   <222> (12) .. (12)
   <223> n is a, c, g, or t
<220>
   <221> misc_feature
   <222> (21) .. (23)
   <223> where n is d-spacer
<400> 100
   cgacgtcgnn nncgacgtcg nnn 23
<210> 101
   <211> 27
   <212> DNA
   <213> Artificial sequence
<220>
   <223> Synthetic oligonucleotide
<220>
   <221> misc_feature
   <222> (11) .. (14)
<220>
   <221> misc_feature
   <222> (11) .. (14)
   <223> n is a, c, g, or t
<220>
   <221> misc_feature
   <222> (25) .. (27)
<220>
   <221> misc_feature
   <222> (25) .. (27)
   <223> n is a, c, g, or t
<400> 101
   tcgacgtcgt nnnnacgacg tcgannn 27
<210> 102
   <211> 27
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<400> 102
   acgtcgtcgt nnnnacgacg acgtnnn 27
<210> 103
   <211> 27
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<400> 103
   tcgtcgacgt nnnnacgtcg acgannn 27
<210> 104
   <211> 27
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> mise_feature 25
   <222> (25) .. (27)
   <223> where n is d-spacer
<220>
   <221> misc_feature
   <222> (25) .. (27)
   <223> n is a, c, g, or t
<400> 104
   tcgacgtcgt nnnnacgacg tcgtnnn 27
<210> 105
   <211> 27
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<400> 105
   acgacgtcgt nnnnacgtcg tcgtnnn 27
<210> 106
   <211> 23
   <212> DNA
   <213> Artificial sequence
<220>
   <223> Synthetic oligonucleotide
<220>
   <221> misc_feature
   <222> (9) .. (12)
   <223> where n is d-spacer
<220>
   <221> misc_feature
   <222> (21) .. (23)
   <223> where n is d-spacer
<400> 106
   acgacgttnn nnaacgtcgt nnn 23
<210> 107
   <211> 21
   <212> DNA
   <213> Artificial sequence
<220>
   <223> Synthetic oligonucleotide
<220>
   <221> misc_feature
   <222> (8) .. (11)
   <223> where n is d-spacer
<220>
   <221> misc_feature
   <222> (19) .. (21)
   <223> where n is d-spacer
<400> 107.
   acgtcgtnnn nacgacgtnn n 21
<210> 108
   <211> 23
   <212> DNA
   <213> Artificial sequence
<220>
   <223> Synthetic oligonucleotide
<220>
   <221> misc_feature
   <222> (9) .. (12)
   <223> where n is d-spacer
<220>
   <221> misc_feature
   <222> (21) .. (23)
   <223> where n is d-spacer
<400> 108
   ggcggccgnn nncggccgcc nnn 23
<210> 109
   <211> 23
   <212> DNA
   <213> Artificial sequence
<220>
   <223> Synthetic oligonucleotide
<220>
   <221> misc_feature
   <222> (9) .. (12)
   <223> where n is d-spacer
<220>
   <221> misc_feature
   <222> (21) .. (23)
   <223> where n is d-spacer
<400> 109
   gcggccggnn nnccggccgc nnn 23
<210> 110
   <211> 24
   <212> DNA
   <213> Artificial sequence
<220>
   <223> Synthetic oligonucleotide
<220>
   <221> misc_feature
   <222> (8) .. (11)
   <223> where n is d-spacer
<220>
   <221> misc_feature
   <222> (22) .. (24)
   <223> where n is d-spacer
<400> 110
   acgtcgtnnn nacgacgtcg tnnn 24
<210> 111
   <211> 26
   <212> DNA
   <213> Artificial sequence
<220>
   <223> Synthetic oligonucleotide
<220>
   <221> misc_feature
   <222> (1) .. (1)
   <223> where n is d-spacer
<220>
   <221> misc_feature
   <222> (12) .. (15)
   <223> where n is d-spacer
<220>
   <221> misc_feature
   <222> (26) .. (26)
   <223> where n is d-spacer
<400> 111
   nacgacgtcg tnnnnacgac gtcgtn 26
<210> 112
   <211> 28
   <212> DNA
   <213> Artificial sequence
<220>
   <223> Synthetic oligonucleotide
<220>
   <221> misc_feature
   <222> (26) .. (27)
   <223> where n is d-spacer
<400> 112
   acgacgtcgt cgaagacgac gtcgtnnt 28
<210> 113
   <211> 28
   <212> DNA
   <213> Artificial sequence
<220>
   <223> Synthetic oligonucleotide
<220>
   <221> misc_feature
   <222> (26) .. (27)
   <223> where n is d-spacer
<400> 113
   tcgacgtcgt cgaagacgtc gtcgtnnt 28
<210> 114
   <211> 29
   <212> DNA
   <213> Artificial sequence
<220>
   <223> Synthetic oligonucleotide
<400> 114
   ccacgacgtc gtcgaagacg acgtcgtgg 29
<210> 115
   <211> 20
   <212> DNA
   <213> Artificial sequence
<220>
   <223> Synthetic oligonucleotide
<220>
   <221> misc_feature
   <222> (5) .. (5)
   <223> where n is d-spacer
<400> 115
   tccangacgt ttttgatgtt 20
<210> 116
   <211> 20
   <212> DNA
   <213> Artificial sequence
<220>
   <223> Synthetic oligonucleotide
<400> 116
   tccatgacgt tdttgatgtt 20
<210> 117
   <211> 19
   <212> DNA
   <213> Artificial sequence
<220>
   <223> Synthetic oligonucleotide
<400> 117
   tccagacgtt tttgatgtt 19
<210> 118
   <211> 19
   <212> DNA
   <213> Artificial sequence
<220>
   <223> Synthetic oligonucleotide
<400> 118
   tccatgacgt tttgatgtt 19
<210> 119
   <211> 20
   <212> DNA
   <213> Artificial sequence
<220>
   <223> Synthetic oligonucleotide
<400> 119
   tccatgacgt ttttgatgtt 20
<210> 120
   <211> 15
   <212> DNA
   <213> Artificial sequence
<220>
   <223> Synthetic oligonucleotide
<400> 120
   gacgtttttg atgtt 15
<210> 121
   <211> 18
   <212> DNA
   <213> Artificial sequence
<220>
   <223> Synthetic oligonucleotide
<400> 121
   tccagacgtt ttgatgtt 18
<210> 122
   <211> 20
   <212> DNA
   <213> Artificial sequence
<220>
   <223> Synthetic oligonucleotide
<220>
   <221> misc_feature
   <222> (5) .. (5)
   <223> where n is d-spacer
<220>
   <221> misc_feature
   <222> (12) .. (12)
   <223> where n is d-spacer
<400> 122
   tccangacgt tnttgatgtt 20
<210> 123
   <211> 28
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (28)
   <223> where n is d-spacer
<400> 123
   acgacgtcgt nnnnacgacg tcgtnnnu 28
<210> 124
   <211> 28
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<220>
   <221> misc_feature
   <222> (28) .. (28)
   <223> where g is a ribonucleoside
<400> 124
   acgacgtcgt nnnnacgacg tcgtnnng 28
<210> 125
   <211> 28
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<220>
   <221> misc_feature
   <222> (28) .. (28)
   <223> where a is a ribonucleoside
<400> 125
   acgacgtcgt nnnnacgacg tcgtnnna 28
<210> 126
   <211> 31
   <212> DNA
   <213> Artificial sequence
<220>
   <223> Synthetic oligonucleotide
<220>
   <221> misc_feature
   <222> (1) .. (3)
   <223> where n is d-spacer
<220>
   <221> misc_feature
   <222> (14) .. (17)
   <223> where n is d-spacer
<220>
   <221> misc_feature
   <222> (28) .. (30)
   <223> where n is d-spacer
<400> 126
   nnnacgacgt cgtnnnnacg acgtcgtnnn u 31
<210> 127
   <211> 31
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<220>
   <221> misc_feature
   <222> (28) .. (31)
   <223> where a is a ribonucleoside
<400> 127
   acgacgtcgt nnnnacgacg tcgtnnnaaa a 31
<210> 128
   <211> 20
   <212> DNA
   <213> Artificial sequence
<220>
   <223> Synthetic oligonucleotide
<400> 128
   tcgatgacgt tcctgacgtt 20
<210> 129
   <211> 31
   <212> DNA
   <213> Artificial sequence
<220>
   <223> Synthetic oligonucleotide
<220>
   <221> misc_feature
   <222> (14) .. (17)
   <223> where n is d-spacer
<220>
   <221> misc_feature
   <222> (28) .. (30)
   <223> where n is d-spacer
<400> 129
   tttacgacgt cgtnnnnacg acgtcgtnnn u 31
<210> 130
   <211> 10
   <212> DNA
   <213> Artificial sequence
<220>
   <223> Synthetic oligonucleotide
<400> 130
   tcgacgtcgt 10
<210> 131
   <211> 22
   <212> DNA
   <213> Artificial sequence
<220>
   <223> Synthetic oligonucleotide
<400> 131
   tcgacgtttt cggcggccgc cg 22
<210> 132
   <211> 22
   <212> DNA
   <213> Artificial sequence
<220>
   <223> Synthetic oligonucleotide
<400> 132
   tcgacgtttt cggcgcgcgc cg 22
<210> 133
   <211> 22
   <212> DNA
   <213> Artificial sequence
<220>
   <223> Synthetic oligonucleotide
<400> 133
   tcgcgacgtt cggcgcgcgc cg 22
<210> 134
   <211> 22
   <212> DNA
   <213> Artificial sequence
<220>
   <223> Synthetic oligonucleotide
<400> 134
   tcgcgacgtt cggcgcgcgc cg 22
<210> 135
   <211> 22
   <212> DNA
   <213> Artificial sequence
<220>
   <223> Synthetic oligonucleotide
<400> 135
   tcgcgacgtt cggcgcgcgc cg 22
<210> 136
   <211> 22
   <212> DNA
   <213> Artificial sequence
<220>
   <223> Synthetic oligonucleotide
<400> 136
   tcgcgacgtt cggcgcgcgc cg 22
<210> 137
   <211> 22
   <212> DNA
   <213> Artificial sequence
<220>
   <223> Synthetic oligonucleotide
<400> 137
   tcgcgacgtt cggcgcgcgc cg 22
<210> 138
   <211> 22
   <212> DNA
   <213> Artificial sequence
<220>
   <223> Synthetic oligonucleotide
<400> 138
   tcgcgacgtt cggcgcgcgc cg 22
<210> 139
   <211> 22
   <212> DNA
   <213> Artificial sequence
<220>
   <223> Synthetic oligonucleotide
<400> 139
   tcgcgacgtt cggcgcgcgc cg 22
<210> 140
   <211> 22
   <212> DNA
   <213> Artificial sequence
<220>
   <223> Synthetic oligonucleotide
<400> 140
   tcgcgacgtt cggcgcgcgc cg 22
<210> 141
   <211> 20
   <212> DNA
   <213> Artificial sequence
<220>
   <223> Synthetic oligonucleotide
<400> 141
   tcgcgacgtt cgcgcgcgcg 20
<210> 142
   <211> 20
   <212> DNA
   <213> Artificial sequence
<220>
   <223> Synthetic oligonucleotide
<220>
   <221> misc_feature
   <222> (1) .. (1)
   <223> where n is d-spacer
<400> 142
   nccatgacgt ttttgatgtt 20
<210> 143
   <211> 20
   <212> DNA
   <213> Artificial sequence
<220>
   <223> Synthetic oligonucleotide
<220>
   <221> misc_feature
   <222> (2) .. (2)
   <223> where n is d-spacer
<400> 143
   tncatgacgt ttttgatgtt 20
<210> 144
   <211> 20
   <212> DNA
   <213> Artificial sequence
<220>
   <223> Synthetic oligonucleotide
<220>
   <221> misc_feature
   <222> (3)..(3)
   <223> where n is d-spacer
<400> 144
   tcnatgacgt ttttgatgtt 20
<210> 145
   <211> 20
   <212> DNA
   <213> Artificial sequence
<220>
   <223> Synthetic oligonucleotide
<220>
   <221> misc_feature
   <222> (4) .. (4)
   <223> where n is d-spacer
<400> 145
   tccntgacgt ttttgatgtt 20
<210> 146
   <211> 20
   <212> DNA
   <213> Artificial sequence
<220>
   <223> Synthetic oligonucleotide
<220>
   <221> misc_feature
   <222> (14) .. (14)
   <223> where n is d-spacer
<400> 146
   tccatgacgt tttngatgtt 20
<210> 147
   <211> 20
   <212> DNA
   <213> Artificial sequence
<220>
   <223> Synthetic oligonucleotide
<220>
   <221> misc_feature
   <222> (13) .. (13)
   <223> where n is d-spacer
<400> 147
   tccatgacgt ttntgatgtt 20
<210> 148
   <211> 21
   <212> DNA
   <213> Artificial sequence
<220>
   <223> Synthetic oligonucleotide
<400> 148
   tcgaacgttc ggcgcgcgcc g 21
<210> 149
   <211> 24
   <212> DNA
   <213> Artificial sequence
<220>
   <223> Synthetic oligonucleotide
<400> 149
   tcgtcgaacg ttcggcgcgc gccg 24
<210> 150
   <211> 25
   <212> DNA
   <213> Artificial sequence
<220>
   <223> Synthetic oligonucleotide
<400> 150
   tcgtcgaacg ttcggcgctg cgccg 25
<210> 151
   <211> 24
   <212> DNA
   <213> Artificial sequence
<220>
   <223> Synthetic oligonucleotide
<400> 151
   tcgcgacgtt cgttgcgcgc gccg 24
<210> 152
   <211> 21
   <212> DNA
   <213> Artificial sequence
<220>
   <223> Synthetic oligonucleotide
<400> 152
   tacgtcgttc ggcgcgcgcc g 21
<210> 153
   <211> 23
   <212> DNA
   <213> Artificial sequence
<220>
   <223> Synthetic oligonucleotide
<400> 153
   ttcgcgacgt tcggcgcgcg ccg 23
<210> 154
   <211> 22
   <212> DNA
   <213> Artificial sequence
<220>
   <223> Synthetic oligonucleotide
<400> 154
   tcggcgcgcg ccgtcgcgac gt 22
<210> 155
   <211> 24
   <212> DNA
   <213> Artificial sequence
<220>
   <223> Synthetic oligonucleotide
<400> 155
   tagcgtgcgt tttgacgttt tttt 24
<210> 156
   <211> 24
   <212> DNA
   <213> Artificial sequence
<220>
   <223> Synthetic oligonucleotide
<400> 156
   tagcgagcgt tttgacgttt tttt 24
<210> 157
   <211> 24
   <212> DNA
   <213> Artificial sequence
<220>
   <223> Synthetic oligonucleotide
<400> 157
   ttgcgagcgt tttgacgttt tttt 24
<210> 158
   <211> 24
   <212> DNA
   <213> Artificial sequence
<220>
   <223> Synthetic oligonucleotide
<400> 158
   atgcgtgcgt tttgacgttt tttt 24
<210> 159
   <211> 24
   <212> DNA
   <213> Artificial sequence
<220>
   <223> Synthetic oligonucleotide
<400> 159
   ttacgtgcgt tttgacgttt tttt 24
<210> 160
   <211> 24
   <212> DNA
   <213> Artificial sequence
<220>
   <223> Synthetic oligonucleotide
<400> 160
   ttgcatgcgt tttgacgttt tttt 24
<210> 161
   <211> 24
   <212> DNA
   <213> Artificial sequence
<220>
   <223> Synthetic oligonucleotide
<400> 161
   ttgcgtacgt tttgacgttt tttt 24
<210> 162
   <211> 24
   <212> DNA
   <213> Artificial sequence
<220>
   <223> Synthetic oligonucleotide
<400> 162
   ttgcgtgcat tttgacgttt tttt 24
<210> 163
   <211> 24
   <212> DNA
   <213> Artificial sequence
<220>
   <223> Synthetic oligonucleotide
<400> 163
   ttgcgtgcga tttgacgttt tttt 24
<210> 164
   <211> 24
   <212> DNA
   <213> Artificial sequence
<220>
   <223> Synthetic oligonucleotide
<400> 164
   ttgcgcgcgt tttgacgttt tttt 24
<210> 165
   <211> 24
   <212> DNA
   <213> Artificial sequence
<220>
   <223> Synthetic oligonucleotide
<400> 165
   ttgcgtgcgc tttgacgttt tttt 24
<210> 166
   <211> 24
   <212> DNA
   <213> Artificial sequence
<220>
   <223> Synthetic oligonucleotide
<400> 166
   ttgcgtgcgt ttcgacgttt tttt 24
<210> 167
   <211> 25
   <212> DNA
   <213> Artificial sequence
<220>
   <223> Synthetic oligonucleotide
<400> 167
   tcgtcgaacg ttcggcgctg cgccg 25
<210> 168
   <211> 25
   <212> DNA
   <213> Artificial sequence
<220>
   <223> Synthetic oligonucleotide
<400> 168
   tcgtcgaacg ttcggcgctg cgccg 25
<210> 169
   <211> 25
   <212> DNA
   <213> Artificial sequence
<220>
   <223> Synthetic oligonucleotide
<400> 169
   tcgtcgaacg ttcggcgctg cgccg 25
<210> 170
   <211> 25
   <212> DNA
   <213> Artificial sequence
<220>
   <223> Synthetic oligonucleotide
<400> 170
   tcgtcgaacg ttcggcgctg cgccg 25
<210> 171
   <211> 25
   <212> DNA
   <213> Artificial sequence
<220>
   <223> Synthetic oligonucleotide
<400> 171
   tcgtcggacg ttcggcgctg cgccg 25
<210> 172
   <211> 24
   <212> DNA
   <213> Artificial sequence
<220>
   <223> Synthetic oligonucleotide
<400> 172
   tcgcgacgtt cgttgcgcgc gccg 24
<210> 173
   <211> 24
   <212> DNA
   <213> Artificial sequence
<220>
   <223> Synthetic oligonucleotide
<400> 173
   tcgcgacgtt cgttgcgcgc gccg 24
<210> 174
   <211> 24
   <212> DNA
   <213> Artificial sequence
<220>
   <223> Synthetic oligonucleotide
<400> 174
   tcgcgacgtt cgttgcgcgc gccg 24
<210> 175
   <211> 20
   <212> DNA
   <213> Artificial sequence
<220>
   <223> Synthetic oligonucleotide
<400> 175
   tcgcgacgtt ttgcgcgcgc 20
<210> 176
   <211> 23
   <212> DNA
   <213> Artificial sequence
<220>
   <223> Synthetic oligonucleotide
<400> 176
   tcgcgacgtc gttgcgcgcg ccg 23
<210> 177
   <211> 24
   <212> DNA
   <213> Artificial sequence
<220>
   <223> Synthetic oligonucleotide
<400> 177
   tcgcgacgtt cgaagcgcgc gccg 24
<210> 178
   <211> 24
   <212> DNA
   <213> Artificial sequence
<220>
   <223> Synthetic oligonucleotide
<400> 178
   tcgcgacgaa cgttgcgcgc gccg 24
<210> 179
   <211> 27
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<400> 179
   tcgacgtcgt nnnntcgacg tcgtnnn 27
<210> 180
   <211> 24
   <212> DNA
   <213> Artificial sequence
<220>
   <223> Synthetic oligonucleotide
<400> 180
   tcgtcgttag ctcgttagct cgtt 24
<210> 181
   <211> 24
   <212> DNA
   <213> Artificial sequence
<220>
   <223> Synthetic oligonucleotide
<400> 181
   tcgtcgttac gtaattacgt cgtt 24
<210> 182
   <211> 24
   <212> DNA
   <213> Artificial sequence
<220>
   <223> Synthetic oligonucleotide
<400> 182
   tcgtcgttac gtcgttacgt aatt 24
<210> 183
   <211> 24
   <212> DNA
   <213> Artificial sequence
<220>
   <223> Synthetic oligonucleotide
<400> 183
   tcgtcgttac gtaattacgt aatt 24
<210> 184
   <211> 19
   <212> DNA
   <213> Artificial sequence
<220>
   <223> Synthetic oligonucleotide
<400> 184
   tcgacgtcga cgtgacggg 19
<210> 185
   <211> 11
   <212> DNA
   <213> Artificial sequence
<220>
   <223> Synthetic oligonucleotide
<400> 185
   tcgacgtcgt t 11
<210> 186
   <211> 11
   <212> DNA
   <213> Artificial sequence
<220>
   <223> Synthetic oligonucleotide
<400> 186
   tcgacgtcgt t 11
<210> 187
   <211> 12
   <212> DNA
   <213> Artificial sequence
<220>
   <223> Synthetic oligonucleotide
<400> 187
   tcgacgtcgt tt 12
<210> 188
   <211> 24
   <212> DNA
   <213> Artificial sequence
<220>
   <223> Synthetic oligonucleotide
<400> 188
   tcgacgtcgt ttttcgacgt cgtt 24
<210> 189
   <211> 23
   <212> DNA
   <213> Artificial sequence
<220>
   <223> Synthetic oligonucleotide
<400> 189
   tcgcgacgtt cggcgcgctg ccg 23
<210> 190
   <211> 23
   <212> DNA
   <213> Artificial sequence
<220>
   <223> Synthetic oligonucleotide
<400> 190
   tcgcgacgtt cggcgcgtcg ccg 23
<210> 191
   <211> 23
   <212> DNA
   <213> Artificial sequence
<220>
   <223> Synthetic oligonucleotide
<400> 191
   tcgcgacgtt cggcggctcg ccg 23
<210> 192
   <211> 23
   <212> DNA
   <213> Artificial sequence
<220>
   <223> Synthetic oligonucleotide
<400> 192
   tcgcgacgtt cggcgcgtcg ccg 23
<210> 193
   <211> 23
   <212> DNA
   <213> Artificial sequence
<220>
   <223> Synthetic oligonucleotide
<400> 193
   tcgcgacgtt cggcggctcg ccg 23
<210> 194
   <211> 23
   <212> DNA
   <213> Artificial sequence
<220>
   <223> Synthetic oligonucleotide
<400> 194
   tcgcgacgtt cggcgcgtcg ccg 23
<210> 195
   <211> 23
   <212> DNA
   <213> Artificial sequence
<220>
   <223> Synthetic oligonucleotide
<400> 195
   tcgcgacgtt cggcggctcg ccg 23
<210> 196
   <211> 10
   <212> DNA
   <213> Artificial sequence
<220>
   <223> Synthetic oligonucleotide
<400> 196
   tcgacgtcgt 10
<210> 197
   <211> 19
   <212> DNA
   <213> Artificial sequence
<220>
   <223> Synthetic oligonucleotide
<400> 197
   tcgacgtcga cgtgacggg 19
<210> 198
   <211> 19
   <212> DNA
   <213> Artificial sequence
<220>
   <223> Synthetic oligonucleotide
<400> 198
   tcgacgtcga cgtgacggg 19
<210> 199
   <211> 19
   <212> DNA
   <213> Artificial sequence
<220>
   <223> Synthetic oligonucleotide
<400> 199
   tcgacgtcga cgtgacgtc 19
<210> 200
   <211> 17
   <212> DNA
   <213> Artificial sequence
<220>
   <223> Synthetic oligonucleotide
<400> 200
   tcgacgtcga cgtgacg 17
<210> 201
   <211> 10
   <212> DNA
   <213> Artificial sequence
<220>
   <223> Synthetic oligonucleotide
<400> 201
   tcgacgtcga 10
<210> 202
   <211> 24
   <212> DNA
   <213> Artificial sequence
<220>
   <223> Synthetic oligonucleotide
<400> 202
   tcgtcgttac gtaactacgt cgtt 24
<210> 203
   <211> 24
   <212> DNA
   <213> Artificial sequence
<220>
   <223> Synthetic oligonucleotide
<400> 203
   tcgtcgttac gtaacgacgt cgtt 24
<210> 204
   <211> 24
   <212> DNA
   <213> Artificial sequence
<220>
   <223> Synthetic oligonucleotide
<400> 204
   tcgtcgttac gtaacgacga cgtt 24
<210> 205
   <211> 24
   <212> DNA
   <213> Artificial sequence
<220>
   <223> Synthetic oligonucleotide
<400> 205
   tcgtcgttag ctaattagct cgtt 24
<210> 206
   <211> 24
   <212> DNA
   <213> Artificial sequence
<220>
   <223> Synthetic oligonucleotide
<400> 206
   tcgtcgttac gtaattagct cgtt 24
<210> 207
   <211> 20
   <212> DNA
   <213> Artificial sequence
<220>
   <223> Synthetic oligonucleotide
<400> 207
   cccatgacgt tcctgacgtt 20
<210> 208
   <211> 20
   <212> DNA
   <213> Artificial sequence
<220>
   <223> Synthetic oligonucleotide
<400> 208
   gccatgacgt tcctgacgtt 20
<210> 209
   <211> 20
   <212> DNA
   <213> Artificial sequence
<220>
   <223> Synthetic oligonucleotide
<400> 209
   accatgacgt tcctgacgtt 20
<210> 210
   <211> 20
   <212> DNA
   <213> Artificial sequence
<220>
   <223> Synthetic oligonucleotide
<400> 210
   tggatgacgt tcctgacgtt 20
<210> 211
   <211> 20
   <212> DNA
   <213> Artificial sequence
<220>
   <223> Synthetic oligonucleotide
<400> 211
   tttatgacgt tcctgacgtt 20
<210> 212
   <211> 20
   <212> DNA
   <213> Artificial sequence
<220>
   <223> Synthetic oligonucleotide
<400> 212
   taaatgacgt tcctgacgtt 20
<210> 213
   <211> 19
   <212> DNA
   <213> Artificial sequence
<220>
   <223> Synthetic oligonucleotide
<400> 213
   ccatgacgtt cctgacgtt 19
<210> 214
   <211> 18
   <212> DNA
   <213> Artificial sequence
<220>
   <223> Synthetic oligonucleotide
<400> 214
   catgacgttc ctgacgtt 18
<210> 215
   <211> 17
   <212> DNA
   <213> Artificial sequence
<220>
   <223> Synthetic oligonucleotide
<400> 215
   atgacgttcc tgacgtt 17
<210> 216
   <211> 16
   <212> DNA
   <213> Artificial sequence
<220>
   <223> Synthetic oligonucleotide
<400> 216
   tgacgttcct gacgtt 16
<210> 217
   <211> 24
   <212> DNA
   <213> Artificial sequence
<220>
   <223> Synthetic oligonucleotide
<400> 217
   tcgacgtcga ddddtcgacg tcga 24
<210> 218
   <211> 24
   <212> DNA
   <213> Artificial sequence
<220>
   <223> Synthetic oligonucleotide
<220>
   <221> misc_feature
   <222> (1) .. (10)
   <223> where the nucleosides are inverse nucleosides
<220>
   <221> misc_feature
   <222> (11) .. (14)
   <223> n is a, c, g, or t
<400> 218
   agctgcagct nnnntcgacg tcga 24
<210> 219
   <211> 22
   <212> DNA
   <213> Artificial sequence
<220>
   <223> Synthetic oligonucleotide
<400> 219
   tcgcgacgtt cggcgcgcgc cg 22
<210> 220
   <211> 23
   <212> DNA
   <213> Artificial sequence
<220>
   <223> Synthetic oligonucleotide
<400> 220
   tcgtcgacgt tcggcgcgcg ccg 23
<210> 221
   <211> 21
   <212> DNA
   <213> Artificial sequence
<220>
   <223> Synthetic oligonucleotide
<400> 221
   tcggacgttc ggcgcgcgcc g 21
<210> 222
   <211> 19
   <212> DNA
   <213> Artificial sequence
<220>
   <223> Synthetic oligonucleotide
<400> 222
   tcggacgttc ggcgcgccg 19
<210> 223
   <211> 20
   <212> DNA
   <213> Artificial sequence
<220>
   <223> Synthetic oligonucleotide
<400> 223
   tcgcgacgtt cggcgcgccg 20
<210> 224
   <211> 20
   <212> DNA
   <213> Artificial sequence
<220>
   <223> Synthetic oligonucleotide
<400> 224
   tcgcgacgtt cgcgcgcgcg 20
<210> 225
   <211> 20
   <212> DNA
   <213> Artificial sequence
<220>
   <223> Synthetic oligonucleotide
<400> 225
   tcgacgttcg gcgcgcgccg 20
<210> 226
   <211> 18
   <212> DNA
   <213> Artificial sequence
<220>
   <223> Synthetic oligonucleotide
<400> 226
   tcgacgttcg gcgcgccg 18
<210> 227
   <211> 18
   <212> DNA
   <213> Artificial sequence
<220>
   <223> Synthetic oligonucleotide
<400> 227
   tcgcgacgtt cggcgccg 18
<210> 228
   <211> 16
   <212> DNA
   <213> Artificial sequence
<220>
   <223> Synthetic oligonucleotide
<400> 228
   tcgcgacgtt cggccg 16
<210> 229
   <211> 16
   <212> DNA
   <213> Artificial sequence
<220>
   <223> Synthetic oligonucleotide
<400> 229
   tcgacgttcg gcgccg 16
<210> 230
   <211> 21
   <212> DNA
   <213> Artificial sequence
<220>
   <223> Synthetic oligonucleotide
<400> 230
   tcgtcgacgt tcggcgcgcc g 21
<210> 231
   <211> 19
   <212> DNA
   <213> Artificial sequence
<220>
   <223> Synthetic oligonucleotide
<400> 231
   tcgtcgacgt tcggcgccg 19
<210> 232
   <211> 23
   <212> DNA
   <213> Artificial sequence
<220>
   <223> Synthetic oligonucleotide
<400> 232
   tcgacgacgt tcggcgcgcg ccg 23
<210> 233
   <211> 23
   <212> DNA
   <213> Artificial sequence
<220>
   <223> Synthetic oligonucleotide
<400> 233
   tcgacgtcgt tcggcgcgcg ccg 23
<210> 234
   <211> 23
   <212> DNA
   <213> Artificial sequence
<220>
   <223> Synthetic oligonucleotide
<400> 234
   tcgtcgacga tcggcgcgcg ccg 23
<210> 235
   <211> 21
   <212> DNA
   <213> Artificial sequence
<220>
   <223> Synthetic oligonucleotide
<400> 235
   tcgtcgacga tcggcgcgcc g 21
<210> 236
   <211> 23
   <212> DNA
   <213> Artificial sequence
<220>
   <223> Synthetic oligonucleotide
<400> 236
   tcgtcgacgt tcgccgcgcg gcg 23
<210> 237
   <211> 24
   <212> DNA
   <213> Artificial sequence
<220>
   <223> Synthetic oligonucleotide
<400> 237
   tcgtcgacgt tcggcgccgt gccg 24
<210> 238
   <211> 23
   <212> DNA
   <213> Artificial sequence
<220>
   <223> Synthetic oligonucleotide
<400> 238
   tcgtcgacgt tcgactcgag tcg 23
<210> 239
   <211> 24
   <212> DNA
   <213> Artificial sequence
<220>
   <223> Synthetic oligonucleotide
<400> 239
   tcgtcgttac gtaacgacga cgtt 24
<210> 240
   <211> 24
   <212> DNA
   <213> Artificial sequence
<220>
   <223> Synthetic oligonucleotide
<400> 240
   tcgtcgttac gtaacgacga cgtt 24
<210> 241
   <211> 19
   <212> DNA
   <213> Artificial sequence
<220>
   <223> Synthetic oligonucleotide
<400> 241
   tcgacgtcga cgtgacgtt 19
<210> 242
   <211> 21
   <212> DNA
   <213> Artificial sequence
<220>
   <223> Synthetic oligonucleotide
<400> 242
   tcgtcgacgt tcggcgcgcc g 21
<210> 243
   <211> 23
   <212> DNA
   <213> Artificial sequence
<220>
   <223> Synthetic oligonucleotide
<400> 243
   tcgtcgacga tcggcgcgcg ccg 23
<210> 244
   <211> 28
   <212> DNA
   <213> Artificial sequence
<220>
   <223> Synthetic oligonucleotide
<220>
   <221> misc_feature
   <222> (11) .. (14)
   <223> where n is d-spacer
<220>
   <221> misc_feature
   <222> (25) .. (27)
   <223> where n is d-spacer
<220>
   <221> misc_feature
   <222> (28) .. (28)
   <223> where the ribonucleoside is an inverse ribonucleoside
<400> 244
   acgacgtcgt nnnnacgacg tcgtnnnu 28
<210> 245
   <211> 24
   <212> DNA
   <213> Artificial sequence
<220>
   <223> Synthetic oligonucleotide
<400> 245
   tcgtcgacga tcggcgccgt gccg 24
<210> 246
   <211> 23
   <212> DNA
   <213> Artificial sequence
<220>
   <223> Synthetic oligonucleotide
<400> 246
   tcgtcgacga cggcgccgtg ccg 23
<210> 247
   <211> 21
   <212> DNA
   <213> Artificial sequence
<220>
   <223> Synthetic oligonucleotide
<400> 247
   tcgtcgacga cgcgccgtgc g 21
<210> 248
   <211> 24
   <212> DNA
   <213> Artificial sequence
<220>
   <223> Synthetic oligonucleotide
<400> 248
   tcgtcgacga tcggcgccgt gccg 24
<210> 249
   <211> 24
   <212> DNA
   <213> Artificial sequence
<220>
   <223> Synthetic oligonucleotide
<400> 249
   tcgtcgacga tcggcgccgt gccg 24
<210> 250
   <211> 24
   <212> DNA
   <213> Artificial sequence
<220>
   <223> Synthetic oligonucleotide
<400> 250
   tcgtcgacga tcggcgccgt gccg 24
<210> 251
   <211> 24
   <212> DNA
   <213> Artificial sequence
<220>
   <223> Synthetic oligonucleotide
<400> 251
   tcgtcgacga tcggcgccgt gccg 24
<210> 252
   <211> 23
   <212> DNA
   <213> Artificial sequence
<220>
   <223> Synthetic oligonucleotide
<400> 252
   tcgtcgacga tcggcgcgcg ccg 23
<210> 253
   <211> 23
   <212> DNA
   <213> Artificial sequence
<220>
   <223> Synthetic oligonucleotide
<400> 253
   tcgtcgacga tcggcgcgcg ccg 23
<210> 254
   <211> 23
   <212> DNA
   <213> Artificial sequence
<220>
   <223> Synthetic oligonucleotide
<400> 254
   tcgtcgacga tcggcgcgcg ccg 23
<210> 255
   <211> 19
   <212> DNA
   <213> Artificial sequence
<220>
   <223> Synthetic oligonucleotide
<400> 255
   tcgacgtcga cgtgacgtt 19
<210> 256
   <211> 19
   <212> DNA
   <213> Artificial sequence
<220>
   <223> Synthetic oligonucleotide
<400> 256
   tcgacgtcga cgtgacgtt 19
<210> 257
   <211> 19
   <212> DNA
   <213> Artificial sequence
<220>
   <223> Synthetic oligonucleotide
<400> 257
   tcgacgtcga cgtgacgtt 19
<210> 258
   <211> 19
   <212> DNA
   <213> Artificial sequence
<220>
   <223> Synthetic oligonucleotide
<400> 258
   tcgtcgacga cgtgtcgat 19
<210> 259
   <211> 19
   <212> DNA
   <213> Artificial sequence
<220>
   <223> Synthetic oligonucleotide
<400> 259
   tcgacgtcga cgtgacgtt 19
<210> 260
   <211> 19
   <212> DNA
   <213> Artificial sequence
<220>
   <223> Synthetic oligonucleotide
<400> 260
   tcgacgtcga cgtgacgtt 19
<210> 261
   <211> 24
   <212> DNA
   <213> Artificial sequence
<220>
   <223> Synthetic oligonucleotide
<400> 261
   tcgtcgacga tcggcgccgt gccg 24
<210> 262
   <211> 24
   <212> DNA
   <213> Artificial sequence
<220>
   <223> Synthetic oligonucleotide
<400> 262
   tcgtcgacga cggcgccgtg ccgt 24
<210> 263
   <211> 24
   <212> DNA
   <213> Artificial sequence
<220>
   <223> Synthetic oligonucleotide
<400> 263
   tcgtcgacga cggcgccgtg ccgt 24
<210> 264
   <211> 25
   <212> DNA
   <213> Artificial sequence
<220>
   <223> Synthetic oligonucleotide
<400> 264
   tcgtcgacga tcggcgccgt gccgt 25
<210> 265
   <211> 25
   <212> DNA
   <213> Artificial sequence
<220>
   <223> Synthetic oligonucleotide
<400> 265
   tcgtcgacgt tcggcgccgt gccgt 25
<210> 266
   <211> 24
   <212> DNA
   <213> Artificial sequence
<220>
   <223> Synthetic oligonucleotide
<400> 266
   tcgtcgacgt cggcgccgtg ccgt 24
<210> 267
   <211> 23
   <212> DNA
   <213> Artificial sequence
<220>
   <223> Synthetic oligonucleotide
<400> 267
   tcgtcgacgc ggcgccgtgc cgt 23
<210> 268
   <211> 23
   <212> DNA
   <213> Artificial sequence
<220>
   <223> Synthetic oligonucleotide
<400> 268
   tcgtcgacgc ggcgccgtgc cgt 23
<210> 269
   <211> 20
   <212> DNA
   <213> Artificial sequence
<220>
   <223> Synthetic oligonucleotide
<400> 269
   tcgtcgacga agtcgacgat 20
<210> 270
   <211> 24
   <212> DNA
   <213> Artificial sequence
<220>
   <223> Synthetic oligonucleotide
<400> 270
   tcgtcgacga gaatcgtcga cgat 24
<210> 271
   <211> 22
   <212> DNA
   <213> Artificial sequence
<220>
   <223> Synthetic oligonucleotide
<400> 271
   tcgtcgtacg gcgccgtgcc gt 22
<210> 272
   <211> 24
   <212> DNA
   <213> Artificial sequence
<220>
   <223> Synthetic oligonucleotide
<400> 272
   tcgtcgacga tcggcgccgt gccg 24
<210> 273
   <211> 24
   <212> DNA
   <213> Artificial sequence
<220>
   <223> Synthetic oligonucleotide
<400> 273
   tcgtcgacga tcggcgccgt gccg 24
<210> 274
   <211> 24
   <212> DNA
   <213> Artificial sequence
<220>
   <223> Synthetic oligonucleotide
<400> 274
   tcgtcgacga tcggcgccgt gccg 24
<210> 275
   <211> 24
   <212> DNA
   <213> Artificial sequence
<220>
   <223> Synthetic oligonucleotide
<400> 275
   tcgtcgacga cggcgccgtg ccgt 24
<210> 276
   <211> 25
   <212> DNA
   <213> Artificial sequence
<220>
   <223> Synthetic oligonucleotide
<400> 276
   tcgtcgacga tcggcgccgt gccgt 25
<210> 277
   <211> 25
   <212> DNA
   <213> Artificial sequence
<220>
   <223> Synthetic oligonucleotide
<400> 277
   tcgtcgacga tcggcgccgt gccgt 25
<210> 278
   <211> 24
   <212> DNA
   <213> Artificial sequence
<220>
   <223> Synthetic oligonucleotide
<400> 278
   tcgtcgacga cggcgccgtg ccgt 24
<210> 279
   <211> 25
   <212> DNA
   <213> Artificial sequence
<220>
   <223> Synthetic oligonucleotide
<400> 279
   tcgtcgacgt tcggcgccgt gccgt 25
<210> 280
   <211> 24
   <212> DNA
   <213> Artificial sequence
<220>
   <223> Synthetic oligonucleotide
<400> 280
   tcgtcgacgt cggcgccgtg ccgt 24
<210> 281
   <211> 20
   <212> DNA
   <213> Artificial sequence
<220>
   <223> Synthetic oligonucleotide
<400> 281
   tcgtcgacga agtcgacgat 20
<210> 282
   <211> 24
   <212> DNA
   <213> Artificial sequence
<220>
   <223> Synthetic oligonucleotide
<400> 282
   tcgtcgacga gaatcgtcga cgat 24
<210> 283
   <211> 19
   <212> DNA
   <213> Artificial sequence
<220>
   <223> Synthetic oligonucleotide
<400> 283
   tcgtcgacga cgtgtcgat 19
<210> 284
   <211> 20
   <212> DNA
   <213> Artificial sequence
<220>
   <223> Synthetic oligonucleotide
<400> 284
   tcgacgtcga agacgtcgat 20
<210> 285
   <211> 24
   <212> DNA
   <213> Artificial sequence
<220>
   <223> Synthetic oligonucleotide
<400> 285
   tcgacgtcga gaatcgacgt cgat 24
<210> 286
   <211> 21
   <212> DNA
   <213> Artificial sequence
<220>
   <223> Synthetic oligonucleotide
<400> 286
   tcgtcgacga cggcgaagcc g 21
<210> 287
   <211> 22
   <212> DNA
   <213> Artificial sequence
<220>
   <223> Synthetic oligonucleotide
<400> 287
   tcgtcgacga cggcgaagcc gt 22
<210> 288
   <211> 19
   <212> DNA
   <213> Artificial sequence
<220>
   <223> Synthetic oligonucleotide
<400> 288
   tcgtcgacga cgtgtcgat 19
<210> 289
   <211> 19
   <212> DNA
   <213> Artificial sequence
<220>
   <223> Synthetic oligonucleotide
<400> 289
   tcgtcgacga cgtgtcgat 19
<210> 290
   <211> 21
   <212> DNA
   <213> Artificial sequence
<220>
   <223> Synthetic oligonucleotide
<400> 290
   tcgacgtcga cgtgacgttg t 21
<210> 291
   <211> 23
   <212> DNA
   <213> Artificial sequence
<220>
   <223> Synthetic oligonucleotide
<400> 291
   tcgtcgacga tcggcgcgcg ccg 23
<210> 292
   <211> 23
   <212> DNA
   <213> Artificial sequence
<220>
   <223> Synthetic oligonucleotide
<400> 292
   tcgtcgacga tcggcgcgcg ccg 23
<210> 293
   <211> 24
   <212> DNA
   <213> Artificial sequence
<220>
   <223> Synthetic oligonucleotide
<220>
   <221> misc_feature
   <222> (24) .. (24)
   <223> where t is an inverse nucleoside
<400> 293
   tcgtcgacga tcggcgcgcg ccgt 24
<210> 294
   <211> 25
   <212> DNA
   <213> Artificial sequence
<220>
   <223> Synthetic oligonucleotide
<220>
   <221> misc_feature
   <222> (25) .. (25)
   <223> where t is an inverse nucleoside
<400> 294
   ttcgtcgacg atcggcgcgc gccgt 25
<210> 295
   <211> 23
   <212> DNA
   <213> Artificial sequence
<220>
   <223> Synthetic oligonucleotide
<400> 295
   tcgtcgacga tcgacgcgcg tcg 23
<210> 296
   <211> 23
   <212> DNA
   <213> Artificial sequence
<220>
   <223> Synthetic oligonucleotide
<400> 296
   tcgtcgacga tcaacgcgcg ttg 23
<210> 297
   <211> 23
   <212> DNA
   <213> Artificial sequence
<220>
   <223> Synthetic oligonucleotide
<400> 297
   tcgtcgacga tcggcacgtg ccg 23
<210> 298
   <211> 23
   <212> DNA
   <213> Artificial sequence
<220>
   <223> Synthetic oligonucleotide
<400> 298
   tcgtcgacga tcggcatatg ccg 23
<210> 299
   <211> 23
   <212> DNA
   <213> Artificial sequence
<220>
   <223> Synthetic oligonucleotide
<400> 299
   tcgtcgacga tgccgcgcgc ggc 23
<210> 300
   <211> 23
   <212> DNA
   <213> Artificial sequence
<220>
   <223> Synthetic oligonucleotide
<400> 300
   tcgtcgacga tgccgcgcgc ggc 23
<210> 301
   <211> 23
   <212> DNA
   <213> Artificial sequence
<220>
   <223> Synthetic oligonucleotide
<400> 301
   tcgtcgacga tgccgcgcgc ggc 23
<210> 302
   <211> 23
   <212> DNA
   <213> Artificial sequence
<220>
   <223> Synthetic oligonucleotide
<400> 302
   tcgtcgacga tgccgcgcgc ggc 23
<210> 303
   <211> 24
   <212> DNA
   <213> Artificial sequence
<220>
   <223> Synthetic oligonucleotide
<400> 303
   tcgtcgacga tgccgcgctg cggc 24
<210> 304
   <211> 24
   <212> DNA
   <213> Artificial sequence
<220>
   <223> Synthetic oligonucleotide
<400> 304
   tcgtcgtacg atgccgcgcg cggc 24
<210> 305
   <211> 25
   <212> DNA
   <213> Artificial sequence
<220>
   <223> Synthetic oligonucleotide
<400> 305
   tcgtcgtacg atgccgcgct gcggc 25
<210> 306
   <211> 23
   <212> DNA
   <213> Artificial sequence
<220>
   <223> Synthetic oligonucleotide
<400> 306
   tcgtcgacga tgccgcgcgc ggc 23
<210> 307
   <211> 23
   <212> DNA
   <213> Artificial sequence
<220>
   <223> Synthetic oligonucleotide
<400> 307
   tcgtcgacga tgccgcgcgc ggc 23
<210> 308
   <211> 24
   <212> DNA
   <213> Artificial sequence
<220>
   <223> Synthetic oligonucleotide
<220>
   <221> misc_feature
   <222> (24) .. (24)
   <223> where t is an inverse nucleoside
<400> 308
   tcgtcgacga tcggcgcgcg ccgt 24
<210> 309
   <211> 24
   <212> DNA
   <213> Artificial sequence
<220>
   <223> Synthetic oligonucleotide
<220>
   <221> misc_feature
   <222> (24) .. (24)
   <223> where t is an inverse nucleoside
<400> 309
   tcgtcgacga tcggcgcgcg ccgt 24
<210> 310
   <211> 24
   <212> DNA
   <213> Artificial sequence
<220>
   <223> Synthetic oligonucleotide
<220>
   <221> misc_feature
   <222> (24) .. (24)
   <223> where t is an inverse nucleoside
<400> 310
   tcgtcgacga tcggcgcgcg ccgt 24
<210> 311
   <211> 23
   <212> DNA
   <213> Artificial sequence
<220>
   <223> Synthetic oligonucleotide
<400> 311
   tcgtgcacga tcggcgcgcg ccg 23
<210> 312
   <211> 23
   <212> DNA
   <213> Artificial sequence
<220>
   <223> Synthetic oligonucleotide
<220>
   <221> misc_feature
   <222> (2)..(2)
   <223> where n is 5-methyl-deoxycytidine
<400> 312
   tngtcgacga tcggcgcgcg ccg 23
<210> 313
   <211> 23
   <212> DNA
   <213> Artificial sequence
<220>
   <223> Synthetic oligonucleotide
<220>
   <221> misc_feature
   <222> (5)..(5)
   <223> where n is 5-methyl-deoxycytidine
<400> 313
   tcgtngacga tcggcgcgcg ccg 23
<210> 314
   <211> 23
   <212> DNA
   <213> Artificial sequence
<220>
   <223> Synthetic oligonucleotide
<220>
   <221> misc_feature
   <222> (8)..(8)
   <223> where n is 5-methyl-deoxycytidine
<400> 314
   tcgtcganga tcggcgcgcg ccg 23
<210> 315
   <211> 23
   <212> DNA
   <213> Artificial sequence
<220>
   <223> Synthetic oligonucleotide
<220>
   <221> misc_feature
   <222> (12)..(12)
   <223> where n is 5-methyl-deoxycytidine
<400> 315
   tcgtcgacga tnggcgcgcg ccg 23
<210> 316
   <211> 18
   <212> DNA
   <213> Artificial sequence
<220>
   <223> Synthetic oligonucleotide
<400> 316
   tcgacgtcga cgtcgacg 18
<210> 317
   <211> 18
   <212> DNA
   <213> Artificial sequence
<220>
   <223> Synthetic oligonucleotide
<400> 317
   tcgacgtcga cgtcgacg 18
<210> 318
   <211> 18
   <212> DNA
   <213> Artificial sequence
<220>
   <223> Synthetic oligonucleotide
<400> 318
   tcgacgtcga cgtcgacg 18
<210> 319
   <211> 25
   <212> DNA
   <213> Artificial sequence
<220>
   <223> Synthetic oligonucleotide
<220>
   <221> misc_feature
   <222> (25)..(25)
   <223> where t is an inverse nucleoside
<400> 319
   tcgtcgacgt tcggcgccgt gccgt 25
<210> 320
   <211> 25
   <212> DNA
   <213> Artificial sequence
<220>
   <223> Synthetic oligonucleotide
<220>
   <221> misc_feature
   <222> (25)..(25)
   <223> where t is an inverse nucleoside
<400> 320
   tcgtcgacgt tcggcgccgt gccgt 25
<210> 321
   <211> 25
   <212> DNA
   <213> Artificial sequence
<220>
   <223> Synthetic oligonucleotide
<220>
   <221> misc_feature
   <222> (25)..(25)
   <223> where t is an inverse nucleoside
<400> 321
   tcgtcgacgt tcggcgccgt gccgt 25
<210> 322
   <211> 23
   <212> DNA
   <213> Artificial sequence
<220>
   <223> Synthetic oligonucleotide
<220>
   <221> misc_feature
   <222> (13)..(23)
   <223> where the nucleosides are inverse nucleosides
<400> 322
   gccgcgcgcg gctagcagct gct 23
<210> 323
   <211> 23
   <212> DNA
   <213> Artificial sequence
<220>
   <223> Synthetic oligonucleotide
<220>
   <221> misc_feature
   <222> (13)..(23)
   <223> where the nucleosides are inverse nucleosides
<400> 323
   cggcgcgcgc cgtagcagct gct 23
<210> 324
   <211> 23
   <212> DNA
   <213> Artificial sequence
<220>
   <223> Synthetic oligonucleotide
<220>
   <221> misc_feature
   <222> (13)..(23)
   <223> where the nucleosides are inverse nucleosides
<400> 324
   gccgcgcgcg gctagcagct gct 23
<210> 325
   <211> 23
   <212> DNA
   <213> Artificial sequence
<220>
   <223> Synthetic oligonucleotide
<220>
   <221> misc_feature
   <222> (13)..(23)
   <223> where the nucleosides are inverse nucleosides
<400> 325
   cggcgcgcgc cgtagcagct gct 23
<210> 326
   <211> 24
   <212> DNA
   <213> Artificial sequence
<220>
   <223> Synthetic oligonucleotide
<220>
   <221> misc_feature
   <222> (14)..(24)
   <223> where the nucleosides are inverse nucleosides
<400> 326
   cggcgccgtg ccgttgcagc tgct 24
<210> 327
   <211> 24
   <212> DNA
   <213> Artificial sequence
<220>
   <223> Synthetic oligonucleotide
<220>
   <221> misc_feature
   <222> (14)..(24)
   <223> where the nucleosides are inverse nucleosides
<400> 327
   gccgtgccgc ggcttgcagc tgct 24
<210> 328
   <211> 24
   <212> DNA
   <213> Artificial sequence
<220>
   <223> Synthetic oligonucleotide
<220>
   <221> misc_feature
   <222> (14)..(24)
   <223> where the nucleosides are inverse nucleosides
<400> 328
   cggcgccgtg ccgttgcagc tgct 24
<210> 329
   <211> 24
   <212> DNA
   <213> Artificial sequence
<220>
   <223> Synthetic oligonucleotide
<220>
   <221> misc_feature
   <222> (14)..(24)
   <223> where the nucleosides are inverse nucleosides
<400> 329
   gccgtgccgc ggcttgcagc tgct 24
<210> 330
   <211> 25
   <212> DNA
   <213> Artificial sequence
<220>
   <223> Synthetic oligonucleotide
<220>
   <221> misc_feature
   <222> (15)..(25)
   <223> where the nucleosides are inverse nucleosides
<400> 330
   tcggcgcgcg ccgatagcag ctgct 25
<210> 331
   <211> 25
   <212> DNA
   <213> Artificial sequence
<220>
   <223> Synthetic oligonucleotide
<220>
   <221> misc_feature
   <222> (15)..(25)
   <223> where the nucleosides are inverse nucleosides
<400> 331
   tcggcgcgcg ccgatagcag ctgct 25
<210> 332
   <211> 25
   <212> DNA
   <213> Artificial sequence
<220>
   <223> Synthetic oligonucleotide
<220>
   <221> misc_feature
   <222> (15)..(25)
   <223> where the nucleosides are inverse nucleosides
<400> 332
   tcggcgccgt gccgttgcag ctgct 25
<210> 333
   <211> 25
   <212> DNA
   <213> Artificial sequence
<220>
   <223> Synthetic oligonucleotide
<220>
   <221> misc_feature
   <222> (15)..(25)
   <223> where the nucleosides are inverse nucleosides
<400> 333
   tcggcgccgt gccgttgcag ctgct 25
<210> 334
   <211> 13
   <212> DNA
   <213> Artificial sequence
<220>
   <223> Synthetic oligonucleotide
<220>
   <221> misc_feature
   <222> (7)..(7)
   <223> where n is a linker
<400> 334
   cggcgcngcg ccg 13
<210> 335
   <211> 23
   <212> DNA
   <213> Artificial sequence
<220>
   <223> Synthetic oligonucleotide
<400> 335
   tcgtcgacgt tcggcgcgcg ccg 23
<210> 336
   <211> 22
   <212> DNA
   <213> Artificial sequence
<220>
   <223> Synthetic oligonucleotide
<400> 336
   tcgtcgacga cggcgcgcgc cg 22
<210> 337
   <211> 23
   <212> DNA
   <213> Artificial sequence
<220>
   <223> Synthetic oligonucleotide
<220>
   <221> misc_feature
   <222> (11)..(11)
   <223> n is a, c, g, or t
<400> 337
   tcgtcgacga ncggcgcgcg ccg 23
<210> 338
   <211> 23
   <212> DNA
   <213> Artificial sequence
<220>
   <223> Synthetic oligonucleotide
<220>
   <221> misc_feature
   <222> (11)..(11)
   <223> n is a, c, g, or t
<400> 338
   tcgtcgacga ncggcgcgcg ccg 23
<210> 339
   <211> 23
   <212> DNA
   <213> Artificial sequence
<220>
   <223> Synthetic oligonucleotide
<220>
   <221> misc_feature
   <222> (11)..(11)
   <223> where n is d-spacer
<400> 339
   tcgtcgacga ncggcgcgcg ccg 23
<210> 340
   <211> 21
   <212> DNA
   <213> Artificial sequence
<220>
   <223> Synthetic oligonucleotide
<400> 340
   ggggacgacg tcgtgggggg g 21
<210> 341
   <211> 15
   <212> DNA
   <213> Artificial sequence
<220>
   <223> Synthetic oligonucleotide
<400> 341
   tcgacgtcgt ggggg 15
<210> 342
   <211> 16
   <212> DNA
   <213> Artificial sequence
<220>
   <223> Synthetic oligonucleotide
<400> 342
   tccaggactt ctctca 16
<210> 343
   <211> 22
   <212> DNA
   <213> Artificial sequence
<220>
   <223> Synthetic oligonucleotide
<400> 343
   tcgtcgtttt cggcgcgcgc cg 22
<210> 344
   <211> 23
   <212> DNA
   <213> Artificial sequence
<220>
   <223> Synthetic oligonucleotide
<220>
   <221> misc_feature
   <222> (6)..(7)
   <223> where the nucleosides are 2'-O-methyl nucleosides
<220>
   <221> misc_feature
   <222> (9)..(10)
   <223> where the nucleosides are 2'-O-methyl nucleosides
<220>
   <221> misc_feature
   <222> (13)..(14)
   <223> where the nucleosides are 2'-O-methyl nucleosides
<220>
   <221> misc_feature
   <222> (16)..(16)
   <223> where the nucleoside is a 2'-O-methyl nucleoside
<220>
   <221> misc_feature
   <222> (18)..(18)
   <223> where the nucleoside is a 2'-O-methyl nucleoside
<220>
   <221> misc_feature
   <222> (20)..(20)
   <223> where the nucleoside is a 2'-O-methyl nucleoside
<220>
   <221> misc_feature
   <222> (23)..(23)
   <223> where the nucleoside is a 2'-O-methyl nucleoside
<400> 344
   tcgtcgacga tcggcgcgcg ccg 23
<210> 345
   <211> 23
   <212> DNA
   <213> Artificial sequence
<220>
   <223> Synthetic oligonucleotide
<220>
   <221> misc_feature
   <222> (3)..(3)
   <223> where the nucleoside is a 2'-O-methyl nucleoside
<220>
   <221> misc_feature
   <222> (6).. (7)
   <223> where the nucleosides are 2'-O-methyl nucleosides
<220>
   <221> misc_feature
   <222> (9)..(10)
   <223> where the nucleosides are 2'-O-methyl nucleosides
<220>
   <221> misc_feature
   <222> (13)..(14)
   <223> where the nucleosides are 2'-O-methyl nucleosides
<220>
   <221> misc_feature
   <222> (16)..(16)
   <223> where the nucleoside is a 2'-O-methyl nucleoside
<220>
   <221> misc_feature
   <222> (18)..(18)
   <223> where the nucleoside is a 2'-O-methyl nucleoside
<220>
   <221> misc_feature
   <222> (20)..(20)
   <223> where the nucleoside is a 2'-O-methyl nucleoside
<220>
   <221> misc_feature
   <222> (23)..(23)
   <223> where the nucleoside is a 2'-O-methyl nucleoside
<400> 345
   tcgtcgacga tcggcgcgcg ccg 23
<210> 346
   <211> 23
   <212> DNA
   <213> Artificial sequence
<220>
   <223> Synthetic oligonucleotide
<220>
   <221> misc_feature
   <222> (6)..(7)
   <223> where the nucleosides are 2'-O-methyl nucleosides
<220>
   <221> misc_feature
   <222> (9)..(10)
   <223> where the nucleosides are 2'-O-methyl nucleosides
<220>
   <221> misc_feature
   <222> (13)..(14)
   <223> where the nucleosides are 2'-O-methyl nucleosides
<220>
   <221> misc_feature
   <222> (16)..(16)
   <223> where the nucleoside is a 2'-O-methyl nucleoside
<220>
   <221> misc_feature
   <222> (18)..(18)
   <223> where the nucleoside is a 2'-O-methyl nucleoside
<220>
   <221> misc_feature
   <222> (20)..(20)
   <223> where the nucleoside is a 2'-O-methyl nucleoside
<220>
   <221> misc_feature
   <222> (23)..(23)
   <223> where the nucleoside is a 2'-O-methyl nucleoside
<400> 346
   tcgtcgacga tcggcgcgcg ccg 23
<210> 347
   <211> 23
   <212> DNA
   <213> Artificial sequence
<220>
   <223> Synthetic oligonucleotide
<220>
   <221> misc_feature
   <222> (3)..(3)
   <223> where the nucleoside is a 2'-O-methyl nucleoside
<220>
   <221> misc_feature
   <222> (6)..(7)
   <223> where the nucleosides are 2'-O-methyl nucleosides
<220>
   <221> misc_feature
   <222> (9)..(10)
   <223> where the nucleosides are 2'-O-methyl nucleosides
<220>
   <221> misc_feature
   <222> (13)..(14)
   <223> where the nucleosides are 2'-O-methyl nucleosides
<220>
   <221> misc_feature
   <222> (16)..(16)
   <223> where the nucleoside is a 2'-O-methyl nucleoside
<220>
   <221> misc_feature
   <222> (18)..(18)
   <223> where the nucleoside is a 2'-O-methyl nucleoside
<220>
   <221> misc_feature
   <222> (20)..(20)
   <223> where the nucleoside is a 2'-O-methyl nucleoside
<220>
   <221> misc_feature
   <222> (23)..(23)
   <223> where the nucleoside is a 2'-O-methyl nucleoside
<400> 347
   tcgtcgacga tcggcgcgcg ccg 23
<210> 348
   <211> 23
   <212> DNA
   <213> Artificial sequence
<220>
   <223> Synthetic oligonucleotice
<400> 348
   tcgtcgtcgt tcggcgcgcg ccg 23